# EUROPEAN PATENT SPECIFICATION

(11) **EP 2 998 316 B1**
(45) Date of publication and mention of the grant of the patent: **12.06.2019**
(21) Application number: 15193141.7
(22) Date of filing: 28.02.2008
(51) Int. Cl.: C07K 14/16, C07K 14/445, C12N 15/861, A61K 39/00, A61K 39/015, A61K 39/12, A61K 39/21, C07K 14/005, C07K 14/35, A61K 39/35

(54) **NOVEL METHOD AND COMPOSITIONS**
NEUES VERFAHREN UND ZUSAMMENSETZUNGEN
NOUVEAU PROCÉDÉ ET NOUVELLES COMPOSITIONS

(30) Priority: 02.03.2007 US 892714 P
(43) Date of publication of application: 23.03.2016
(62) Divisional of application: 08709258.1
(73) Proprietor: GlaxoSmithKline Biologicals S.A., 1330 Rixensart (BE)
(72) Inventor: VOSS, Gerald Hermann, 1330 Rixensart (BE)
(74) Representative: Drury, Elizabeth Anne

(56) References cited:
- WO-A-2004/110482
- WO-A2-2006/117240
- HAVENGA M ET AL: "Novel replication-incompetent adenoviral B-group vectors: high vector stability and yield in PER.C6 cells", JOURNAL OF GENERAL VIROLOGY, SOCIETY FOR GENERAL MICROBIOLOGY, SPENCERS WOOD, GB, vol. 87, no. Part 8, 1 August 2006 (2006-08-01), pages 2135-2143, XP002496482, ISSN: 0022-1317, DOI: 10.1099/VIR.0.81956-0
- XING ET AL: "Use of recombinant virus-vectored tuberculosis vaccines for respiratory mucosal immunization", TUBERCULOSIS, ELSEVIER, GB, vol. 86, no. 3-4, 1 May 2006 (2006-05-01), pages 211-217, XP005416881, ISSN: 1472-9792, DOI: 10.1016/J.TUBE.2006.01.017
- XING ZHOU ET AL: "Recent advances in the development of adenovirus- and poxvirus-vectored tuberculosis vaccines", CURRENT GENE THERAPY, BENTHAM SCIENCE PUBLISHERS LTD, NL, vol. 5, no. 5, 1 October 2005 (2005-10-01) , pages 485-492, XP009113281, ISSN: 1566-5232, DOI: 10.2174/156652305774329230
- BENNEKOV THOMAS ET AL: "Alteration of epitope recognition pattern in Ag85B and ESAT-6 has a profound influence on vaccine-induced protection against Mycobacterium tuberculosis", EUROPEAN JOURNAL OF IMMUNOLOGY, vol. 36, no. 12, December 2006 (2006-12), pages 3346-3355, XP009188073, ISSN: 0014-2980
- GANNE V ET AL: "Enhancement of the efficacy of a replication-defective adenovirus-vectored vaccine by the addition of oil adjuvants", VACCINE, BUTTERWORTH SCIENTIFIC. GUILDFORD, GB, vol. 12, no. 13, 1 January 1994 (1994-01-01), pages 1190-1196, XP002393618, ISSN: 0264-410X
- MOORE A C ET AL: "Combination vaccines: Synergistic simultaneous induction of antibody and T-cell immunity", EXPERT REVIEW OF VACCINES, FUTURE DRUGS, LONDON, GB, vol. 6, no. 1, 1 February 2007 (2007-02-01), pages 111-121, XP009188043, ISSN: 1476-0584, DOI: 10.1586/14760584.6.1.111
- JONES T R ET AL: "Protection of Aotus monkeys by Plasmodium falciparum EBA-175 region II DNA prime-protein boost immunization regimen", JOURNAL OF INFECTIOUS DISEASES. JID, UNIVERSITY OF CHICAGO PRESS, US, vol. 183, no. 2, 15 January 2001 (2001-01-15), pages 303-312, ISSN: 0022-1899, DOI: 10.1086/317933
- DATABASE BIOSIS [Online] BIOSCIENCES INFORMATION SERVICE, PHILADELPHIA, PA, US; April 2011 (2011-04), DERRICK STEVEN C ET AL: "Vaccine-induced anti-tuberculosis protective immunity in mice correlates with the magnitude and quality of multifunctional CD4 T cells", Database accession no. PREV201100333439 & VACCINE, vol. 29, no. 16, April 2011 (2011-04), pages 2902-2909, ISSN: 0264-410X(print), DOI: 10.1016/J.VACCINE.2011.02.010

## Description

### Field of the invention

This invention relates to novel vaccine compositions and their use in the stimulation of immune responses in mammals, especially humans, and in particular for the prevention and treatment of infection by pathogens, specifically *Mycobaterium spp.* In particular it relates to compositions capable of inducing CD4+ and CD8+ T-cell responses as well as antibody responses in subjects without recourse to complex prime-boost schedules.

### Background to the invention

Inactivated whole organisms have been used in successful vaccination since the late nineteenth century. In more recent times, vaccines involving the administration of extracts, subunits, toxoids and capsular polysaccharides have been employed. Since genetic engineering techniques have been available, the use of recombinant proteins has been a favoured strategy, obviating many of the risks associated with use of purified proteins from natural sources.

Early vaccine approaches were based on the administration of proteins which stimulated some aspect of the immune response in vivo. Subsequently it was appreciated that immune responses could also be raised by administration of DNA which could be transcribed and translated by the host into an immunogenic protein.

The mammalian immune response has two key components: the humoral response and the cell-mediated response. The humoral response involves the generation of circulating antibodies which will bind to the antigen to which they are specific, thereby neutralising the antigen and favouring its subsequent clearance by a process involving other cells that are either cytotoxic or phagocytic. B-cells are responsible for generating antibodies (plasma B cells), as well as holding immunological humoral memory (memory B-cells), i.e. the ability to recognise an antigen some years after first exposure to it eg through vaccination. The cell mediated response involves the interplay of numerous different types of cells, among which are the T cells. T-cells are divided into a number of different subsets, mainly the CD4+ and CD8+ T cells.

Antigen-presenting cells (APC) such as macrophages and dendritic cells act as sentinels of the immune system, screening the body for foreign antigens. When extracellular foreign antigens are detected by APC, these antigens are phagocytosed (engulfed) inside the APC where they will be processed into smaller peptides. These peptides are subsequently presented on major histocompatibility complex class II (MHC II) molecules at the surface of the APC where they can be recognised by antigen-specific T lymphocytes expressing the CD4 surface molecules (CD4+ T cells). When CD4+ T cells recognise the antigen to which they are specific on MHCII molecules in the presence of additional adequate co-stimulatory signals, they become activated and secrete an array of cytokines that subsequently activate the other arms of the immune system. In general, CD4+T cells are classified into T helper 1 (Th1) or T helper 2 (Th2) subsets depending on the type of response they generate following antigen recognition. Upon recognition of a peptide-MHC II complex, Th1 CD4+ T cells secrete interleukins and cytokines such as interferon gamma thereby activating macrophages to release toxic chemicals such as nitric oxide and reactive oxygen/nitrogen species. IL-2 and TNF-alpha are also commonly categorized as Th1 cytokines. In contrast, Th2 CD4+ T cells generally secrete interleukins such as IL-4, IL-5 or IL-13.

Other functions of the T helper CD4+ T cells include providing help to activate B cells to produce and release antibodies. They can also participate to the activation of antigen-specific CD8+ T cells, the other major T cell subset beside CD4+ T cells.

CD8+ T cells recognize the peptide to which they are specific when it is presented on the surface of a host cell by major histocompatibility class I (MHC I) molecules in the presence of appropriate costimulatory signals. In order to be presented on MHC I molecules, a foreign antigen need to directly access the inside of the cell (the cytosol or nucleus) such as it is the case when a virus or intracellular bacteria directly penetrate a host cell or after DNA vaccination. Inside the cell, the antigen is processed into small peptides that will be loaded onto MHC I molecules that are redirected to the surface of the cell. Upon activation CD8+T cells secrete an array of cytokines such as interferon gamma that activates macrophages and other cells. In particular, a subset of these CD8+ T cells secretes lytic and cytotoxic molecules (e.g. granzyme, perforin) upon activation. Such CD8+ T cells are referred to as cytotoxic T cells.

More recently, an alternative pathway of antigen presentation involving the loading of extracellular antigens or fragments thereof onto MHCI complexes has been described and called "cross-presentation".

The nature of the T-cell response is also influenced by the composition of the adjuvant used in a vaccine. For instance, adjuvants containing MPL & QS21 have been shown to activate Th1 CD4+ T cells to secrete IFN-gamma (Stewart et al. Vaccine. 2006, 24 (42-43):6483-92).

Whereas adjuvants are well known to have value in enhancing immune responses to protein antigens, they have not generally been used in conjunction with DNA or DNA-based vector vaccination. There are several hypotheses as to why adjuvants have not been used in conjunction with DNA-vector based vaccines. Indeed, interferences between the adjuvant and the vector may have an impact on their stability. In addition, one might expect that adding an adjuvant to an attenuated vector could increase the reactogenicity induced by such product. Finally, increasing the immunogenicity of a DNA-vector based vaccine may lead to an enhanced neutralizing immune response against the vector itself, thereby precluding any boosting effect of subsequent injections of the same vector-based vaccine. In fact, in a vaccination protocol directed towards protection against *P. falciparum* infection, Jones et al (2001, J Infect Diseases 183, 303-312) have reported an adverse outcome after combining DNA, recombinant protein and adjuvant as a boosting composition following a prime by DNA. Indeed, the levels of parasitemia were significantly lower in a group in which the boosting composition contained protein and adjuvant only. It was concluded that use of the combination of DNA, recombinant protein and adjuvant in this protocol adversely affected the outcome on parasitemia as well as antibody responses.

On the other hand, there has been a report of enhancement of the efficacy of an adjuvanted DNA-based vector vaccine (Ganne et al. Vaccine (1994) 12(13) 1190-1196). In particular, the enhanced efficacy of a replication-defective adenovirus-vectored vaccine by the addition of oil adjuvants was correlated with higher antibody levels but the impact on CD4 and CD8 T cell responses was not reported.

The use of an apathogenic virus as an adjuvant has been disclosed in WO2007/016715. It was not mentioned that said virus could contain any heterologous polynucleotide.

It is generally thought that stimulation of both CD4+ and CD8+ cells are needed for optimal protective immunity, especially in certain diseases such as HIV infection/AIDS. In order to induce an optimal immune response either prophylactically or therapeutically, stimulation of both CD4+ and CD8+ cells is desirable. This is one of the main goal of "prime-boost" vaccination strategies in which the alternate administration of protein-based vaccines (inducing mostly CD4+T cells) with DNA-vector based vaccines, i.e. naked DNA, viral vectors or intracellular bacterial vectors such as listeria, (inducing mostly CD8+T cells) or vice versa most likely activates both CD4+ and CD8+ T cell responses.

However, although prime-boost vaccine strategies may generally give rise to a greater or more balanced response, the requirement to vaccinate on more than one occasion and certainly on more than two occasions can be burdensome or even unviable, especially in mass immunization programs for the developing world.

Furthermore, as already mentioned above, it is often not possible to boost the viral vector component because of immunity that may have been raised against the vector itself.

Thus the objects of the invention include one or more of the following: (a) to provide a complete vaccination protocol and a vaccine composition which stimulates the production of CD4+ and/or CD8+ cells and/or antibodies and in particular which obviates or mitigates the need for repeated immunizations; (b) to provide a vaccination protocol and a vaccine composition which better stimulates production of CD4+ cells and/or CD8+cells and/or antibodies relative to vaccine compositions containing an immunogenic polypeptide alone or a polynucleotide alone or relative to a conventional prime-boost protocol involving separate administration of immunogenic polypeptide and polynucleotide; (c) to provide a vaccine composition which stimulates or better stimulates Th1 responses; (d) to provide a vaccine composition and vaccination protocol in which required doses of components, especially viral vectors, are minimised; and (e) more generally to provide a useful vaccine composition and vaccination protocol for treatment or prevention of diseases caused by pathogens. By "better stimulates" is meant that the intensity and/or persistence of the response is enhanced.

### Summary of the invention

Thus according to the invention there is provided one or more first immunogenic polypeptides for use in a method of raising an immune response against *Mycobacterium spp.* wherein the one or more first immunogenic polypeptides are administered concomitantly with (i) one or more adenoviral vectors comprising one or more heterologous polynucleotides encoding one or more second immunogenic polypeptides and (ii) an adjuvant which comprise QS21 and 3D-MPL, wherein the one or more first and second polypeptides comprises residues 4-725 of SEQ ID NO: 10, and wherein the term "concomitantly" means wherein the one or more first and second immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more first and second immunogenic polypeptides, the one or more adenoviral vectors and the adjuvant are administered sequentially or simultaneously .

According to another aspect of the invention there is provided one or more adenoviral vectors comprising one or more heterologous polynucleotides encoding one or more second immunogenic polypeptides derived from *Mycobacterium spp.* for use in a method of raising an immune response against said *Mycobacterium spp.* wherein the one or more adenoviral vectors are administered concomitantly with (i) one or more first immunogenic polypeptides derived from said *Mycobacterium spp.* and (ii) an adjuvant which comprises QS21 and 3D-MPL, wherein the one or more first and second polypeptides comprises residues 4-725 of SEQ ID NO: 10, and wherein the term "concomitantly" means wherein the one or more first and second immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more first and second immunogenic polypeptides, the one or more adenoviral vectors and the adjuvant are administered sequentially or simultaneously. According to another specific aspect of the invention there is provided a vaccine composition comprising (i) one or more first immunogenic polypeptides; (ii) one or more adenoviral vectors comprising one or more heterologous polynucleotides encoding one or more second immunogenic polypeptides; and (iii) an adjuvant which comprises QS21 and 3D-MPL, wherein the one or more first and second polypeptides comprise residues 4-725 of SEQ ID NO: 10.

There is also disclosed an immunogenic composition comprising (i) one or more first immunogenic polypeptides derived from a pathogen; (ii) one or more adenoviral vectors comprising one or more heterologous polynucleotides encoding one or more second immunogenic polypeptides derived from said pathogen; and (iii) an adjuvant.

Said vaccines and immunogenic compositions suitably stimulate production of pathogen-specific CD4+ T-cells and/or CD8+ T-cells and/or antibodies.

By "pathogen-specific CD4+ T-cells and/or CD8+ T-cells and/or antibodies" is meant CD4+ T-cells and/or CD8+ T-cells and/or antibodies which specifically recognise the whole pathogen or a part (eg an immunogenic subunit) thereof. By "specifically recognise" is meant that the CD4+ T-cells and/or CD8+ T-cells and/or antibodies recognise in an immunospecific rather than a non-specific manner said pathogen (or part thereof).

There is also disclosed a method of stimulating an immune response in a mammal which comprises administering to a subject an immunologically effective amount of such a composition.

There is also provided use of such a composition in the manufacture of a medicament for stimulating an immune response in a mammal.

There is also provided such a composition for use in stimulating an immune response in a mammal.

There is also disclosed a method of stimulating the production of pathogen-specific CD4+ T-cells and/or CD8+ T-cells and/or antibodies in mammals which comprises administering to said mammal (i) one or more first immunogenic polypeptides derived from a pathogen; (ii) one or more adenoviral vectors comprising one or more heterologous polynucleotides encoding one or more second immunogenic polypeptides derived from said pathogen; and (iii) an adjuvant; wherein the one or more first immunogenic polypeptides, the one or more adenoviral vectors and the adjuvant are administered concomitantly, for example by administering an immunologically effective amount of an aforeseaid composition.

There is also provided use of aforesaid compositions in the manufacture of a medicament for stimulating the production of pathogen specific CD4+ and/or CD8+ cells and/or antibodies in mammals.

For example, production of CD4+ T-cells or CD8+ T-cells or antibodies is stimulated.

Suitably production of 2 and especially 3 of CD4+ T-cells and/or CD8+ T-cells and/or antibodies is stimulated.

Suitably production of CD8+ T-cells is stimulated. Suitably production of CD4+ and CD8+ T-cells is stimulated. Suitably production of CD4+ and CD8+ T-cells and antibodies is stimulated.

Alternatively suitably production of CD4+ T-cells is stimulated. Suitably production of CD4+ and antibodies is stimulated.

Alternatively suitably production of antibodies is stimulated.

The methods of the disclosure are suitably intended to provide the steps adequate for a complete method for raising an immune response (although the method may, if desired, be repeated). Therefore suitably the methods disclosed do not involve use of a priming dose of any immunogenic polypeptide or polynucleotide (e.g. in the form of a vector such as an adenoviral vector) encoding any immunogenic polypeptide.

For example there is disclosed a method of raising an immune response against a pathogen which consists of (a) administering (i) one or more first immunogenic polypeptides derived from said pathogen; (ii) one or more adenoviral vectors comprising one or more heterologous polynucleotides encoding one or more second immunogenic polypeptides derived from said pathogen; and (iii) an adjuvant; wherein the one or more immunogenic polypeptide, the one or more adenoviral vector and the adjuvant are administered concomitantly; and (b) optionally repeating the steps of (a).

The steps of the method may be repeated (e.g. repeated once) if a repeat gives rise to an improved immune response. An adequate response, at least as far as a T-cell response is concerned, may be obtained without any need for repetition.

There is also disclosed a method of raising an immune response against a pathogen which comprises (a) administering (i) one or more first immunogenic polypeptides derived from said pathogen; (ii) one or more adenoviral vectors comprising one or more heterologous polynucleotides encoding one or more second immunogenic polypeptides derived from said pathogen; and (iii) an adjuvant; wherein the one or more first immunogenic polypeptides, the one or more adenoviral vectors and the adjuvant are administered concomitantly; and wherein the method does not involve administering any priming dose of immunogenic polypeptide or polynucleotide encoding immunogenic polypeptide.

There is also provided a kit comprising (i) one or more first immunogenic polypeptides derived from *Mycobacterium spp* ; (ii) one or more adenoviral vectors comprising one or more heterologous polynucleotides encoding one or more second immunogenic polypeptides derived from *Mycobacterium spp;* and (iii) an adjuvant which comprises QS21 and 3D-MPL, wherein the one more first and second polypeptides comprise residues 4-725 of SEQ ID NO: 10, and wherein the one or more first and second immunogenic polypeptides, the one or more adenoviral vectors and the adjuvant are for administration concomitantly, wherein the term "concomitantly" means wherein the one or more first and second immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more first and second immunogenic polypeptides, the one or more adenoviral vectors and the adjuvant are administered sequentially or simultaneously.

Compositions and methods of the invention may be useful for the prevention of infection by pathogens in naive subjects, or prevention of re-infection in subjects who have previously been infected by pathogen or treatment of subjects who have been infected by pathogen.

### Brief description of the figures

Figure 1 shows a graphical representation of the construction of plasmid p73i-Tgrn
Figures 2-8 show the results of experiments discussed in Example 1, specifically:
   Figures 2a, 2b, 3a, 3b: CD4+ and CD8+ T-cell responses in response to restimulation by pools of peptides derived from p24, RT, Nef and p17 following various immunization protocols and at different timepoints;
   Figure 4: antibody responses against F4;
   Figures 5-8 antibody responses against F4 components p24, RT, p17 and Nef respectively;
Figure 9 shows the results of experiments discussed in Example 2, specifically:
   CD4+ T-cell responses in response to restimulation by pools of peptides derived from p24 and RT following various immunization protocols;
Figures 10-12 show the results of experiments discussed in Example 3, specifically:

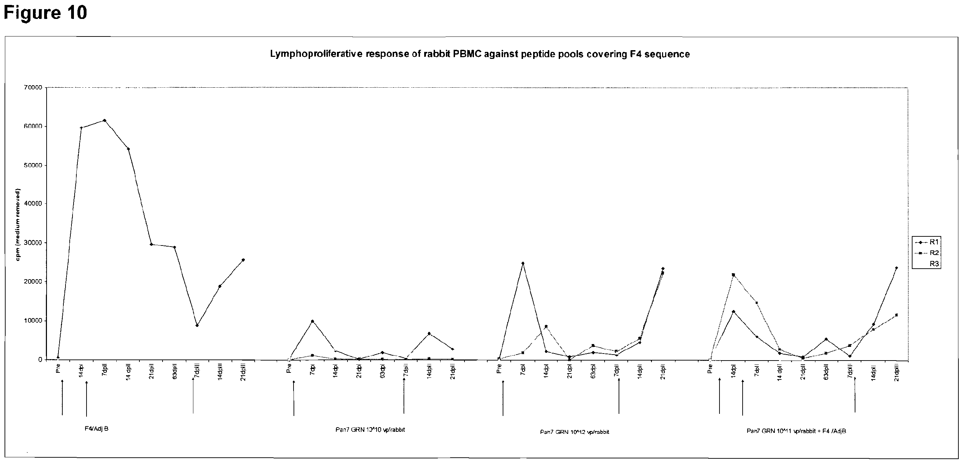

   Figure 10 shows the lymphoproliferative response of rabbit PBMC against peptide pools covering the F4 sequence;

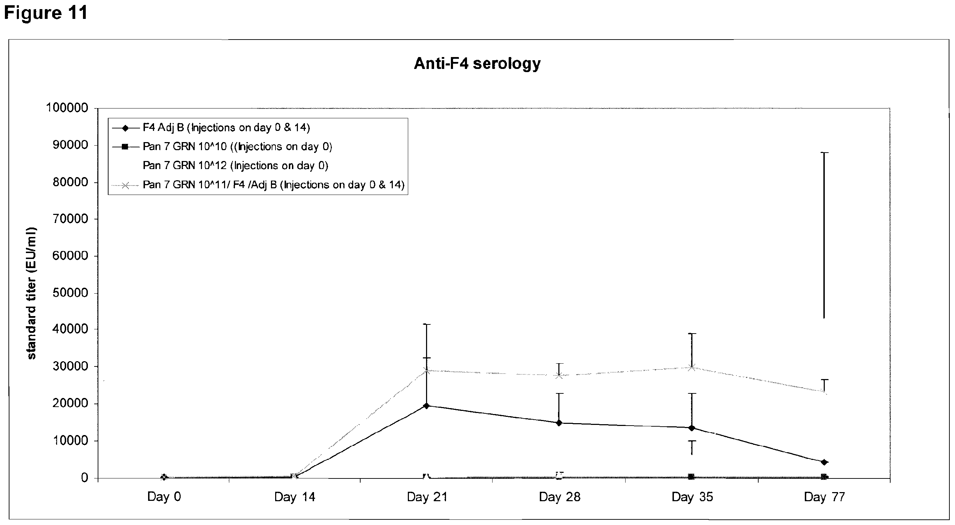

   Figure 11 shows the timecourse of antibody responses against F4;

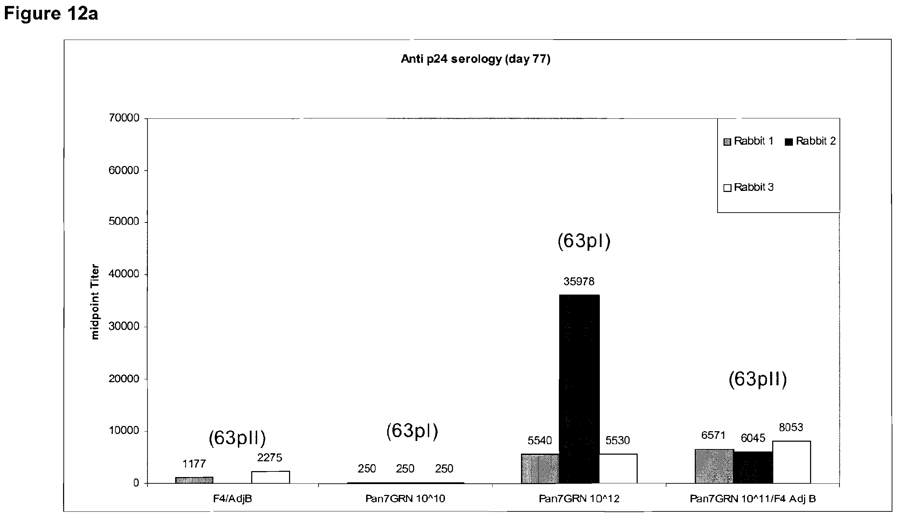

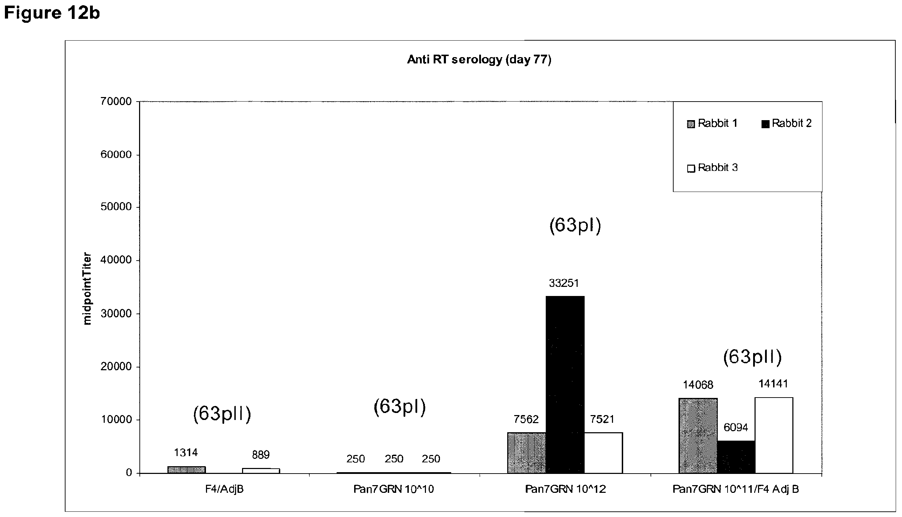

   Figures 12a and 12b shows antibody responses (on day 77) against F4 components p24 and RT respectively;

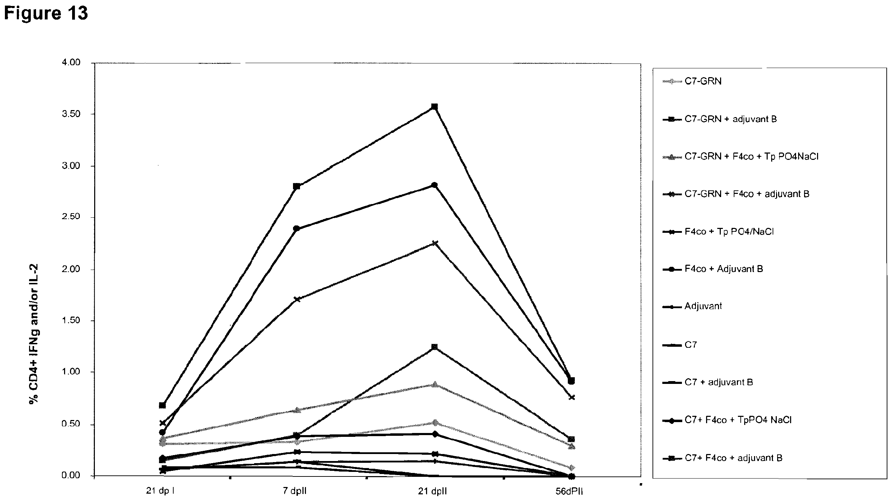

Figure 13 shows the quantification of HIV-1-specific CD4 T cells;

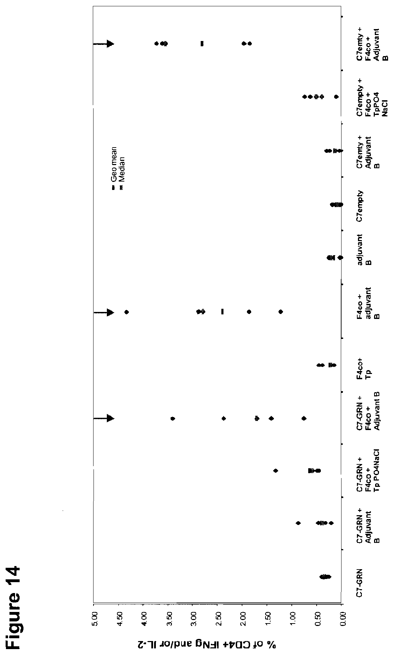

Figure 14 shows distribution of the frequency of F4-specific CD4 T cells 7 days after two immunizations;

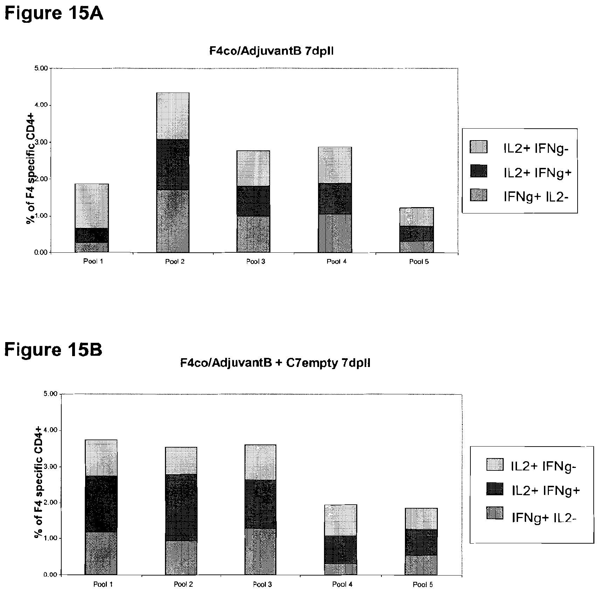

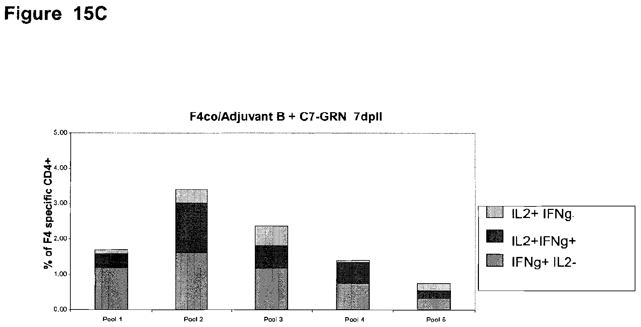

Figure 15 shows cytokine production of F4-specific CD4 T cells 7 days after two immunizations;

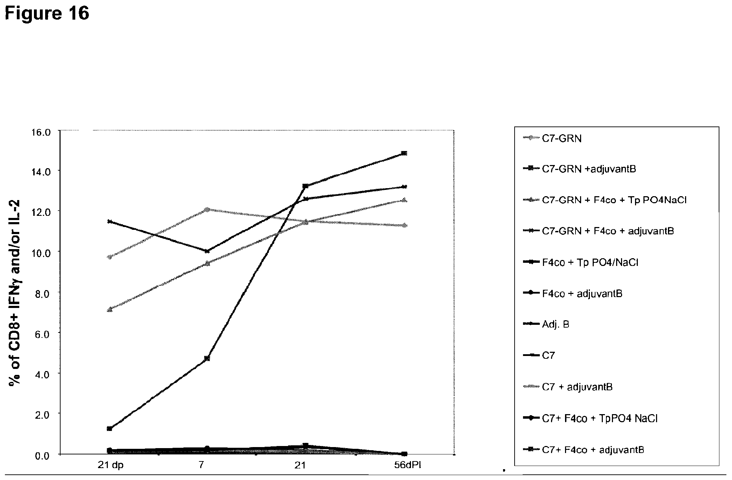

Figure 16 shows quantification of HIV-1-specific CD8 T cells;

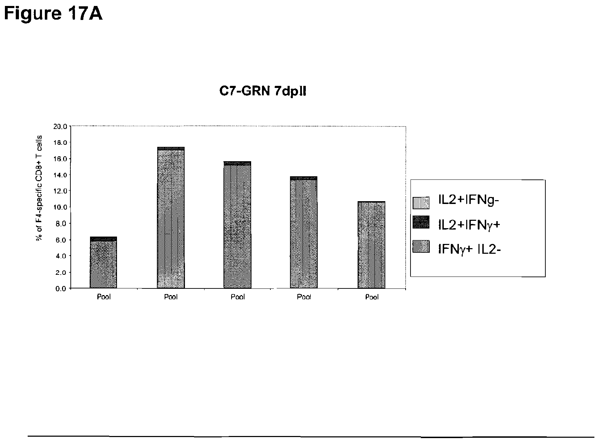

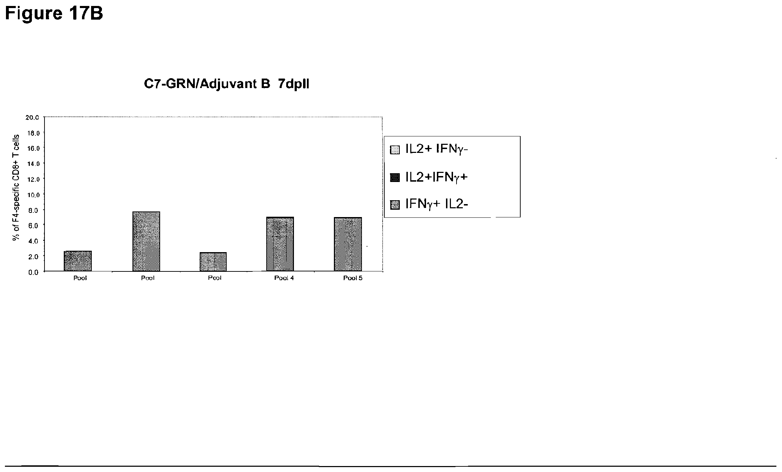

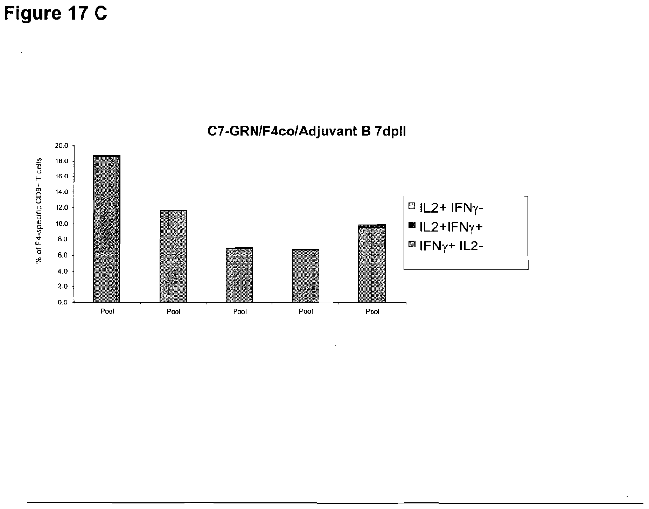

Figure 17 shows cytokine production of F4-specific CD8 T cells 7 days after two immunizations;

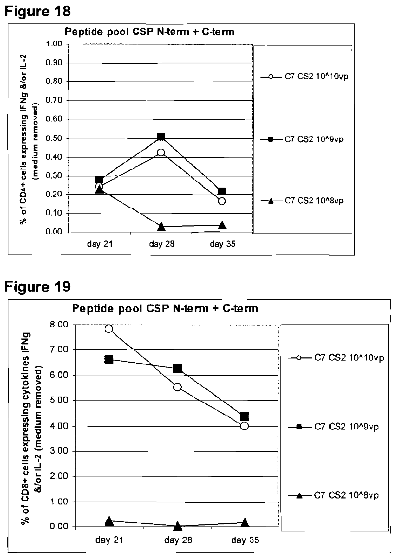

Figure 18 shows quantification of CSP-specific CD4 T cells;
Figure 19 shows quantification of CSP-specific CD8 T cells;

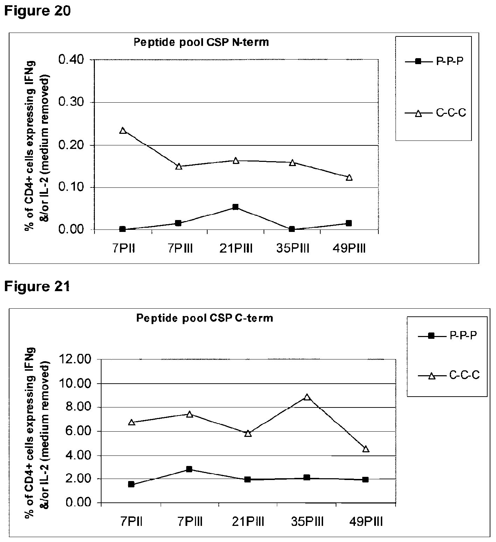

Figure 20 shows quantification of CSP(N-term)-specific CD4 T cells;
Figure 21 shows quantification of CSP(C-term)-specific CD4 T cells;

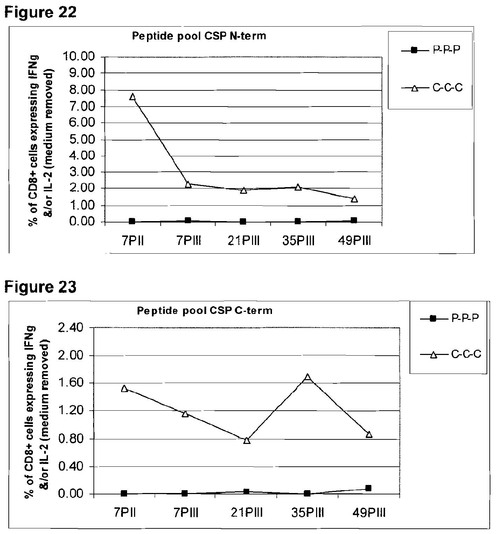

Figure 22 shows quantification of CSP(N-term)-specific CD8 T cells;
Figure 23 shows quantification of CSP(C-term)-specific CD8 T cells;

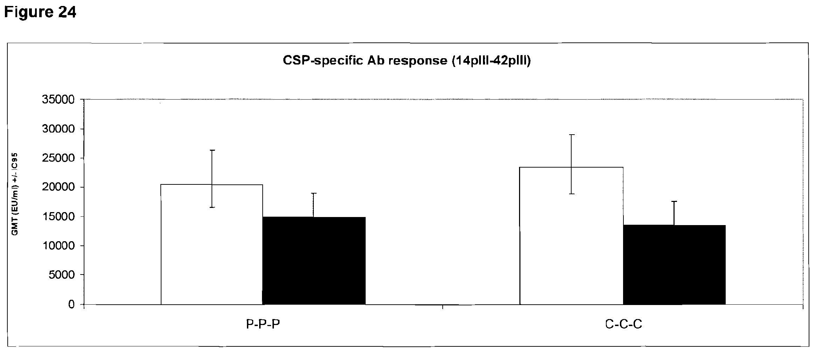

Figure 24 shows quantification of CSP-specific antibody titers.

**Summary of sequence listings**

| Amino acid or polynucleotide description | Sequence Identifier (SEQ ID No) |
|---|---|
| HIV Gag-RT-Nef ("GRN") (Clade B) (cDNA) | 1 |
| HIV Gag-RT-Nef ("GRN") (Clade B) (amino acid) | 2 |
| HIV Gag-RT-integrase-Nef ("GRIN") (Clade A) (cDNA) | 3 |
| HIV Gag-RT-integrase-Nef ("GRIN") (Clade A) (amino acid) | 4 |
| HIV gp140 (Clade A) (cDNA) | 5 |
| HIV gp140 (Clade A) (amino acid) | 6 |
| HIV gp120 (Clade B) (cDNA) | 7 |
| HIV gp120 (Clade B) (amino acid) | 8 |
| TB antigens fusion protein M72 (cDNA) | 9 |
| TB antigens fusion protein M72 (amino acid) | 10 |
| P. falciparum CS protein-derived antigen (cDNA) | 11 |
| P. falciparum CS protein-derived antigen (amino acid) | 12 |
| P. falciparum CS protein-derived fusion protein "RTS" (cDNA) | 13 |
| P. falciparum CS protein-derived fusion protein "RTS" (amino acid) | 14 |
| HIV p24-RT-Nef-p17 (cDNA) | 15 |
| HIV p24-RT-Nef-p17 (amino acid) | 16 |

The above recited sequences 9 and 10 may be employed as polypeptides or polynucletides encoding polypeptides of use in exemplary aspects of the invention. Said polypeptides may consist of or comprise the above mentioned sequences. Initial Met residues are optional. N-terminal His residues (including His residues immediately following an initial Met, as in SEQ ID No 9) are optional or an N-terminal His tag of a different length may be employed (eg typically up to 6 His residues may be employed to facilitate isolation of the protein). Analogue proteins which have significant sequence identity eg greater than 80% eg greater than 90% eg greater than 95% eg greater than 99% sequence identity over the whole length of the reference sequence may be employed, especially when the analogue protein has a similar function and particularly when the analogue protein is similarly immunogenic. For example up to 20 eg up to 10 eg 1-5 susbtitutions (eg conservative substitutions) may be tolerated. Nucleic acids which differ from those recited above which encode the same proteins, or the aforementioned analogue proteins, may be employed. Sequence identity may be determined by conventional means eg using BLAST. In one specific variant of SEQ ID No 16 that may be mentioned, reside 398 is Ser and not Cys.

### Detailed description of the invention.

As used herein the term "concomitantly" means wherein the one or more immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more immunogenic polypeptides, the one or more adenoviral vectors and the adjuvant are administered sequentially or simultaneously.

As used herein, the term "epitope" refers to an immunogenic amino acid sequence. An epitope may refer to a minimum amino acid sequence of typically 6-8 amino acids which minimum sequence is immunogenic when removed from its natural context, for example when transplanted into a heterologous polypeptide. An epitope may also refer to that portion of a protein which is immunogenic, where the polypeptide containing the epitope is referred to as the antigen (or sometimes "polypeptide antigen"). A polypeptide or antigen may contain one or more (eg 2 or 3 or more) distinct epitopes. The term "epitope" embraces B-cell and T-cell epitopes. The term "T-cell epitope" embraces CD4+ T-cell epitopes and CD8+ T-cell epitopes (sometimes also referred to as CTL epitopes).

The term "immunogenic polypeptide" refers to a polypeptide which is immunogenic, that is to say it is capable of eliciting an immune response in a mammal, and therefore contains one or more epitopes (eg T-cell and/or B-cell epitopes). Immunogenic polypeptides may contain one or more polypeptide antigens eg in an unnatural arrangement such as in a fusion protein.

Immunogenic polypeptides will typically be recombinant proteins produced eg by expression in a heterologous host such as a bacterial host, in yeast or in cultured mammalian cells.

The term "polypeptide derived from a pathogen" means a polypeptide which partially or wholly contains sequences (i.e. antigens) which occur naturally in pathogens or bear a high degree of sequence identity thereto (eg more than 95% identity over a stretch of at least 10 eg at least 20 amino acids).

Immunogenic polypeptides may contain one or more (eg 1, 2, 3 or 4) polypeptide antigens.

Unless otherwise specified, an "immune response" may be a cellular and/or a humoral response.

In one embodiment of the invention one or more of said one or more first immunogenic polypeptides is substantially the same as one or more of said one or more second mmunogenic polypeptides. For example one of the at least one first immunogenic polypeptides and one of the at least one second immunogenic polypeptides may have an overall sequence identity of 90% or more eg 95% or more eg 98% or 99% or more over the length of one or other immunogenic polypeptides.

In another embodiment of the invention one or more of said one or more first immunogenic polypeptides contains at least one antigen which is substantially the same as an antigen contained in one or more of said one or more second immunogenic polypeptides. For example one of the at least one first immunogenic polypeptides and one of the at least one second immunogenic polypeptides may have an overall sequence identity of 90% or more eg 95% or more eg 98% or 99% or more over a stretch of 20 amino acids or more eg 40 amino acids or more eg 60 amino acids or more.

Suitably one or more first immunogenic polypeptides comprise at least one T cell epitope.

Suitably one or more second immunogenic polypeptides comprise at least one T cell epitope.

Suitably the one or more first immunogenic polypeptides comprise at least one B cell epitope.

Suitably the one or more second immunogenic polypeptides comprise at least one B cell epitope

In another embodiment of the invention one or more of said one or more first immunogenic polypeptides and one or more of said one or more second immunogenic polypeptides share one or more identical B-cell and/or T-cell epitopes. Suitably they share one or more identical amino acid sequences of length 10 amino acids or more eg 15 amino acids or more eg 25 amino acids or more.

Also disclosed are examples in which none of the one or more of said one or more first immunogenic polypeptides is substantially the same as or contains any antigen in common with one or more of said one or more second immunogenic polypeptides, for example they may have an overall sequence identity of less than 90% over a stretch of 20 amino acids or more eg 40 amino acids or more eg 60 amino acids or more.

Thus, they may not share any B-cell or T-cell epitopes. For example, they may note share any identical amino acid sequences of length 10 amino acids or more eg at 15 amino acids or more eg 25 amino acids or more.

In one specific embodiment of the invention a first immunogenic polypeptide and a second immunogenic polypeptide contain the same antigens in the same arrangement or in a different arrangement (eg in a different arrangement). By "different arrangement" is meant that they may be arranged in a different order and/or they may be divided. In another specific embodiment of the invention a first immunogenic polypeptide and a second immunogenic polypeptide are the same.

The composition according to the invention may contain one first immunogenic polypeptide as the only immunogenic polypeptide in the composition. Alternatively the composition according to the invention may contain more than one first immunogenic polypeptides eg 2 or 3 or 4 or more immunogenic polypeptides.

The composition according to the invention may comprise one adenoviral vector. Alternatively it may comprise more than one adenoviral vector eg 2 adenoviral vectors.

In compositions according to the invention a adenoviral vector may comprise a heterologous polynucleotide which encodes for one second immunogenic polypeptide or it may comprise more than one heterologous polynucleotide which together encode for more than one second immunogenic polypeptide under the control of more than one promoter.

As well as for prophylactic vaccination, the compositions of the invention may also be used in individuals that are already infected with pathogen, and result in improved immunological control of the established infection.

### Antigens

Antigens of use according to the invention are derived from pathogens, more specifically *Mycobaterium spp.*
Pathogens include viruses, bacteria, protozoa and other parasitic organisms harmful to mammals including man.

Suitable polypeptide antigens to be administered as polypeptide or polynucleotide encoding polypeptide according to this disclosure include antigens derived from HIV (eg HIV-1), human herpes viruses (such as gH, gL gM gB gC gK gE or gD or derivatives thereof or Immediate Early protein such as ICP27 , ICP 47, ICP4, ICP36 from HSV1 or HSV2), cytomegalovirus, especially Human, (such as gB or derivatives thereof), Epstein Barr virus (such as gp350 or derivatives thereof), Varicella Zoster Virus (such as gpI, II, III and IE63), or from a hepatitis virus such as hepatitis B virus (for example Hepatitis B Surface antigen, PreS1, PreS2 and Surface env proteins, Hepatitis B core antigen or pol), hepatitis C virus (eg Core, E1, E2, P7, NS2, NS3, NS4A, NS4B, NS5A and B) and hepatitis E virus antigen, or from other viral pathogens, such as paramyxoviruses: Respiratory Syncytial virus (such as F and G proteins or derivatives thereof), or antigens from parainfluenza virus, measles virus, mumps virus, human papilloma viruses (for example HPV6, 11, 16, 18, eg L1, L2, E1, E2, E3, E4, E5, E6, E7), flaviviruses (e.g. Yellow Fever Virus, Dengue Virus, Tick-borne encephalitis virus, Japanese Encephalitis Virus) or Influenza virus (such as haemaggluttin, nucleoprotein, NA, or M proteins, or combinations thereof), or antigens derived from bacterial pathogens such as *Neisseria spp,* including *N. gonorrhea* and *N. meningitidis,* eg, transferrin-binding proteins, lactoferrin binding proteins, PilC, adhesins); *S. pyogenes* (for example M proteins or fragments thereof, C5A protease, *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis, M. bovis, M. leprae, M. avium, M. paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E. coli,* enteropathogenic *E. coli* (for example shiga toxin-like toxin or derivatives thereof); *Vibrio spp,* including *V. cholera* (for example cholera toxin or derivatives thereof*); Shigella spp,* including *S. sonnei, S. dysenteriae, S. flexnerii; Yersinia spp,* including *Y.* enterocolitica (for example a Yop protein), *Y. pestis, Y. pseudotuberculosis; Campylobacter spp,* including *C. jejuni* (for example toxins, adhesins and invasins) and *C. coli; Salmonella spp,* including *S. typhi, S. paratyphi, S. choleraesuis, S. enteritidis; Listeria spp.,* including *L. monocytogenes; Helicobacter spp,* including *H.* pylori (for example urease, catalase, vacuolating toxin); *Pseudomonas spp,* including *P. aeruginosa; Staphylococcus spp.,* including *S. aureus, S. epidermidis; Enterococcus spp.,* including *E. faecalis, E. faecium; Clostridium spp.,* including C. tetani (for example tetanus toxin and derivative thereof), *C. botulinum* (for example botulinum toxin and derivative thereof)*, C*. *difficile* (for example clostridium toxins A or B and derivatives thereof); *Bacillus spp.,* including *B. anthracis* (for example botulinum toxin and derivatives thereof); *Corynebacterium spp.,* including *C. diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B*. *afzelii* (for example OspA, OspC, DbpA, DbpB), *B. andersonii* (for example OspA, OspC, DbpA, DbpB), *B. hermsii; Ehrlichia spp.,* including *E. equi* and the agent of the Human Granulocytic Ehrlichiosis; *Rickettsia spp,* including *R. rickettsii; Chlamydia spp., including C. trachomatis, C. pneumoniae, C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum* and *P. vivax; Toxoplasma spp.,* including *T. gondii (for example SAG2, SAG3, Tg34); Entamoeba spp.,* including *E. histolytica; Babesia spp.,* including *B. microti; Trypanosoma spp.,* including *T. cruzi; Giardia spp.,* including *G. lamblia; leishmania spp.,* including *L. major; Pneumocystis spp.,* including *P. carinii; Trichomonas spp.,* including *T. vaginalis; Schisostoma spp.,* including *S. mansoni,* or derived from yeast such as *Candida spp.,* including *C. albicans; Cryptococcus spp.,* including *C. neoformans.*

Further bacterial antigens include antigens derived from *Streptococcus spp,* including *S. pneumoniae* (PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other bacterial antigens include antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy variants or fusion proteins thereof.

The methods or compositions of the present invention are used to protect against or treat bacterial diseases such as those caused by *Mycobacterium tuberculosis* (TB).

### TB antigens

In the present invention, the pathogen is *Mycobacterium tuberculosis.*

Exemplary antigens derived from *M. tuberculosis* are for example alpha-crystallin (HspX), HBHA, Rv1753, Rv2386, Rv2707, Rv2557, Rv2558, RPFs: Rv0837c, Rv1884c, Rv2389c, Rv2450, Rv1009, aceA (Rv0467), ESAT6, Tb38-1, Ag85A, -B or -C, MPT 44, MPT59, MPT45, HSP10, HSP65, HSP70, HSP 75, HSP90, PPD 19kDa [Rv3763], PPD, 38kDa [Rv0934]), PstS1, (Rv0932), SodA (Rv3846), Rv2031c, 16kDa, Ra12, TbH9, Ra35, Tb38-1, Erd 14, DPV, MTI, MSL, DPPD, mTCC1, mTCC2, hTCC1 (WO 99/51748) and hTCC2, and especially Mtb32a, Ra35, Ra12, DPV, MSL, MTI, Tb38-1, mTCC1, TbH9 (Mtb39a), hTCC1, mTCC2 and DPPD. Antigens derived from *M. tuberculosis* also include fusion proteins and variants thereof where at least two, or for example, three polypeptides of *M. tuberculosis* are fused into a larger protein. Such fusions may comprise or consist of Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748), Ra12-Tbh9-Ra35-Ag85B and Ra12-Tbh9-Ra35- mTCC2. A particular Ra12-Tbh9-Ra35 sequence that may be mentioned is defined by SEQ ID No 6 of WO2006/117240 together with variants in which Ser 704 of that sequence is mutated to other than serine, eg to Ala, and derivatives thereof incorporating an N-terminal His tag of an appropriate length (eg SEQ ID No 2 or 4 of WO2006/117240). See also SEQ ID No 10 which is a sequence containing an optional starting M and an optional N-terminal His-His tag (positions 2 and 3) and in which the Ala mutated relative to the wild-type Ser is at position 706.

### Chlamydia antigens

In other examples, the pathogen may, for example, be a *Chlamydia sp.* eg *C trachomatis.*

Exemplary antigens derived from *Chlamydia sp* eg *C trachomatis* are selected from CT858, CT 089, CT875, MOMP, CT622, PmpD, PmpG and fragments thereof, SWIB and immunogenic fragments of any one thereof (such as PmpDpd and PmpGpd) and combinations thereof. Preferred combinations of antigens include CT858, CT089 and CT875. Specific sequences and combinations that may be employed are described in WO2006/104890.

### Plasmodium antigens

In other examples, the pathogen may, for example be a parasite that causes malaria such as a *Plasmodium sp.* eg *P falciparum* or *P vivax.*

For example, antigens derived from *P falciparum* include circumsporozoite protein (CS protein), PfEMP-1, Pfs 16 antigen, MSP-1, MSP-3, LSA-1, LSA-3, AMA-1 and TRAP. A particular hybrid antigen that may be mentioned is RTS. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. When expressed in yeast RTS is produced as a lipoprotein particle, and when it is co-expressed with the S antigen from HBV it produces a mixed particle known as RTS,S The structure or RTS and RTS,S is disclosed in WO 93/10152. TRAP antigens are described in WO 90/01496. Other *Plasmodium* antigens include *P. falciparum* EBA, GLURP, RAP1, RAP2, Sequestrin, Pf332, STARP, SALSA, PfEXP1, Pfs25, Pfs28, PFS27/25, Pfs48/45, Pfs230 and their analogues in other *Plasmodium* spp. One example disclosed herein is a composition comprising RTS,S or CS protein or a fragment thereof such as the CS portion of RTS, S in combination with one or more further malarial antigens which may be selected for example from the group consisting of MSP-1, MSP-3, AMA-1, Pfs 16, LSA-1 or LSA-3. Possible antigens from *P vivax* include circumsporozoite protein (CS protein) and Duffy antigen binding protein and immunogenic fragments thereof, such as PvRII (see eg WO02/12292).

In this example the first and second immunogenic polypeptides may be selected from antigens derived from *Plasmodium falciparum* and/or *Plasmodium vivax.*

For example, the first and/or second immunogenic polypeptides are selected from antigens derived from *Plasmodium falciparum* and/or *Plasmodium vivax* are selected from RTS (eg as RTS,S), circumsporozoite (CS) protein, MSP-1, MSP-3, AMA-1, LSA-1, LSA-3 and immunogenic derivatives thereof or immunogenic fragments thereof.

One specific derivative that may be mentioned is the hybrid protein known as RTS, especially when presented in the form of a mixed particle known as RTS,S.

An exemplary RTS sequence is shown in SEQ ID No 14.

An exemplary P. falciparum CS protein-derived antigen is shown in SEQ ID No 12. This particular sequence corresponds to the CSP sequence of *P.falciparum* (3D7 strain), which also contains a 19 aa insertion coming from 7G8 strain (81-100).

In another example a first immunogenic polypeptide is RTS,S and a second immunogenic polypeptide is the CS protein from *Plasmodium falciparum* or an immunogenic fragment thereof.

### HPV antigens

In other examples, the pathogen may, for example, be a Human Papilloma Virus.

Thus antigens of use may, for example, be derived from the Human Papilloma Virus (HPV) considered to be responsible for genital warts (HPV 6 or HPV 11 and others), and/or the HPV viruses responsible for cervical cancer (HPV16, HPV18, HPV33, HPV51, HPV56, HPV31, HPV45, HPV58, HPV52 and others). In one example the forms of genital wart prophylactic, or therapeutic, compositions comprise L1 particles or capsomers, and fusion proteins comprising one or more antigens selected from the HPV proteins E1, E2, E5 E6, E7, L1, and L2. The forms of fusion protein may be: L2E7 as disclosed in WO96/26277, and proteinD (1/3)-E7 disclosed in PCT/EP98/05285.

A preferred HPV cervical infection or cancer, prophylaxis or therapeutic composition may comprise HPV 16 or 18 antigens. For example, L1 or L2 antigen monomers, or L1 or L2 antigens presented together as a virus like particle (VLP) or the L1 alone protein presented alone in a VLP or capsomer structure. Such antigens, virus like particles and capsomer are per se known. See for example WO94/00152, WO94/20137, WO94/05792, and WO93/02184. Additional early proteins may be included alone or as fusion proteins such as E7, E2 or preferably E5 for example; particularly preferred disclosure of this includes a VLP comprising L1E7 fusion proteins (WO 96/11272). In one example the HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277). Alternatively the HPV 16 or 18 early proteins E6 and E7, may be presented in a single molecule, preferably a Protein D- E6/E7 fusion. Such a composition may optionally provide either or both E6 and E7 proteins from HPV 18, preferably in the form of a Protein D - E6 or Protein D - E7 fusion protein or Protein D E6/E7 fusion protein. Additionally antigens from other HPV strains, preferably from strains HPV 31 or 33 may be employed.

### HIV antigens

In other examples the pathogen may, for example, be HIV eg HIV-1.

Thus, antigens may be selected from HIV derived antigens, particularly HIV-1 derived antigens.

HIV Tat and Nef proteins are early proteins, that is, they are expressed early in infection and in the absence of structural protein.

The Nef gene encodes an early accessory HIV protein which has been shown to possess several activities. For example, the Nef protein is known to cause the removal of CD4, the HIV receptor, from the cell surface, although the biological importance of this function is debated. Additionally Nef interacts with the signal pathway of T cells and induces an active state, which in turn may promote more efficient gene expression. Some HIV isolates have mutations or deletions in this region, which cause them not to encode functional protein and are severely compromised in their replication and pathogenesis in vivo.

The Gag gene is translated from the full-length RNA to yield a precursor polyprotein which is subsequently cleaved into 3 - 5 capsid proteins; the matrix protein p17, capsid protein p24 and nucleic acid binding protein (Fundamental Virology, Fields BN, Knipe DM and Howley M 1996 2. Fields Virology vol 2 1996).

The Gag gene gives rise to the 55-kilodalton (Kd) Gag precursor protein, also called p55, which is expressed from the unspliced viral mRNA. During translation, the N terminus of p55 is myristoylated, triggering its association with the cytoplasmic aspect of cell membranes. The membrane-associated Gag polyprotein recruits two copies of the viral genomic RNA along with other viral and cellular proteins that triggers the budding of the viral particle from the surface of an infected cell. After budding, p55 is cleaved by the virally encoded protease (a product of the Pol gene) during the process of viral maturation into four smaller proteins designated MA (matrix [p17]), CA (capsid [p24]), NC (nucleocapsid [p9]), and p6.(4).

In addition to the 3 major Gag proteins (p17, p24 and p9), all Gag precursors contain several other regions, which are cleaved out and remain in the virion as peptides of various sizes. These proteins have different roles e.g. the p2 protein has a proposed role in regulating activity of the protease and contributes to the correct timing of proteolytic processing.

The MA polypeptide is derived from the N-terminal, myristoylated end of p55. Most MA molecules remain attached to the inner surface of the virion lipid bilayer, stabilizing the particle. A subset of MA is recruited inside the deeper layers of the virion where it becomes part of the complex which escorts the viral DNA to the nucleus. These MA molecules facilitate the nuclear transport of the viral genome because a karyophilic signal on MA is recognized by the cellular nuclear import machinery. This phenomenon allows HIV to infect non-dividing cells, an unusual property for a retrovirus.

The p24 (CA) protein forms the conical core of viral particles. Cyclophilin A has been demonstrated to interact with the p24 region of p55 leading to its incorporation into HIV particles. The interaction between Gag and cyclophilin A is essential because the disruption of this interaction by cyclosporine inhibits viral replication.

The NC region of Gag is responsible for specifically recognizing the so-called packaging signal of HIV. The packaging signal consists of four stem loop structures located near the 5' end of the viral RNA, and is sufficient to mediate the incorporation of a heterologous RNA into HIV-1 virions. NC binds to the packaging signal through interactions mediated by two zinc-finger motifs. NC also facilitates reverse transcription.

The p6 polypeptide region mediates interactions between p55 Gag and the accessory protein Vpr, leading to the incorporation of Vpr into assembling virions. The p6 region also contains a so-called late domain which is required for the efficient release of budding virions from an infected cell.

The Pol gene encodes three proteins having the activities needed by the virus in early infection, reverse transcriptase RT, protease, and the integrase protein needed for integration of viral DNA into cellular DNA. The primary product of Pol is cleaved by the virion protease to yield the amino terminal RT peptide which contains activities necessary for DNA synthesis (RNA and DNA directed DNA polymerase, ribonuclease H) and carboxy terminal integrase protein. HIV RT is a heterodimer of full-length RT (p66) and a cleavage product (p51) lacking the carboxy terminal RNase H domain.

RT is one of the most highly conserved proteins encoded by the retroviral genome. Two major activities of RT are the DNA Pol and ribonuclease H. The DNA Pol activity of RT uses RNA and DNA as templates interchangeably and like all DNA polymerases known is unable to initiate DNA synthesis de novo, but requires a pre existing molecule to serve as a primer (RNA).

The RNase H activity inherent in all RT proteins plays the essential role early in replication of removing the RNA genome as DNA synthesis proceeds. It selectively degrades the RNA from all RNA - DNA hybrid molecules. Structurally the polymerase and ribo H occupy separate, non-overlapping domains within the Pol covering the amino two thirds of the Pol.

The p66 catalytic subunit is folded into 5 distinct subdomains. The amino terminal 23 of these have the portion with RT activity. Carboxy terminal to these is the RNase H domain.

After infection of the host cell, the retroviral RNA genome is copied into linear double stranded DNA by the reverse transcriptase that is present in the infecting particle. The integrase (reviewed in Skalka AM '99 Adv in Virus Res 52 271-273) recognises the ends of the viral DNA, trims them and accompanies the viral DNA to a host chromosomal site to catalyse integration. Many sites in the host DNA can be targets for integration. Although the integrase is sufficient to catalyse integration in vitro, it is not the only protein associated with the viral DNA in vivo - the large protein - viral DNA complex isolated from the infected cells has been denoted the pre integration complex. This facilitates the acquisition of the host cell genes by progeny viral genomes.

The integrase is made up of 3 distinct domains, the N terminal domain, the catalytic core and the C terminal domain. The catalytic core domain contains all of the requirements for the chemistry of polynucleotidyl transfer.

HIV-1 derived antigens may thus for example be selected from Gag (for example full length Gag), p17 (a portion of Gag), p24 (another portion of Gag), p41, p40, Pol (for example full length Pol), RT (a portion of Pol), p51 (a portion of RT), integrase (a portion of Pol), protease (a portion of Pol), Env, gp120, gp140 or gp160, gp41, Nef, Vif, Vpr, Vpu, Rev, Tat and immunogenic derivatives thereof and immunogenic fragments thereof, particularly Env, Gag, Nef and Pol and immunogenic derivatives thereof and immunogenic fragments thereof including p17, p24, RT and integrase. HIV vaccines may comprise polypeptides and/or polynucleotides encoding polypeptides corresponding to multiple different HIV antigens for example 2 or 3 or 4 or more HIV antigens which may be selected from the above list. Several different antigens may, for example, be comprised in a single fusion protein. More than one first immunogenic polypeptide and/or more than one second immunogenic polypeptide each of which is an HIV antigen or a fusion of more than one antigen may be employed.

For example an antigen may comprise Gag or an immunogenic derivative or immunogenic fragment thereof, fused to RT or an immunogenic derivative or immunogenic fragment thereof, fused to Nef or an immunogenic derivative or immunogenic fragment thereof wherein the Gag portion of the fusion protein is present at the 5' terminus end of the polypeptide.

A Gag sequence may exclude the Gag p6 polypeptide encoding sequence. A particular example of a Gag sequence comprises p17 and/or p24 encoding sequences.

A RT sequence may contain a mutation to substantially inactivate any reverse transcriptase activity (see WO03/025003).

The RT gene is a component of the bigger *pol* gene in the HIV genome. It will be understood that the RT sequence employed according to the present disclosure may be present in the context of Pol, or a fragment of Pol corresponding at least to RT. Such fragments of Pol retain major CTL epitopes of Pol. In one specific example, RT is included as just the p51 or just the p66 fragment of RT.

The RT component of the fusion protein or composition optionally comprises a mutation to remove a site which serves as an internal initiation site in prokaryotic expression systems.

Optionally the Nef sequence is truncated to remove the sequence encoding the N terminal region i.e. removal of from 30 to 85 amino acids, for example from 60 to 85 amino acids, particularly the N terminal 65 amino acids (the latter truncation is referred to herein as trNef). Alternatively or additionally the Nef may be modified to remove the myristylation site. For example the Gly 2 myristylation site may be removed by deletion or substitution. Alternatively or additionally the Nef may be modified to alter the dileucine motif of Leu 174 and Leu 175 by deletion or substitution of one or both leucines. The importance of the dileucine motif in CD4 downregulation is described e.g. in Bresnahan P.A. et al (1998) Current Biology, 8(22): 1235-8.

The Env antigen may be present in its full length as gp160 or truncated as gp140 or shorter (optionally with a suitable mutation to destroy the cleavage site motif between gp120 and gp41). The Env antigen may also be present in its naturally occurring processed form as gp120 and gp41. These two derivatives of gp160 may be used individually or together as a combination. The aforementioned Env antigens may further exhibit deletions (in particular of variable loops) and truncations. Fragments of Env may be used as well.

An exemplary gp120 sequence is shown in SEQ ID No 8. An exemplary gp140 sequence is shown in SEQ ID No 6.

Immunogenic polypeptides according to the disclosure may comprise Gag, Pol, Env and Nef wherein at least 75%, or at least 90% or at least 95%, for example, 96% of the CTL epitopes of these native antigens are present.

In immunogenic polypeptides according to the disclosure which comprise p17/p24 Gag, p66 RT, and truncated Nef as defined above, 96% of the CTL epitopes of the native Gag, Pol and Nef antigens are suitably present.

One example provides an immunogenic polypeptide containing p17, p24 Gag, p66 RT , truncated Nef (devoid of nucleotides encoding terminal amino-acids 1-85 - "trNef") in the order Gag, RT, Nef. In polynucleotides encoding immunogenic polypeptides, suitably the P24 Gag and P66 RT are codon optimized.

Specific polynucleotide constructs and corresponding polypeptide antigens according to the present disclosure include:
1. p17, p24 (codon optimised) Gag - p66 RT (codon optimised) - truncated Nef;
2. truncated Nef - p66 RT (codon optimised) - p17, p24 (codon optimised) Gag;
3. truncated Nef - p17, p24 (codon optimised) Gag - p66 RT (codon optimised);
4. p66 RT (codon optimised) - p17, p24 (codon optimised) Gag - truncated Nef;
5. p66 RT (codon optimised) - truncated Nef - p17, p24 (codon optimised) Gag;
6. p17, p24 (codon optimised) Gag - truncated Nef - p66 RT (codon optimised).

An exemplary fusion is a fusion of Gag, RT and Nef particularly in the order Gag-RT-Nef (see eg SEQ ID No 2). Another exemplary fusion is a fusion of p17, p24, RT and Nef particularly in the order p24-RT-Nef-p17 (see eg SEQ ID No 16, referred to elsewhere herein as "F4").

In one example an immunogenic polypeptide contains Gag, RT, integrase and Nef, especially in the order Gag-RT-integrase-Nef (see eg SEQ ID No 4).

In other examples the HIV antigen may be a fusion polypeptide which comprises Nef or an immunogenic derivative thereof or an immunogenic fragment thereof, and p17 Gag and/or p24 Gag or immunogenic derivatives thereof or immunogenic fragments thereof, wherein when both p17 and p24 Gag are present there is at least one HIV antigen or immunogenic fragment between them.

For example, Nef is suitably full length Nef.

For example p17 Gag and p24 Gag are suitably full length p17 and p24 respectively.

In one example an immunogenic polypeptide comprises both p17 and p24 Gag or immunogenic fragments thereof. In such a construct the p24 Gag component and p17 Gag component are separated by at least one further HIV antigen or immunogenic fragment, such as Nef and/or RT or immunogenic derivatives thereof or immunogenic fragments thereof. See WO2006/013106 for further details.

In fusion proteins which comprise p24 and RT, it may be preferable that the p24 precedes the RT in the construct because when the antigens are expressed alone in *E. coli* better expression of p24 than of RT is observed.

Some exemplary constructs according to the disclosure include the following:
1. p24 - RT - Nef - p17
2. p24 - RT* - Nef - p17
3. p24 - p51RT - Nef - p17
4. p24 - p51RT* - Nef - p17
5. p17 - p51RT - Nef
6. p17 - p51RT* - Nef
7. Nef - p17
8. Nef - p17 with linker
9. p17 - Nef
10. p17 - Nef with linker
   * represents RT methionine₅₉₂ mutation to lysine

Also disclosed is a fusion protein of HIV antigens comprising at least four HIV antigens or immunogenic fragments, wherein the four antigens or fragments are or are derived from Nef, Pol and Gag. Preferably Gag is present as two separate components which are separated by at least one other antigen in the fusion. Preferably the Nef is full length Nef. Preferably the Pol is p66 or p51RT. Preferably the Gag is p17 and p24 Gag. Other preferred features and properties of the antigen components of the fusion are as described herein.

Preferred examples of this aspect of the disclosure are the four component fusions as already listed above:
1. p24 - RT - Nef - p17
2. p24 - RT* - Nef - p17
3. p24 - p51RT - Nef - p17
4. p24 - p51RT* - Nef - p17

The immunogenic polypeptides of the present disclosure may have linker sequences present in between the sequences corresponding to particular antigens such as Gag, RT and Nef. Such linker sequences may be, for example, up to 20 amino acids in length. In a particular example they may be from 1 to 10 amino acids, or from 1 to 6 amino acids, for example 4-6 amino acids.

Further description of such suitable HIV antigens can be found in WO03/025003.

HIV antigens of the present disclosure may be derived from any HIV clade, for example clade A, clade B or clade C. For example the HIV antigens may be derived from clade A or B, especially B .

In one specific example a first immunogenic polypeptide is a polypeptide comprising Gag and/or Pol and/or Nef or a fragment or derivative of any of them (eg p24-RT-Nef-p17) . In one specific example a second immunogenic polypeptide is a polypeptide comprising Gap and/or Pol and/or Nef or a fragment or derivative of any of them (eg Gag-RT-Nef or Gag-RT-integrase-Nef).

Thus in one specific example, a polypeptide comprising Gap and/or Pol and/or Nef or a fragment or derivative of any of them (eg p24-RT-Nef-p17) is a first immunogenic polypeptide and a polypeptide comprising Gap and/or Pol and/or Nef or a fragment or derivative of any of them (eg Gag-RT-Nef or Gag-RT-integrase-Nef) is a second immunogenic polypeptide.

In another specific example, a first immunogenic polypeptide is Env or a fragment or derivative thereof eg gp120, gp140 or gp160 (especially gp120). In one specific example a second immunogenic polypeptide is a polypeptide comprising Gag and/or Pol and/or Nef or a fragment or derivative of any of them (eg p24-RT-Nef-p17).

Thus in one specific example, Env or a fragment or derivative thereof eg gp120, gp140 or gp160 (especially gp120) is a first immunogenic polypeptide and a polypeptide comprising Gag and/or Pol and/or Nef or a fragment or derivative of any of them (eg p24-RT-Nef-p17) is a second immunogenic polypeptide.

In another specific example, a first immunogenic polypeptide is a polypeptide comprising Gag and/or Pol and/or Nef or a fragment or derivative of any of them (eg p24-RT-Nef-p17). In one specific example a second immunogenic polypeptide is Env or a fragment or derivative thereof eg gp120, gp140 or gp160 (especially gp120).

Thus in one specific example, a polypeptide comprising Gag and/or Pol and/or Nef or a fragment or derivative of any of them (eg p24-RT-Nef-p17) is a first immunogenic polypeptide and Env or a fragment or derivative thereof eg gp120, gp140 or gp160 (especially gp120) is a second immunogenic polypeptide.

### Immunogenic derivatives and immunogenic fragments of antigens

The aforementioned antigens may be employed in the form of immunogenic derivatives or immunogenic fragments thereof rather than the whole antigen.

As used herein the term "immunogenic derivative" in relation to an antigen of native origin refers to an antigen that have been modified in a limited way relative to its native counterparts. For example it may include a point mutation which may change the properties of the protein eg by improving expression in prokaryotic systems or by removing undesirable activity, eg enzymatic activity. Immunogenic derivatives will however be sufficiently similar to the native antigens such that they retain their antigenic properties and remain capable of raising an immune response against the native antigen. Whether or not a given derivative raises such an immune response may be measured by a suitably immunological assay such as an ELISA (for antibody responses) or flow cytometry using suitable staining for cellular markers (for cellular responses).

Immunogenic fragments are fragments which encode at least one epitope, for example a CTL epitope, typically a peptide of at least 8 amino acids. Fragments of at least 8, for example 8-10 amino acids or up to 20, 50, 60, 70, 100, 150 or 200 amino acids in length are considered to fall within the scope of the disclosure as long as the polypeptide demonstrates antigenicity, that is to say that the major epitopes (eg CTL epitopes)are retained by the polypeptide.

### Adenovirus

Adenoviral vectors of the present invention comprise one or more heterologous polynucleotides (DNA) which encode one or more immunogenic polypeptides.

Adenoviral vectors of use in the present invention may be derived from a range of mammalian hosts.

Adenoviruses (herein referred to as "Ad" or "Adv") have a characteristic morphology with an icosohedral capsid consisting of three major proteins, hexon (II), penton base (III) and a knobbed fibre (IV), along with a number of other minor proteins, VI, VIII, IX, IIIa and IVa2 (Russell W.C. 2000, Gen Viriol, 81:2573-2604). The virus genome is a linear, doublestranded DNA with a terminal protein attached covalently to the 5' termini, which have inverted terminal repeats (ITRs). The virus DNA is intimately associated with the highly basic protein VII and a small peptide termed mu. Another protein, V, is packaged with this DNA-protein complex and provides a structural link to the capsid via protein VI. The virus also contains a virus-encoded protease, which is necessary for processing of some of the structural proteins to produce mature infectious virus.

Over 100 distinct serotypes of adenovirus have been isolated which infect various mammalian species, 51 of which are of human origin. Thus one or more of the adenoviral vectors may be derived from a human adenovirus. Examples of such human-derived adenoviruses are Ad1, Ad2, Ad4, Ad5, Ad6, Ad11, Ad 24, Ad34, Ad35, particularly Ad5, Ad11 and Ad35. The human serotypes have been categorised into six subgenera (A-F) based on a number of biological, chemical, immunological and structural criteria.

Although Ad5-based vectors have been used extensively in a number of gene therapy trials, there may be limitations on the use of Ad5 and other group C adenoviral vectors due to preexisting immunity in the general population due to natural infection. Ad5 and other group C members tend to be among the most seroprevalent serotypes. Immunity to existing vectors may develop as a result of exposure to the vector during treatment. These types of preexisting or developed immunity to seroprevalent vectors may limit the effectiveness of gene therapy or vaccination efforts. Alternative adenovirus serotypes, thus constitute very important targets in the pursuit of gene delivery systems capable of evading the host immune response.

One such area of alternative serotypes are those derived from non human primates, especially chimpanzee adenoviruses. See US Patent 6,083,716 which describes the genome of two chimpanzee adenoviruses.

It has been shown that chimpanzee ("Pan" or "C") adenoviral vectors induce strong immune responses to transgene products as efficiently as human adenoviral vectors (Fitzgerald et al. J. Immunol. 170:1416).

Non human primate adenoviruses can be isolated from the mesenteric lymph nodes of chimpanzees. Chimpanzee adenoviruses are sufficiently similar to human adenovirus subtype C to allow replication of E1 deleted virus in HEK 293 cells. Yet chimpanzee adenoviruses are phylogenetically distinct from the more common human serotypes (Ad2 and Ad5). Pan 6 is less closely related to and is serologically distinct from Pans 5, 7 and 9.

Thus one or more of the adenoviral vectors may be derived from a non-human primate adenovirus eg a chimpanzee adenovirus such as one selected from serotypes Pan5, Pan6, Pan7 and Pan9.

Adenoviral vectors may also be derived from more than one adenovirus serotype, and each serotype may be from the same or different source. For example they may be derived from more than one human serotype and/or more than one non-human primate serotype. Methods for constructing chimeric adenoviral vectors are disclosed in WO2005/001103.

There are certain size restrictions associated with inserting heterologous DNA into adenoviruses. Human adenoviruses have the ability to package up to 105% of the wild type genome length (Bett et al 1993, J Virol 67 (10), 5911-21). The lower packaging limit for human adenoviruses has been shown to be 75% of the wild type genome length (Parks et al 1995, J Virol 71(4), 3293-8).

One example of adenoviruses of use in the present invention are adenoviruses which are distinct from prevalent naturally occurring serotypes in the human population such as Ad2 and Ad5. This avoids the induction of potent immune responses against the vector which limits the efficacy of subsequent administrations of the same serotype by blocking vector uptake through neutralizing antibody and influencing toxicity.

Thus, the adenovirus may be an adenovirus which is not a prevalent naturally occurring human virus serotype. Adenoviruses isolated from animals have immunologically distinct capsid, hexon, penton and fibre components but are phylogenetically closely related. Specifically, the virus may be a non-human adenovirus, such as a simian adenovirus and in particular a chimpanzee adenovirus such as Pan 5, 6, 7 or 9. Examples of such strains are described in WO03/000283 and are available from the American Type Culture Collection, 10801 University Boulevard, Manassas, Virginia 20110-2209, and other sources. Desirable chimpanzee adenovirus strains are Pan 5 [ATCC VR-591], Pan 6 [ATCC VR-592], and Pan 7 [ATCC VR-593].

Use of chimpanzee adenoviruses is thought to be advantageous over use of human adenovirus serotypes because of the lack of pre-existing immunity, in particular the lack of cross-neutralising antibodies, to adenoviruses in the target population. Cross-reaction of the chimpanzee adenoviruses with pre-existing neutralizing antibody responses is only present in 2% of the target population compared with 35% in the case of certain candidate human adenovirus vectors. The chimpanzee adenoviruses are distinct from the more common human subtypes Ad2 and Ad5, but are more closely related to human Ad4 of subgroup E, which is not a prevalent subtype. Pan 6 is less closely related to Pan 5, 7 and 9.

The adenovirus of the invention may be replication defective. This means that it has a reduced ability to replicate in non-complementing cells, compared to the wild type virus. This may be brought about by mutating the virus e.g. by deleting a gene involved in replication, for example deletion of the E1a, E1b, E3 or E4 gene.

The adenoviral vectors in accordance with the present invention may be derived from replication defective adenovirus comprising a functional E1 deletion. Thus the adenoviral vectors according to the invention may be replication defective due to the absence of the ability to express adenoviral E1a and E1b, i.e., are functionally deleted in E1a and E1b. The recombinant adenoviruses may also bear functional deletions in other genes [see WO 03/000283] for example, deletions in E3 or E4 genes. The adenovirus delayed early gene E3 may be eliminated from the adenovirus sequence which forms part of the recombinant virus. The function of E3 is not necessary to the production of the recombinant adenovirus particle. Thus, it is unnecessary to replace the function of this gene product in order to package a recombinant adenovirus useful in the invention. In one particular embodiment the recombinant adenoviruses have functionally deleted E1 and E3 genes. The construction of such vectors is described in Roy et al., Human Gene Therapy 15:519-530, 2004.

Recombinant adenoviruses may also be constructed having a functional deletion of the E4 gene, although it may be desirable to retain the E4 ORF6 function. Adenovirus vectors according to the invention may also contain a deletion in the delayed early gene E2a. Deletions may also be made in any of the late genes L1 through to L5 of the adenovirus genome. Similarly deletions in the intermediate genes IX and IVa may be useful.

Other deletions may be made in the other structural or non-structural adenovirus genes. The above deletions may be used individually, i.e. an adenovirus sequence for use in the present invention may contain deletions of E1 only. Alternatively, deletions of entire genes or portions thereof effective to destroy their biological activity may be used in any combination. For example in one exemplary vector, the adenovirus sequences may have deletions of the E1 genes and the E4 gene, or of the E1, E2a and E3 genes, or of the E1 and E3 genes (such as functional deletions in E1a and E1b, and a deletion of at least part of E3), or of the E1, E2a and E4 genes, with or without deletion of E3 and so on. Such deletions may be partial or full deletions of these genes and may be used in combination with other mutations, such as temperature sensitive mutations to achieve a desired result.

The adenoviral vectors can be produced on any suitable cell line in which the virus is capable of replication. In particular, complementing cell lines which provide the factors missing from the viral vector that result in its impaired replication characteristics (such as E1 and/or E4) can be used. Without limitation, such a cell line may be HeLa [ATCC Accession No. CCL 2], A549 [ATCC Accession No. CCL 185], HEK 293, KB [CCL 17], Detroit [e.g., Detroit 510, CCL 72] and WI-38 [CCL 75] cells, among others. These cell lines are all available from the American Type Culture Collection, 10801 University Boulevard, Manassas, Virginia 20110-2209. Other suitable parent cell lines may be obtained from other sources, such as PER.C6© cells, as represented by the cells deposited under ECACC no. 96022940 at the European Collection of Animal Cell Cultures (ECACC) at the Centre for Applied Microbiology and Research (CAMR, UK) or Her 96 cells (Crucell).

The polynucleotide sequences which encode immunogenic polypeptides may be codon optimised for mammalian cells. Such codon-optimisation is described in detail in WO05/025614. Codon optimization for certain HIV sequences is further described in WO 03/025003.

In one embodiment of the present invention the polynucleotide constructs comprise an N-terminal leader sequence. The signal sequence, transmembrane domain and cytoplasmic domain are individually all optionally present or deleted. In one embodiment of the present invention all these regions are present but modified.

A promoter for use in the adenoviral vector according to the invention may be the promoter from HCMV IE gene, for example wherein the 5' untranslated region of the HCMV IE gene comprising exon 1 is included and intron A is completely or partially excluded as described in WO 02/36792.

When several antigens are fused into a fusion protein, such protein would be encoded by a polynucleotide under the control of a single promoter.

In an alternative embodiment of the invention, several antigens may be expressed separately through individual promoters, each of said promoters may be the same or different. In yet another embodiment of the invention some of the antigens may form a fusion, linked to a first promoter and other antigen(s) may be linked to a second promoter, which may be the same or different from the first promoter.

Thus, the adenoviral vector may comprise one or more expression cassettes each of which encode one antigen under the control of one promoter. Alternatively or additionally it may comprise one or more expression cassettes each of which encode more than one antigen under the control of one promoter, which antigens are thereby expressed as a fusion. Each expression cassette may be present in more than one locus in the adenoviral vector.

The polynucleotide or polynucleotides encoding immunogenic polypeptides to be expressed may be inserted into any of the adenovirus deleted regions, for example into the E1 deleted region.

Although two or more polynucleotides encoding immunogenic polypeptides may be linked as a fusion, the resulting protein may be expressed as a fusion protein, or it may be expressed as separate protein products, or it may be expressed as a fusion protein and then subsequently broken down into smaller subunits.

### Adjuvant

Adjuvants are described in general in Vaccine Design - the Subunit and Adjuvant Approach eg Powell and Newman, Plenum Press, New York, 1995.

Adjuvants may include an aluminium salt such as aluminium hydroxide or aluminium phosphate, but may also be a salt of calcium, iron or zinc, or may be an insoluble suspension of acylated tyrosine, or acylated sugars, cationically or anionically derivatised polysaccharides, or polyphosphazenes.

In the formulation of the invention it is preferred that the adjuvant composition preferentially induces a Th1 response. However it will be understood that other responses, including other humoral responses, are not excluded.

It is known that certain vaccine adjuvants are particularly suited to the stimulation of either Th1 or Th2 - type cytokine responses. Traditionally the best indicators of the Th1:Th2 balance of the immune response after a vaccination or infection includes direct measurement of the production of Th1 or Th2 cytokines by T lymphocytes *in vitro* after restimulation with antigen, and/or the measurement of the lgG1:lgG2a ratio of antigen specific antibody responses.

Thus, a Th1-type adjuvant is one which stimulates isolated T-cell populations to produce high levels of Th1-type cytokines *in vivo* (as measured in the serum) or *ex vivo* (cytokines that are measured when the cells are re-stimulated with antigen *in vitro*), and induces antigen specific immunoglobulin responses associated with Th1-type isotype.

Preferred Th1-type immunostimulants which may be formulated to produce adjuvants suitable for use in the present invention include the following:
The Toll like receptor (TLR) 4 ligands, especially an agonist such as a lipid A derivative particularly monophosphoryl lipid A or more particularly 3 Deacylated monophoshoryl lipid A (3 D - MPL).

3 D -MPL is sold under the trademark MPL® by GlaxoSmithKline and primarily promotes CD4+ T cell responses characterized by the production of IFN-g (Th1 cells i.e. CD4 T helper cells with a type-1 phenotype). It can be produced according to the methods disclosed in GB 2 220 211 A. Chemically it is a mixture of 3-deacylated monophosphoryl lipid A with 3, 4, 5 or 6 acylated chains. Preferably in the compositions of the present invention small particle 3 D- MPL is used. Small particle 3 D -MPL has a particle size such that it may be sterile-filtered through a 0.22µm filter. Such preparations are described in International Patent Application No. WO94/21292. Synthetic derivatives of lipid A are known and thought to be TLR 4 agonists including, but not limited to:
**OM174** (2-deoxy-6-o-[2-deoxy-2-[(R)-3-dodecanoyloxytetra-decanoylamino]-4-o-phosphono-β-D-glucopyranosyl]-2-[(R)-3-hydroxytetradecanoylamino]-α-D-glucopyranosyldihydrogenphosphate), (WO 95/14026)
**OM 294** DP (3S, 9 R) -3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO99 /64301 and WO 00/0462)
**OM 197** MP-Ac DP (3S-, 9R) -3-[(R) -dodecanoyloxytetradecanoylamino]-4-oxo-5-aza-9-[(R)-3-hydroxytetradecanoylamino]decan-1,10-diol,1 -dihydrogenophosphate 10-(6-aminohexanoate) (WO 01/46127)

Other TLR4 ligands are alkyl Glucosaminide phosphates (AGPs) such as those disclosed in WO9850399 or US6303347 (processes for preparation of AGPs are also disclosed), or pharmaceutically acceptable salts of AGPs as disclosed in US6764840. Some AGPs are TLR4 agonists, and some are TLR4 antagonists. Both are thought to be useful as adjuvants.

Saponins are also preferred Th1 immunostimulants in accordance with the invention. Saponins are well known adjuvants and are taught in: Lacaille-Dubois, M and Wagner H. (1996. A review of the biological and pharmacological activities of saponins. Phytomedicine vol 2 pp 363-386). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1. The haemolytic saponins QS21 and QS17 (HPLC purified fractions of Quil A) have been described as potent systemic adjuvants, and the method of their production is disclosed in US Patent No. 5,057,540 and EP 0 362 279 B1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711. One such system is known as an Iscom and may contain one or more saponins.

The adjuvant of the present invention comprises a Toll like receptor (TLR) 4 ligand, especially 3D-MPL, in combination with a saponin derivative, especially QS21..

In other examples, adjuvants include TLR 9 ligands (agonists). Thus another preferred immunostimulant is an immunostimulatory oligonucleotide containing unmethylated CpG dinucleotides ("CpG"). CpG is an abbreviation for cytosine-guanosine dinucleotide motifs present in DNA. CpG is known in the art as being an adjuvant when administered by both systemic and mucosal routes (WO 96/02555, EP 468520, Davis et al., J.Immunol, 1998, 160(2):870-876; McCluskie and Davis, J.Immunol., 1998, 161(9):4463-6). Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the CG motif is not methylated, but other unmethylated CpG sequences are known to be immunostimulatory and may be used in the present disclosure.

In certain combinations of the six nucleotides a palindromic sequence is present. Several of these motifs, either as repeats of one motif or a combination of different motifs, can be present in the same oligonucleotide. The presence of one or more of these immunostimulatory sequences containing oligonucleotides can activate various immune subsets, including natural killer cells (which produce interferon y and have cytolytic activity) and macrophages (Wooldrige et al Vol 89 (no. 8), 1977). Other unmethylated CpG containing sequences not having this consensus sequence have also now been shown to be immunomodulatory.

CpG when formulated into vaccines, is generally administered in free solution together with free antigen (WO 96/02555; McCluskie and Davis, *supra*) or covalently conjugated to an antigen (WO 98/16247), or formulated with a carrier such as aluminium hydroxide ((Hepatitis surface antigen) Davis *et al. supra* ; Brazolot-Millan et al., Proc.Natl.Acad.Sci., USA, 1998, 95(26), 15553-8).

In other examples, other TLR9 agonists of potential interest include immunostimulatory CpR motif containing oligonucleotides and YpG motif containing oligonucleotides (Idera).

Such immunostimulants as described above may be formulated together with carriers, such as for example liposomes, oil in water emulsions, and or metallic salts, including aluminium salts (such as aluminium hydroxide). For example, 3D-MPL may be formulated with aluminium hydroxide (EP 0 689 454) or oil in water emulsions (WO 95/17210); QS21 may be advantageously formulated with cholesterol containing liposomes (WO 96/33739), oil in water emulsions (WO 95/17210) or alum (WO 98/15287); CpG may be formulated with alum (Davis *et al. supra* ; Brazolot-Millan *supra*) or with other cationic carriers.

Combinations of immunostimulants are also preferred, in particular a combination of a monophosphoryl lipid A and a saponin derivative (WO 94/00153; WO 95/17210; WO 96/33739; WO 98/56414; WO 99/12565; WO 99/11241), more particularly the combination of QS21 and 3D-MPL as disclosed in WO 94/00153. Alternatively, a combination of CpG plus a saponin such as QS21 also forms a potent adjuvant for use in the present disclosure. Alternatively the saponin may be formulated in a liposome or in an Iscorn and combined with an immunostimulatory oligonucleotide.

Thus, suitable adjuvant systems include, for example, a combination of monophosphoryl lipid A, preferably 3D-MPL, together with an aluminium salt (eg as described in WO00/23105).

An enhanced system involves the combination of a monophosphoryl lipid A and a saponin derivative particularly the combination of QS21 and 3D-MPL as disclosed in WO 94/00153, or a less reactogenic composition where the QS21 is quenched in cholesterol containing liposomes (DQ) as disclosed in WO 96/33739. This combination may additionally comprise an immunostimulatory oligonucleotide.

Thus an example adjuvant comprises QS21 and MPL.

A particularly potent adjuvant formulation involving QS21, 3D-MPL & tocopherol in an oil in water emulsion is described in WO 95/17210 and is another preferred formulation for use in the invention.

Another preferred formulation comprises a CpG oligonucleotide alone or together with an aluminium salt.

In a further aspect of the present disclosure there is provided a method of manufacture of a vaccine formulation as herein described, wherein the method comprises admixing one or more first immunogenic polypeptides according to the invention with a suitable adjuvant.

Particularly preferred adjuvants for use in the formulations according to the invention are as follows:
i) 3D-MPL + QS21 in a liposome (see eg Adjuvant B below)
ii) Alum + QS21 in a liposome + 3D-MPL
iii) 3D-MPL + QS21 + oil in water emulsion
iv) 3D-MPL + QS21 (eg in a liposome) + CpG

Particularly preferred adjuvants for use in the formulations according to the disclosure are as follows:
v) Alum + 3D-MPL
vi) Alum + CpG
vii) CpG
viii) QS21 + CpG.

Preferably, the adjuvant is presented in the form of a liposome, ISCOM or an oil-in-water emulsion. In one example embodiment of the invention the adjuvant comprises an oil-in-water emulsion. In another example embodiment of the invention the adjuvant comprises liposomes.

Suitably the adjuvant component does not contain any virus. Thus suitably, compositions for use according to the invention do not contain any virus other than the one or more more adenoviral vectors comprising one or more heterologous polynucleotides encoding one or more second immunogenic polypeptides derived from a pathogen.

### Compositions, dosage and administration

In methods of the disclosure, the immunogenic polypeptide(s), the adenoviral vector(s) and the adjuvant are administered concomitantly.

Typically the adjuvant will be co-formulated with an immunogenic polypeptide. Suitably the adjuvant will also be co-formulated with any other immunogenic polypeptide to be administered.

A method of raising an immune response comprises administering (i) one or more first immunogenic polypeptides co-formulated with an adjuvant; and (ii) one or more adenoviral vectors comprising one or more heterologous polynucleotides encoding one or more second immunogenic polypeptides; wherein one or more first immunogenic polypeptides and adjuvant, and one or more adenoviral vectors are administered concomitantly.

By "co-formulated" is meant that the first immunogenic polypeptide and the adjuvant are contained within the same composition eg a pharmaceutical composition.

Typically the adenoviral vector is contained in a composition eg a pharmaceutical composition.

Alternatively, the one or more first immunogenic polypeptides, the one or more adenoviral vectors and an adjuvant are co-formulated.

Thus, there are provided compositions according to the invention which comprise one or more immunogenic polypeptides, one or more adenoviral vectors, and an adjuvant.

Compositions and methods according to the disclosure may involve use of more than one immunogenic polypeptide and/or more than one adenoviral vector. Use of multiple antigens is especially advantageous in raising protective immune responses to certain pathogens, such as HIV, *M. tuberculosis* and *Plasmodium sp.* Compositions according to the invention may comprise more than one adjuvant.

Compositions and methods employed according to the disclosure may typically comprise a carrier eg an aqueous buffered carrier. Protective components such as sugars may be included.

Compositions should be administered in sufficient amounts to transduce the target cells and to provide sufficient levels of gene transfer and expression and to permit pathogen-specific immune responses to develop thereby to provide a prophylactic or therapeutic benefit without undue adverse or with medically acceptable physiological effects, which can be determined by those skilled in the medical arts. Conventional and pharmaceutically acceptable routes of administration include, but are not limited to, direct delivery to the retina and other intraocular delivery methods, direct delivery to the liver, inhalation, intranasal, intravenous, intramuscular, intratracheal, subcutaneous, intradermal, epidermal, rectal, oral and other parenteral routes of administration. Routes of administration may be combined, if desired, or adjusted depending upon the gene product or the condition. The route of administration primarily will depend on the nature of the condition being treated. Most suitably the route is intramuscular, intradermal or epidermal.

Preferred tissues to target are muscle, skin and mucous membranes. Skin and mucous membranes are the physiological sites where most infectious antigens are normally encountered.

When the first immunogenic polypeptide, adjuvant and adenoviral vector are not co-formulated, the different formulations (eg polypeptide/adjuvant and adenoviral vector formulations) may be administered by the same route of administration or by different routes of administration.

Dosages of compositions in the methods will depend primarily on factors such as the condition being treated, the age, weight and health of the subject, and may thus vary among subjects. For example, a therapeutically effective adult human or veterinary dosage is generally in the range of from about 100 µL to about 100 mL of a carrier containing concentrations of from about 1 x 10⁶ to about 1 x 10¹⁵ particles, about 1 x 10¹¹ to 1 x 10¹³ particles, or about 1 x 10⁹ to 1x 10¹² particles of virus together with around 1-1000ug, or about 2-100ug eg around 4-40ug immunogenic polypeptide. Dosages will range depending upon the size of the animal and the route of administration. For example, a suitable human or veterinary dosage (for about an 80 kg animal) for intramuscular injection is in the range of about 1 x 10⁹ to about 5 x 10¹² virus particles and 4-40 ug protein per mL, for a single site. One of skill in the art may adjust these doses, depending on the route of administration, and the therapeutic or vaccinal application for which the composition is employed.

The amount of adjuvant will depend on the nature of the adjuvant and the immunogenic polypeptide, the condition being treated and the age, weight and health of the subject. Typically for human administration an amount of adjuvant of 1-100ug eg 10-50 ug per dose may be suitable.

Suitably an adequate immune response is achieved by a single concomitant administration of the composition or compositions of the invention in methods of the invention. However if the immune response is further enhanced by administration of a further dose of first immunogenic polypeptide, adjuvant and adenoviral vector on a second or subsequent occasion (for example after a month or two months) then such a protocol is embraced by the invention.

We have found that good pathogen-specific CD4+ and/or CD8+ T-cell responses may typically be raised after a single concomitant administration of the composition or compositions of the invention in methods of the invention. However we have found that good pathogen-specific antibody responses may require a second or further concomitant administration of the composition or compositions of the invention.

The components of the invention may be combined or formulated with any suitable pharmaceutical excipient such as water, buffers and the like.

### Examples

### Adjuvant preparations

### 1) The preparation of oil in water emulsion followed the protocol as set forth in WO 95/17210.

The emulsion contains: 42.72 mg/ml squalene, 47.44 mg/ml tocopherol, 19.4 mg/ml Tween 80. The resulting oil droplets have a size of approximately 180 nm

Tween 80 was dissolved in phosphate buffered saline (PBS) to give a 2% solution in the PBS. To provide 100 ml two fold concentrate, emulsion 5g of DL alpha tocopherol and 5ml of squalene were vortexed until mixed thoroughly. 90ml of PBS/Tween solution was added and mixed thoroughly. The resulting emulsion was then passed through a syringe and finally microfluidised by using an M110S microfluidics machine. The resulting oil droplets have a size of approximately 180 nm

### 2) Preparation of oil in water emulsion with QS21 and MPL

Sterile bulk emulsion was added to PBS to reach a final concentration of 500 µl of emulsion per ml (v/v). 3 D-MPL was then added. QS21 was then added Between each addition of component, the intermediate product was stirred for 5 minutes. Fifteen minutes later, the pH was checked and adjusted if necessary to 6.8 +/- 0.1 with NaOH or HCI. The final concentration of 3D-MPL and QS21 was 100 µg per ml for each.

### 3) Preparation of liposomal MPL

A mixture of lipid (such as phosphatidylcholine either from egg-yolk or synthetic) and cholesterol and 3 D-MPL in organic solvent, was dried down under vacuum (or alternatively under a stream of inert gas). An aqueous solution (such as phosphate buffered saline) was then added, and the vessel agitated until all the lipid was in suspension. This suspension was then microfluidised until the liposome size was reduced to about 100 nm, and then sterile filtered through a 0.2 µm filter. Extrusion or sonication could replace this step. Typically the cholesterol: phosphatidylcholine ratio was 1:4 (w/w), and the aqueous solution was added to give a final cholesterol concentration of 10 mg/ml.

The final concentration of MPL is 2 mg/ml.

The liposomes have a size of approximately 100 nm and are referred to as SUV (for small unilamelar vesicles). The liposomes by themselves are stable over time and have no fusogenic capacity.

### 4) Preparation of Adjuvant B ("adj B")

Sterile bulk of SUV was added to PBS. PBS composition was Na₂HPO₄: 9 mM; KH₂PO₄: 48 mM; NaCl: 100 mM pH 6.1. QS21 in aqueous solution was added to the SUV. The final concentration of 3D-MPL and QS21 was 100 µg per ml for each. This mixture is referred as **Adjuvant B.** Between each addition of component, the intermediate product was stirred for 5 minutes. The pH was checked and adjusted if necessary to 6.1 +/- 0.1 with NaOH or HCI.

### Preparation of p24-RT-Nef-P17 protein ("F4")

F4 was prepared as described in WO2006/013106 Example 1, codon-optimised method.

### Preparation of Chimp adenovirus Pan7 containing Gag-RT-Nef transgene ("Pan7GRN")

### Construction of Gag, RT, Nef plasmid.

### Plasmid p73i-Tgrn

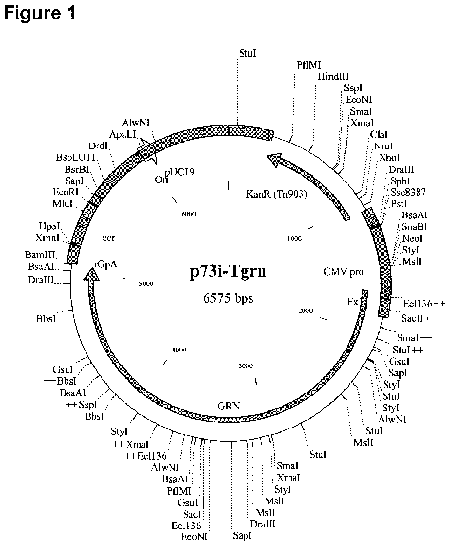

The full sequence of the Tgrn plasmid insert is given in SEQ ID No 1 and the plasmid construction shown graphically in Fig 1. This contains p17 p24 (codon optimised) Gag, p66 RT (codon optimised and inactivated) and truncated Nef.

The plasmid P73i-Tgrn was prepared as described in WO03/025003 Examples 1 - 13.

### Construction of the E1/E3 deleted Pan 7 Adenovirus

The E1/E3 deleted Pan 7 Adenovirus was prepared as described in WO2006/120034 Example 1.

Other serotypes of vectors can be constructed in a similar way. A full description of the construction of E1, E3 and E4 deletions in this and other Pan Adenovirus serotypes is given in WO03/0046124. Further information is also available in Human Gene Therapy 15:519-530.

### Insertion of Gag, RT, Nef sequence into Adenovirus

Using plasmid P73i-Tgrn, the GRN expression cassette was inserted into E1/E3 deleted Pan 7 adenovirus to produce C7-GRNc as described in WO2006/120034 Example 3. C7-GRNc is the Pan7GRN adenovirus component used in the examples set out herein.

### Example 1

### Immunogenicity study in mice immunised with adenovirus component (Pan7GRN) and protein component (F4/adjuvant B) separately or with both adenovirus and protein components co-formulated together

The mouse strain used was CB6F1 and 3 mice were used per timepoint. For immunisation with F4/adjuvant B (P), 1/10 of the human dose was injected i.e. 9 ug of F4 protein in 50uL of adjuvant B. For immunisation with Pan7GRN (A), 10 x 10⁸ virus particles in 50uL of saline (0.9% NaCl water for injection solution) was used. The Pan7GRN chimp adenovirus carries the genes coding for Gag (G), RT (R) and Nef (N).

The vaccination schedule was as follows:

| Group | Day 0 | Day 21 | Day 42 | Day 63 |
|---|---|---|---|---|
| 1 | - | - | F4/adj B | F4/adj B |
| 2 | | | Pan7GRN | Pan7GRN |
| 3 | F4/ adj B | F4/adj B | Pan7GRN | Pan7GRN |
| 4 | Pan7GRN | Pan7GRN | F4/adj B | F4/adj B |
| 5 | - | - | - | F4/adj B/ Pan7GRN |
| 6 | - | - | F4/adj B/ Pan7GRN | F4/adj B/ Pan7GRN |
| 7 | - | - | adj B | adj B |
| 8 | - | - | - | - |

Thus it can be seen that in groups 1 and 2, the mice were immunized with 2 injections of protein (PP) or adenovirus (AA), respectively. Mice from groups 3 and 4, received a conventional prime-boost schedule: protein then adenovirus (PPAA) or the other way round (AAPP) whereas in groups 5 and 6, the mice received one or two injections of a combination (combo) of protein and adenovirus together according to the invention. Mice from group 7 only received adjuvant control whereas mice from group 6 were naive.

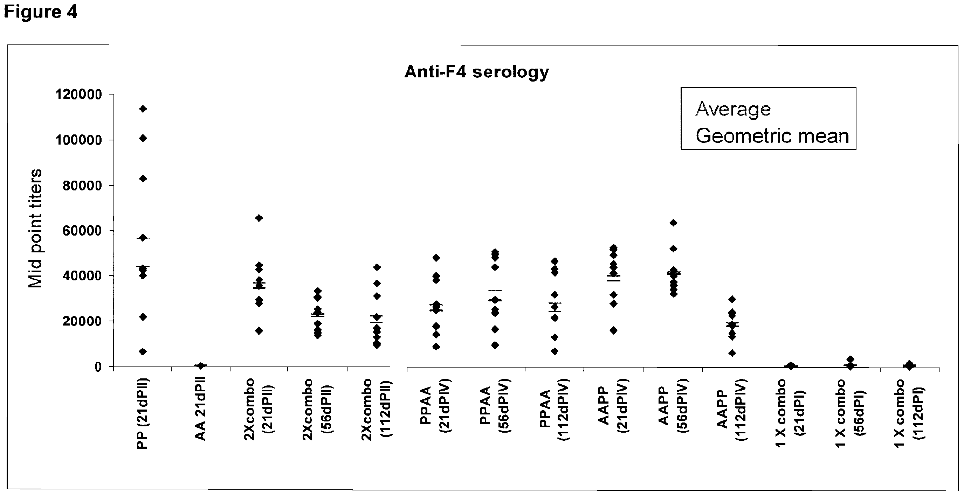

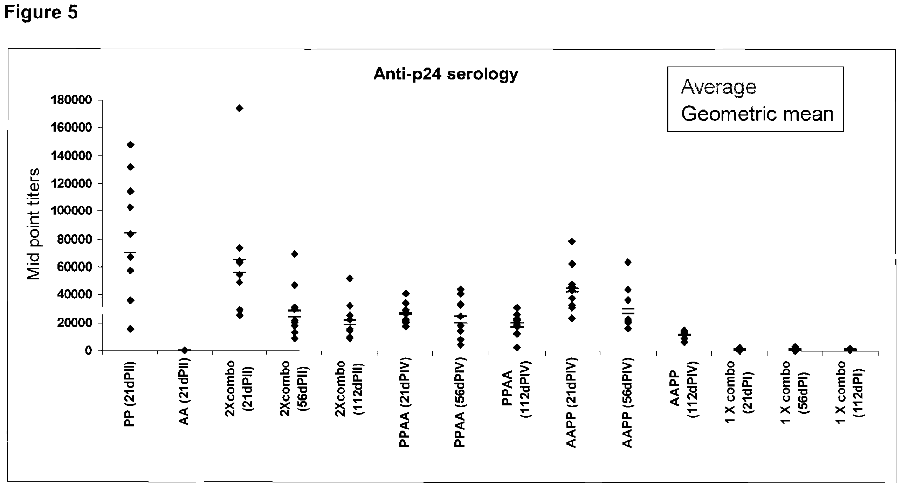

The following read-outs were performed:
Antibody responses (ELISA performed on the sera from each individual animals from each group):
- antibody response against F4 (Figure 4)
- antibody response against F4 components p24, RT, Nef and p17 (Figure 5-8)

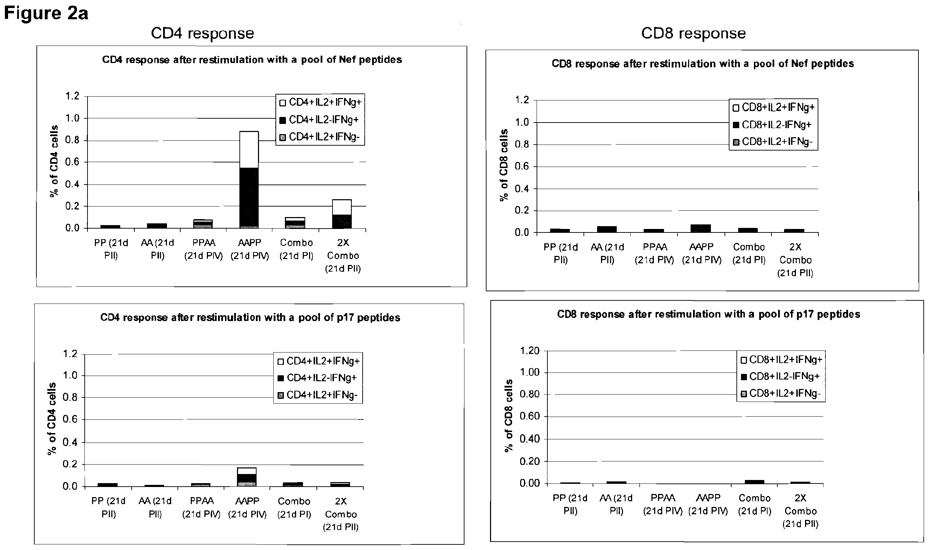

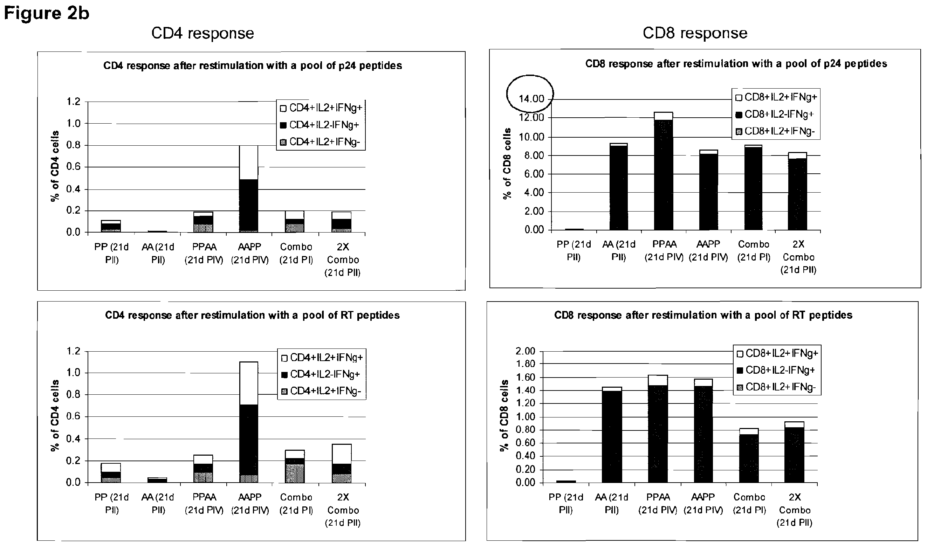

Cellular responses (Figures 2-3):
- measured by flow cytometry following surface and intracellular cytokine staining after overnight restimulation of spleen cells with pools of peptides of p24, RT, Nef or p17. The spleen cells of 3 mice per timepoint and per group were pooled for the analysis.

For groups 1 and 2, samples were taken for measurement 21 days after the corresponding final immunisation. For the remaining groups, measurements were taken 21 days, 56 days and 112 days after the corresponding final immunisation.

### Results:

The results are shown in Figures 2-8.

The X axis labels correspond as follows:
PP - Group 1 animals following second immunisation
AA - Group 2 animals following second immunisation
PPAA - Group 3 animals following fourth immunisation
AAPP - Group 4 animals following fourth immunisation
Combo - Group 5 animals following immunisation
Combo x 2 - Group 6 animals following second immunisation

The measurement timepoints (21, 56 or 112 days post last immunisation) are indicated in parentheses.

### Cellular responses (Figure 2-3):

At the timepoints analysed, the data show that CD4+ T-cell responses were observed mainly against p24, RT and Nef.

As shown in Figures 2a and 2b (left panels), 21 days post last immunisation, the highest CD4+ T-cell responses are observed with two immunisations of adenovirus followed by two immunisations of protein/adjuvant (Group 4 animals). One injection of the combination of adenovirus/protein/adjuvant induces higher CD4+ T-cell levels than two injections of protein/adjuvant following restimulation with p24, RT or Nef peptides.
For restimulation by RT and Nef, two immunisations with the combination of adenovirus/protein/adjuvant induces a CD4+ T-cell response slightly higher than with one immunisation with the combination, whereas the responses with one or two immunisations were identical for p24.

At the timepoints analysed, the CD8+ T-cell responses are mainly observed against the p24 and RT peptides, and no significant numbers of CD8+ T-cells specific for Nef or p17 were detected.
As shown in Figures 2a and 2b (right panels), 21 days post last immunisation CD8+ T-cell responses were similar after one or two immunisations with the combination of adenovirus/protein/adjuvant. The CD8 response against p24 observed in groups immunised either (i) twice with adenovirus or (ii) twice with adenovirus followed by twice with protein or (iii) once or twice with the combination of adenovirus/protein/adjuvant were comparable to each other and slightly lower than the one from the group immunised twice with protein followed by twice with adenovirus. The CD8 response against RT observed in groups immunised once or twice with the combination of adenovirus/protein/adjuvant were comparable and slightly lower to the one from the groups immunised either (i) twice with adenovirus or (ii) twice with adenovirus followed by twice with protein or (iii) twice with protein followed by twice with adenovirus.

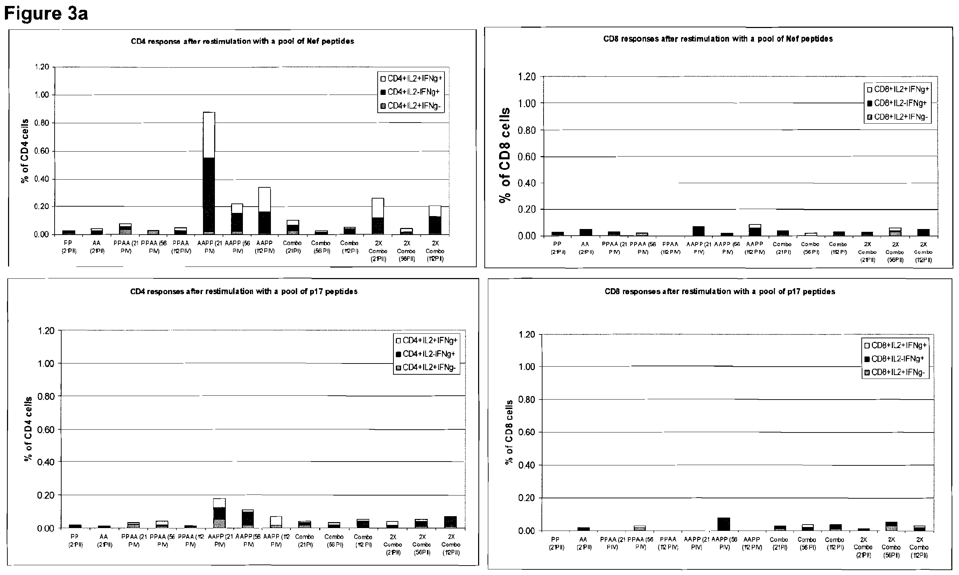

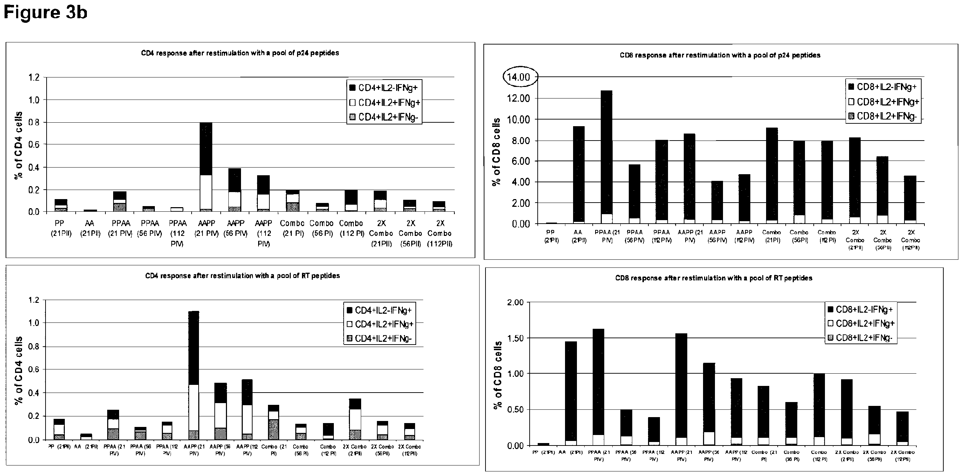

The CD4 and CD8 T cell responses were also analysed at later timepoints (56 and 112 days post last immunisation), when persistence of the responses can be determined (Figures 3a and 3b). The CD4 responses (Fig 3a and 3b, left panels) are mainly observed against p24, RT and Nef. At these timepoints, the highest CD4 responses are observed in the animals immunised twice with adenovirus followed by twice with protein. The CD4 response in mice immunised once or twice with the combination of adenovirus/protein/adjuvant were comparable to each other and generally higher than the response observed in groups immunised twice with protein followed by twice with adenovirus.
At the later timepoints, the CD8 response against p24 is the highest in the group immunised once with the combination of adenovirus/protein/adjuvant (Fig 3b, right panel). It is comparable to the one from animals immunised twice with protein followed by twice with adenovirus and slightly higher than the one from the animals immunised either (i) twice with the combination of adenovirus/protein/adjuvant or (ii) twice with adenovirus followed by twice with protein. The latter two are comparable between each other. The CD8 response against RT is the highest and similar in groups immunised (i) twice with the combination of adenovirus/protein/adjuvant or (ii) twice with adenovirus followed by twice with protein. The CD8 response against RT from groups immunised (i) twice with the combination of adenovirus/protein/adjuvant or (ii) twice with protein followed by twice with adenovirus was slightly lower but comparable between each other (Figure 3). As shown in figure 3a (right panel), no significant numbers of CD8+ T-cells specific for Nef or p17 were detected.

### Antibody responses:

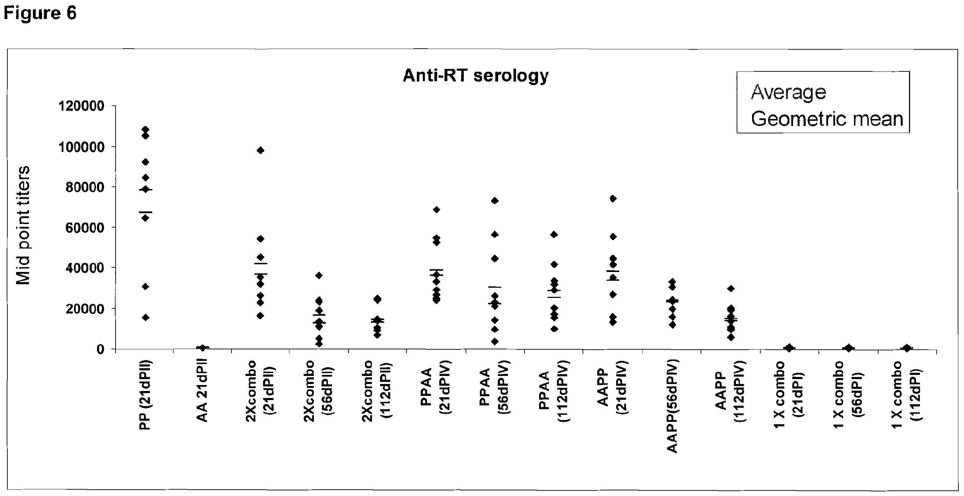

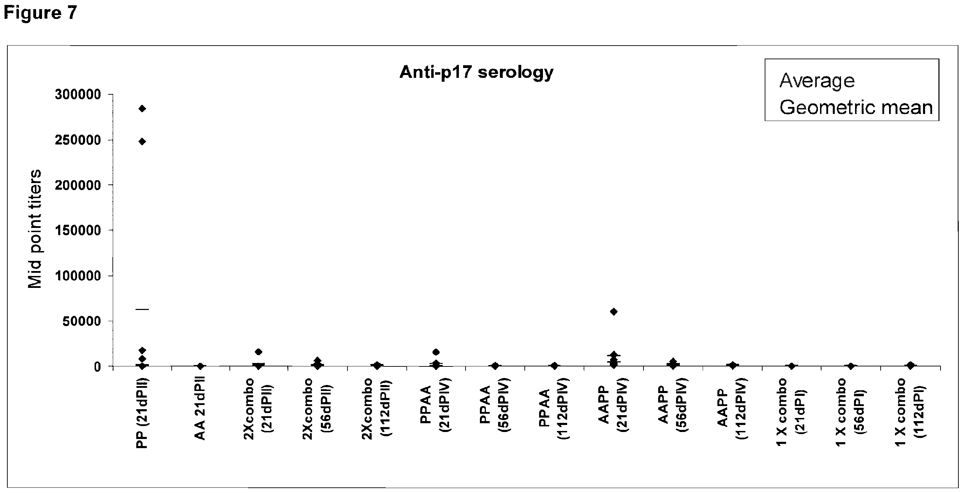

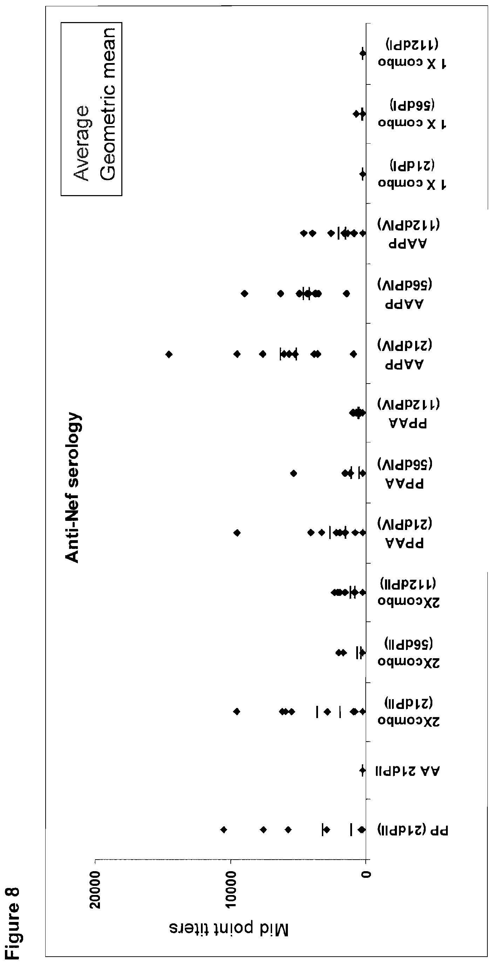

As shown in Figures 4 to 8, the antibody responses detected are mainly directed against p24 (Fig 5), RT (Fig 6) and Nef (Fig 8). The anti-F4 (Fig 4) response generally mimics the response observed against each of the p24, RT or Nef components and can be characterized as follows:
- Low to no antibody response is detected in groups immunised (i) twice with adenovirus or (ii) once with the combination of adenovirus/protein/adjuvant;
- The highest antibody responses usually detected in group immunised twice with the protein at 21 days post immunisation. However, it is also in this group that the highest variability between individuals is observed. In addition, for the anti-Nef serology, the group immunised twice with adenovirus followed by twice with protein appears to display the highest response, when compared to the other groups;
- The response observed in groups immunised (i)) twice with the combination of adenovirus/protein/adjuvant or (ii) twice with protein followed by twice with adenovirus or (iii) twice with adenovirus followed by twice with protein are comparable, peak at 21 days post last immunisation and then slightly decrease over time.
Antibody responses against p17 (Fig 7) were very low to undetectable in all groups.

### Conclusion:

Globally, the highest antigen-specific cell-mediated immune response is observed in the AAPP treatment group after 4 immunisations. However, when comparing groups after 2 immunisations (i.e. AA, PP and 2xcombo groups), the induction of both antigen-specific CD4 and CD8 T cell responses is only observed in the group immunised twice with the protein/adenovirus/adjuvant combination. In addition, similar levels of CD4 and CD8 T cell responses can be reached after a single injection of the protein/adenovirus/adjuvant combination. Moreover, in terms of persistence, the antigen-specific T cell responses observed 112 days after the 2^{nd} immunisation with the protein/adenovirus/adjuvant combination are comparable to the ones observed 112 days after the 4^{th} immunisations in the AAPP treatment group. Finally, it appears that 2 immunisations with the protein/adenovirus/adjuvant combination are needed to obtain an antibody response comparable to the one obtained in the group immunised twice with the adjuvanted protein, group that provided the highest antibody responses in general.

### Example 2

### Immunogenicity study in mice immunised with Pan7GRN adenovirus and F4 protein/adjuvant B co-formulated together

The mouse strain used was CB6F1 with 9 mice per group. Mice were immunized once with a co-formulation of the F4 protein (1/10 of the human dose was injected i.e. 9 ug) together with 10 x 10⁸ virus particles of Pan7GRN, in 50uL of adjuvant B or a dilution of the latter (1/2, 1/4 or 1/10). The CD4 and CD8 cellular responses against a pool of either Nef, p17, p24 or RT peptides were determined 21 days post immunization (3 pools of 3 spleens for each group).

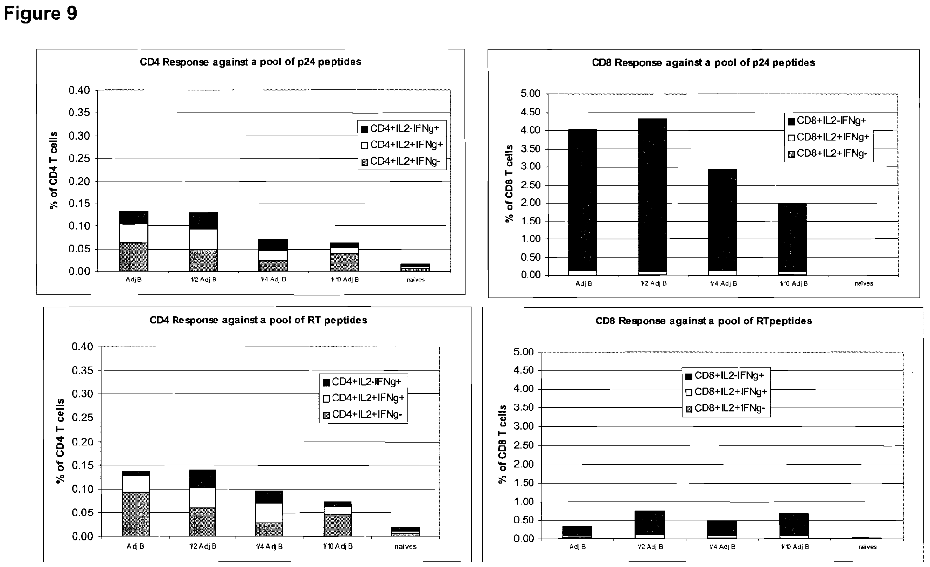

The following read-out was performed:
Cellular responses (Figure 9):
- measured by flow cytometry following surface and intracellular cytokine staining after overnight restimulation of spleen cells with pools of peptides of p24, RT, Nef or p17. The spleen cells were pooled (3 pools of 3 spleens per group) for the analysis.

### Results:

The results shown in Figure 9 represent the cellular responses observed after restimulation with a pool of p24 or RT peptides.

The X axis labels correspond as follows:
Adj B - Mice immunised with 9µgF4/ 10⁸vpPan7GRN/ non-diluted adjuvant B
1/2 Adj B - Mice immunised with 9µgF4/ 10⁸vpPan7GRN/ adjuvant B diluted 1/2
1/4 Adj B - Mice immunised with 9µgF4/ 10⁸vpPan7GRN/ adjuvant B diluted 1/4
1/10 Adj B - Mice immunised with 9µgF4/ 10⁸vpPan7GRN/ adjuvant B diluted 1/10

### Naive - Naive mice (no immunisation)

The results indicate that CD4 (Figure 9, left panel) and CD8 (Figure 9, right panel) responses are mainly observed against p24 and RT, with the CD8 T cell response specific to RT being lower than the one specific to p24. In addition, the results indicate that the CD4 responses against p24 and RT at 21 days post-immunisations in the groups immunised with the non-diluted adjuvant B or a 1/2 dilution of it are similar. These CD4 responses tend to decrease when the adjuvant is diluted 1/4. When the adjuvant B is diluted at 1/10, the CD4 responses observed are similar to the ones from groups immunised with the 1/4 dilution of the adjuvant B. The anti-CD8 responses against p24 are comparable whether the adjuvant is diluted 1/2 or not. However, the response decreases when the adjuvant B is diluted 1/4 and even more so if it is diluted 1/10. In contrast, such trends are not seen for the anti-RT CD8 responses where there is not a real dose range effect of the dose of adjuvant used.

### Conclusion:

CD4+ cells and CD8+ cells against F4 components were induced by a single administration of a composition containing an immunogenic polypeptide, an adenoviral vector containing a heterologous polynucleotide encoding an immunogenic polypeptide and an adjuvant, even when the latter was diluted. The impact of adjuvant dilution differed depending on the antigen-specific CD4 or CD8 responses of interest. In particular the highest responses observed were against p24 and the anti-p24 CD4 and CD8 T cell responses show a dose range effect correlating with the dose of adjuvant used in the combination vaccine. While the same effect can be observed for the anti-RT CD4 T cell response, the dose range effect of the dose of adjuvant used in the combo is less clear for the anti-RT CD8 T cell response. Finally, if we consider the global antigen-specific CD4 and CD8 T cell responses and sum the responses against the 4 antigens, a dose range can be observed.

### Example 3:

### Immunogenicity study in New Zealand white rabbits immunised with Pan7GRN or F4/adjuvant B sequentially or with both adenovirus and protein components co-formulated together

For immunisation with F4/adjuvant B, the human dose was injected i.e. 90 ug of F4 protein in 500uL of adjuvant B. For immunisation with Pan7GRN, 10 x 10¹⁰ or 10 x 10¹² virus particles in 500uL of saline were used. For the immunization with both adenovirus and protein components co-formulated together, 90µg of F4 protein, 10 x 10¹¹ virus particles of Pan7 GRN in 500uL of adjuvant B were used.

The vaccination schedule was as follows:

| **Group** | **Day 0** | **Day 14** | **Day 126** |
|---|---|---|---|
| 1 | F4/ adj B | F4/ adj B | F4/adj B |
| 2 | Pan7GRN 10^10 | | Pan7GRN 10^10 |
| 3 | Pan7GRN 10^12 | | Pan7GRN 10^12 |
| 4 | F4/adj B/Pan7GRN 10^11 | F4/adj B/ Pan7GRN 10^11 | F4/adj B/Pan7GRN 10^11 |

There were 3 rabbits per group except for group 1 which included only 2 rabbits.

The following read-outs were performed:
Antibody responses (ELISA performed on the sera from each individual animals from each group):
- antibody response against F4
- antibody response against F4 components p24, RT, Nef and p17

### Lymphoproliferative responses:

The lymphoproliferation was determined by the uptake of tritiated thymidine by peripheral blood mononuclear cells (isolated from whole blood after a density gradient) restimulated in vitro with pools of Nef, p17, p24 and/or RT peptides for 88 hours in the presence of tritiated thymidine for the last 16 hours of the incubation.

### Results:

### Lymphoproliferative response:

As shown in Figure 10, the highest lymphoproliferative responses are observed in the group immunised twice with protein. The lymphoproliferative response from animals immunised twice with the combination of adenovirus/protein/adjuvant was observed in all rabbits from the group. It actually peaked after one injection and could be further recalled (at similar levels than after the 1^{st} injection) following a third injection of the combination of adenovirus/protein/adjuvant, suggesting that the first two injections did not induce a neutralizing response that would inhibit any response to a further similar injection. In its intensity, the proliferative response observed in rabbits immunised with the combination of adenovirus/protein/adjuvant was comparable to the one observed in animals immunised once or twice with 10¹² viral particles of adenovirus and appeared higher than the one from animals immunised once or twice with 10¹⁰ viral particles of adenovirus. Altogether, this suggests that using the combination of adenovirus/protein/adjuvant could decrease the dose of adenovirus to be used. Finally, after a third injection of the combination of adenovirus/protein/adjuvant, the response observed in group 4 was similar to the one from animals immunised 3 times with the protein (group 1).

### Serology:

As shown in Figure 11, the kinetic of the anti-F4 antibody response observed in the animals immunised twice with the combination of adenovirus/protein/adjuvant is similar to the one from animals immunised twice with the protein: it is already detected at 7 days post-2^{nd} injection and then decrease over time. However, in terms of intensity, the anti-F4 response of animals immunised twice with the combination of adenovirus/protein/adjuvant remains higher at later timepoints (21 and 63 days post-2^{nd} immunisation) when compared to the anti-F4 response from animals immunised twice with the protein. No anti-F4 antibody response is observed in rabbits immunised once with 10¹⁰ viral particles of adenovirus. In rabbits immunised once with 10¹² viral particles of adenovirus, an anti-F4 response is only detected at 21 and 63 days post-immunisation. In that group, the high variability of the response observed at the 63 day post-immunisation timepoint (d77) results from a single animal (out of the 3) displaying higher titers against the different F4 components, especially p24 and RT as shown in Figures 12a and 12b respectively. The anti-F4 antibody response is mainly composed of antibodies targeting p24 and RT and to a much lesser extent Nef and p17.

### Conclusion:

Lymphoproliferative and antibody responses could be induced in rabbits after two injections of a composition containing an immunogenic polypeptide, an adenoviral vector containing a heterologous polynucleotide encoding an immunogenic polypeptide and an adjuvant. In addition, we have evidence that a lymphoproliferative response can be recalled after a third injection of such composition. Finally, the best antibody response (in intensity and persistence) is observed with the adenovirus/protein/adjuvant combination.

### Example 4

### Immunogenicity of F4 (codon optimized)/adjuvant B and C7-GRN when administrated as a combination in CB6F1 mice.

### Experimental design

CB6F1 mice were immunized twice (days 0 and 21) with different combinations listed below. F4co/ adjuvant B was used at 9µg F4co/animal in 50µl AdjuvantB (1/10 human dose) and the C7-GRN virus at 10⁸ viral particles/animal. F4co in Example 4 is F4 prepared as described in WO2006/013106 Example 1, codon-optimised method.

### Combinations

C7-GRN
C7-GRN/ adjuvant B
C7-GRN/F4co
C7-GRN/F4co/ adjuvant B
F4co
F4co/ adjuvant B
adjuvant B
C7 empty
C7empty/ adjuvant B
C7empty/F4co
C7empty/F4co/ adjuvant B

### Schedule of immunizations and immune response analysis

Immunisations were carried out at day 0 and day 21. Intracellular cytokine staining (ICS) was carried out at 21 days, 28 days (7 days post immunisation 2), 42 days (21 days post immunisation 2), and 77 days (56 days post immunisation 2).

### Results

### HIV-specific CD4 T cell responses

The results are shown in the following figures:
Figure 13. Quantification of HIV-1-specific CD4 T cells. The % of CD3 CD4 T cells secreting IFN-γ and/or IL-2 is represented for each protocol of immunization at four time-points. Peripheral blood lymphocytes (PBLs) were stimulated *ex vivo* (2 hours before addition of the Brefeldin then overnight) with a pool of peptides covering F4 sequence and the cytokine production was measured by ICS. Each value is the geometric mean of 5 pools of 3 mice.
Figure 14. Distribution of the frequency of F4-specific CD4 T cells 7 days after two immunizations. The frequency of F4-specific circulating CD4 T cells at 7 days after two immunizations is represented for each protocol. Each dot represents the value obtained for one pool of 3 mice.
Figure 15. Cytokine production of F4-specific CD4 T cells 7 days after two immunizations. The % of F4-specific CD4 T cells secreting IL-2 and/or IFN-γ is represented for 5 pools of 3 mice. Results for the immunization with F4co/ adjuvant B **(A),** F4co/ adjuvant B /C7 empty **(B)** and F4co/ adjuvant B /C7-GRN **(C)** are presented.

The frequency of F4-specific circulating CD4 T cells reaches 2.82% 21 days after two immunizations with the F4co/ adjuvant B combination and declines to 0.91% 56 days post-immunization (Figure 13). Two doses of the C7-GRN virus alone result in 0.52% of F4-specific circulating CD4 T cells 21 days post last immunization and the presence of the adjuvant adjuvant B does not alter this response.

The presence of the empty vector C7 or the recombinant C7-GRN virus in addition of the F4co/ adjuvant B mix does not increase nor interfere with the frequency of F4-specific CD4 T cell response (3.58% and 2.82% respectively, 21 days post-last immunization). Even if no statistical analysis has been performed, the population distribution suggests that the intensity of the F4-specific CD4 T cell responses is not different between the three protocols F4co/ adjuvant B, F4co/ adjuvant B /C7 empty and F4co/ adjuvant B /C7-GRN (Figure 14).
As expected, administration of the F4co without adjuvant B does not induce significant F4-specific CD4 T cells.

The profile of cytokine production shows that after immunization with F4co/ adjuvant B, the F4-specific CD4 T cells secrete both IFN-γ and IL-2. Addition of C7empty or C7-GRN in the immunization protocol does not alter this profile.

As a result, these data suggest that the greatest F4-specific CD4 T cell response is obtained after immunization with the F4co/ adjuvant B combination and that the presence of the C7-GRN virus does not improve nor alter this response.

### Antigen-specific CD8 T cell responses

The results are shown in the following figures
Figure 16. Quantification of HIV-1-specific CD8 T cells. The % of CD3 CD8 T cells secreting IFN-γ and/or IL-2 is represented for each protocol of immunization at four time-points. Peripheral blood lymphocytes (PBLs) were stimulated *ex vivo* (2 hours before addition of Brefeldin then overnight) with a pool of peptides covering F4 and the cytokine production was measured by ICS. Each value is the geometric mean of 5 pools of 3 mice.
Figure 17. Cytokine production of F4-specific CD8 T cells 7 days after two immunizations. The % of F4-specific CD8 T cells secreting IL-2 and/or IFN-γ is represented for 5 pools of 3 mice. Results for the immunization with C7-GRN **(A),** C7-GRN/ adjuvant B **(B)** and C7-GRN+F4co/ adjuvant B **(C)** are presented.

After one injection, the recombinant vector C7-GRN induces a high frequency of F4-specific circulating CD8 T cells (9,70% of total CD8 T cells, 21 days post-immunization) (Figure 4). A second injection does not boost the F4-specific CD8 T cell response. The F4co/ adjuvant B combination induces low to undetectable F4-specific CD8 T cells and adding this combination to the C7-GRN does not improve or impair the F4-specific CD8 T cell response.

The F4-specific CD8 T cell response is delayed when the adjuvant B is added to the C7-GRN, but reaches the same level as with the C7-GRN alone or the C7-GRN/F4co/ adjuvant B combination at 21 days post-second immunization.

The F4-specific CD8 T cells mainly secrete IFN-γ whether the C7-GRN vector is injected alone or in combination with F4co/ adjuvant B (Figure 17).

Interestingly, the F4-specific CD8 T cell response persists up to 56 days post-last immunization without declining, suggesting that the C7 vector elicits high and persistent CD8 T cells.

### Conclusions

The F4co/adjuvant B vaccine induces a high frequency of poly-functional HIV-specific CD4 T cells but no HIV-specific CD8 T cells in CB6F1 mice. In the same animal model, the recombinant adenovirus C7 expressing Gag, RT and Nef (Ad C7-GRN) induces a high antigen-specific CD8 T cell response and low to undetectable antigen-specific CD4 T cells. A combination of F4/ adjuvant B and Ad C7-GRN elicits both antigen-specific CD4 and CD8 T cells at the same time. A combination of three components, F4co, adjuvantB and C7-GRN elicts the highest levels of both antigen specific CD4 and CD8 T cells at the same time.

Combining F4/ adjuvant B and Ad C7-GRN has an additive effect concerning the intensity of both arms of the cellular immune response. The effect of the antigen-specific CD4 T cell response on the functionality of antigen-specific CD8 T cell response remains to be determined in this model.

### Example 5

### Immunogenicity of the chimpadenovirus C7 expressing CS2 construct of CSP protein from Plasmodium falciparum (C7-CS2) when administered alone

### Experimental design:

CB6F1 mice were immunized once intramuscularly with a dose range (10¹⁰, 10⁹ & 10⁸ viral particles) of the C7 chimpadenovirus expressing the CSP malaria antigen and the CSP-specific (C-term and N-term) CD4 and CD8 T cell responses were determined 21, 28 and 35 days post-injection by ICS (Intra-cellular Cytokine Staining).

### CSP-specific CD4 T cell responses

The results are shown in the following figures:
Figure 18. Quantification of CSP-specific CD4 T cells. The % of CD4 T cells secreting IFN-γ and/or IL-2 is represented for each protocol of immunization at three time-points. Peripheral blood lymphocytes (PBLs) were stimulated *ex vivo* (2 hours before addition of the Brefeldin then overnight) with a pool of peptides covering CSP N-term or CSP C-term sequences and the cytokine production was measured by ICS. The responses to the C-term and N-term peptide pools were added up and each value is the average of 5 pools of 4 mice.
Figure 19. Quantification of CSP-specific CD8 T cells. The % of CD8 T cells secreting IFN-γ and/or IL-2 is represented for each protocol of immunization at three time-points. Peripheral blood lymphocytes (PBLs) were stimulated *ex vivo* (2 hours before addition of the Brefeldin then overnight) with a pool of peptides covering CSP N-term or CSP C-term sequences and the cytokine production was measured by ICS. The responses to the C-term and N-term peptide pools were added up and each value is the average of 5 pools of 4 mice.

These results indicate that both 10¹⁰ and 10⁹ doses of C7-CS2 elicit similar levels of CSP-specific CD4 T cell responses (peak 0.5%) and similar levels of CSP-specific CD8 T cell responses (peak 8%). The dose of 10¹⁰ of C7-CS2 was chosen in subsequent experiments where the immunogenicity of C7-CS2 in combination with RTS,S was tested (see below).

### Example 6

### Immunogenicity of C7-CS2 and RTS,S when administered as a combination in CB6F1 mice

### Experimental design:

CB6F1 mice were immunized three times intramuscularly (day 0, 14 & 28) with either a combination of the malaria vaccine candidate RTS,S (5µg) in 50µl of Adjuvant B (referred as P-P-P in the figures below) or a combination of RTS,S (5µg) and C7-CS2(10¹⁰ viral particles) in 50µl of Adjuvant B (referred as C-C-C in the figures below). The CSP-specific (C-term and N-term) CD4 and CD8 T cell responses were determined at the following time-points:
- 7 days post 2 immunizations
- 7, 21, 35 and 49 days post 3 immunizations
CSP-specific T cell responses were determined by ICS (Intra-cellular Cytokine Staining).

The CSP-specific antibody responses in the sera from immunized animals were also determined by ELISA at 14 and 42 days post- 3^{rd} immunization.

### CSP-specific CD4 T cell responses

The results are shown in the following figures:
Figure 20. Quantification of CSP(N-term)-specific CD4 T cells. The % of CD4 T cells secreting IFN-γ and/or IL-2 is represented for each protocol of immunization at five time-points. Peripheral blood lymphocytes (PBLs) were stimulated *ex vivo* (2 hours before addition of the Brefeldin then overnight) with a pool of peptides covering the CSP N-term sequence and the cytokine production (IFNg and/or IL-2) was measured by ICS. Each value is the average of 4 pools of 7 mice.
Figure 21. Quantification of CSP(C-term)-specific CD4 T cells. The % of CD4 T cells secreting IFN-γ and/or IL-2 is represented for each protocol of immunization at five time-points. Peripheral blood lymphocytes (PBLs) were stimulated *ex vivo* (2 hours before addition of the Brefeldin then overnight) with a pool of peptides covering the CSP C-term sequence and the cytokine production (IFNg and/or IL-2) was measured by ICS. Each value is the average of 4 pools of 7 mice.

These results indicate that mice immunized with 3 injections of the combination [RTS,S + C7-CS2 10¹⁰ +Adjuvant B] display higher antigen-specific CD4 T cell responses (both against the C-term and N-term part of CSP) than the mice immunized with 3 injections of RTS,S+Adjuvant B.

### CSP-specific CD8 T cell responses

The results are shown in the following figures:
Figure 22. Quantification of CSP(N-term)-specific CD8 T cells. The % of CD8 T cells secreting IFN-γ and/or IL-2 is represented for each protocol of immunization at five time-points. Peripheral blood lymphocytes (PBLs) were stimulated *ex vivo* (2 hours before addition of the Brefeldin then overnight) with a pool of peptides covering the CSP N-term sequence and the cytokine production (IFNg and/or IL-2) was measured by ICS. Each value is the average of 4 pools of 7 mice.
Figure 23. Quantification of CSP(C-term)-specific CD8 T cells. The % of CD8 T cells secreting IFN-γ and/or IL-2 is represented for each protocol of immunization at five time-points. Peripheral blood lymphocytes (PBLs) were stimulated *ex vivo* (2 hours before addition of the Brefeldin then overnight) with a pool of peptides covering the CSP C-term sequence and the cytokine production (IFNg and/or IL-2) was measured by ICS. Each value is the average of 4 pools of 7 mice.

These results indicate that mice immunized with 3 injections of the combination [RTS,S + C7-CS2 10¹⁰ + Adjuvant B] display higher antigen-specific CD8 T cell responses (both against the C-term and N-term part of CSP) than the mice immunized with 3 injections of RTS,S+Adjuvant B.

### CSP-specific antibody responses

The results are shown in the following figure:
Figure 24. Quantification of CSP-specific antibody titers.The sera from the mice were collected at 14 and 42 days post 3^{rd} immunization. The anti-CSP antibody titers were measured in each of these individual sera by ELISA. The data shown is the geometric mean antibody titers + 95% confidence interval.

These results indicate that mice immunized with 3 injections of the combination [RTS,S + C7-CS2 10¹⁰ + Adjuvant B] display similar CSP-specific antibody titers than the mice immunized with 3 injections of RTS,S+Adjuvant B.

### Conclusions

The RTS,S/adjuvant B vaccine induces a high frequency of CSP C-term-specific CD4 T cells but no CSP N-term specific CD4 T cells. In addition, the RTS,S/adjuvant B vaccine induces low to undetectable CSP C& N-term specific CD8 T cells. In the same animal model, the recombinant adenovirus C7 expressing CSP induces high CSP(C-term and N-term)-specific CD8 T cell responses and lower CSP(C-term and N-term)-specific CD4 T cell responses. A combination of RTS,S/ adjuvant B and Ad C7-CS2 elicits high levels of both CSP(C-term and N-term)-specific CD4 and CD8 T cells at the same time. Combining RTS,S/ adjuvant B and Ad C7-CS2 has an additive effect concerning the intensity of both arms of the T cell response. Finally, the combination of RTS,S/ adjuvant B and Ad C7-CS2 elicits high levels of CSP-specific antibody responses that are comparable to the ones induced by RTS,S/adjuvant B.

### SEQUENCES

SEQ ID No 1:
SEQ ID No 2:
SEQ ID No 3:
SEQ ID No 4:
SEQ ID No 5:
SEQ ID No 6:
SEQ ID No 7:
SEQ ID No 8:
SEQ ID No 9:
SEQ ID No 10:
SEQ ID No 11:
SEQ ID No 12:
SEQ ID No 13:
SEQ ID No 14:
SEQ ID No 15:
SEQ ID No 16:

### SEQUENCE LISTING

<110> GlaxoSmithKline Biologicals S.A.
   VOSS, Gerald Hermann
<120> Novel method and composition
<130> VB62209
<160> 16
<170> FastSEQ for Windows Version 4.0
<210> 1
   <211> 3204
   <212> DNA
   <213> Artificial Sequence
<220>
   <223> HIV
<400> 1
   atgggtgccc gagcttcggt actgtctggt ggagagctgg acagatggga gaaaattagg 60
   ctgcgcccgg gaggcaaaaa gaaatacaag ctcaagcata tcgtgtgggc ctcgagggag 120
   cttgaacggt ttgccgtgaa cccaggcctg ctggaaacat ctgagggatg tcgccagatc 180
   ctggggcaat tgcagccatc cctccagacc gggagtgaag agctgaggtc cttgtataac 240
   acagtggcta ccctctactg cgtacaccag aggatcgaga ttaaggatac caaggaggcc 300
   ttggacaaaa ttgaggagga gcaaaacaag agcaagaaga aggcccagca ggcagctgct 360
   gacactgggc atagcaacca ggtatcacag aactatccta ttgtccaaaa cattcagggc 420
   cagatggttc atcaggccat cagcccccgg acgctcaatg cctgggtgaa ggttgtcgaa 480
   gagaaggcct tttctcctga ggttatcccc atgttctccg ctttgagtga gggggccact 540
   cctcaggacc tcaatacaat gcttaatacc gtgggcggcc atcaggccgc catgcaaatg 600
   ttgaaggaga ctatcaacga ggaggcagcc gagtgggaca gagtgcatcc cgtccacgct 660
   ggcccaatcg cgcccggaca gatgcgggag cctcgcggct ctgacattgc cggcaccacc 720
   tctacactgc aagagcaaat cggatggatg accaacaatc ctcccatccc agttggagaa 780
   atctataaac ggtggatcat cctgggcctg aacaagatcg tgcgcatgta ctctccgaca 840
   tccatccttg acattagaca gggacccaaa gagcctttta gggattacgt cgaccggttt 900
   tataagaccc tgcgagcaga gcaggcctct caggaggtca aaaactggat gacggagaca 960
   ctcctggtac agaacgctaa ccccgactgc aaaacaatct tgaaggcact aggcccggct 1020
   gccaccctgg aagagatgat gaccgcctgt cagggagtag gcggacccgg acacaaagcc 1080
   agagtgttga tgggccccat cagtcccatc gagaccgtgc cggtgaagct gaaacccggg 1140
   atggacggcc ccaaggtcaa gcagtggcca ctcaccgagg agaagatcaa ggccctggtg 1200
   gagatctgca ccgagatgga gaaagagggc aagatcagca agatcgggcc ggagaaccca 1260
   tacaacaccc ccgtgtttgc catcaagaag aaggacagca ccaagtggcg caagctggtg 1320
   gatttccggg agctgaataa gcggacccag gatttctggg aggtccagct gggcatcccc 1380
   catccggccg gcctgaagaa gaagaagagc gtgaccgtgc tggacgtggg cgacgcttac 1440
   ttcagcgtcc ctctggacga ggactttaga aagtacaccg cctttaccat cccatctatc 1500
   aacaacgaga cccctggcat cagatatcag tacaacgtcc tcccccaggg ctggaagggc 1560
   tctcccgcca ttttccagag ctccatgacc aagatcctgg agccgtttcg gaagcagaac 1620
   cccgatatcg tcatctacca gtacatggac gacctgtacg tgggctctga cctggaaatc 1680
   gggcagcatc gcacgaagat tgaggagctg aggcagcatc tgctgagatg gggcctgacc 1740
   actccggaca agaagcatca gaaggagccg ccattcctga agatgggcta cgagctccat 1800
   cccgacaagt ggaccgtgca gcctatcgtc ctccccgaga aggacagctg gaccgtgaac 1860
   gacatccaga agctggtggg caagctcaac tgggctagcc agatctatcc cgggatcaag 1920
   gtgcgccagc tctgcaagct gctgcgcggc accaaggccc tgaccgaggt gattcccctc 1980
   acggaggaag ccgagctcga gctggctgag aaccgggaga tcctgaagga gcccgtgcac 2040
   ggcgtgtact atgacccctc caaggacctg atcgccgaaa tccagaagca gggccagggg 2100
   cagtggacat accagattta ccaggagcct ttcaagaacc tcaagaccgg caagtacgcc 2160
   cgcatgaggg gcgcccacac caacgatgtc aagcagctga ccgaggccgt ccagaagatc 2220
   acgaccgagt ccatcgtgat ctgggggaag acacccaagt tcaagctgcc tatccagaag 2280
   gagacctggg agacgtggtg gaccgaatat tggcaggcca cctggattcc cgagtgggag 2340
   ttcgtgaata cacctcctct ggtgaagctg tggtaccagc tcgagaagga gcccatcgtg 2400
   ggcgcggaga cattctacgt ggacggcgcg gccaaccgcg aaacaaagct cgggaaggcc 2460
   gggtacgtca ccaaccgggg ccgccagaag gtcgtcaccc tgaccgacac caccaaccag 2520
   aagacggagc tgcaggccat ctatctcgct ctccaggact ccggcctgga ggtgaacatc 2580
   gtgacggaca gccagtacgc gctgggcatt attcaggccc agccggacca gtccgagagc 2640
   gaactggtga accagattat cgagcagctg atcaagaaag agaaggtcta cctcgcctgg 2700
   gtcccggccc ataagggcat tggcggcaac gagcaggtcg acaagctggt gagtgcgggg 2760
   attagaaagg tgctgatggt gggttttcca gtcacacctc aggtaccttt aagaccaatg 2820
   acttacaagg cagctgtaga tcttagccac tttttaaaag aaaagggggg actggaaggg 2880
   ctaattcact cccaaagaag acaagatatc cttgatctgt ggatctacca cacacaaggc 2940
   tacttccctg attggcagaa ctacacacca gggccagggg tcagatatcc actgaccttt 3000
   ggatggtgct acaagctagt accagttgag ccagataagg tagaagaggc caataaagga 3060
   gagaacacca gcttgttaca ccctgtgagc ctgcatggga tggatgaccc ggagagagaa 3120
   gtgttagagt ggaggtttga cagccgccta gcatttcatc acgtggcccg agagctgcat 3180
   ccggagtact tcaagaactg ctga 3204
<210> 2
   <211> 1067
   <212> PRT
   <213> Artificial Sequence
<220>
   <223> HIV
<400> 2
   Met Gly Ala Arg Ala Ser Val Leu Ser Gly Gly Glu Leu Asp Arg Trp
    1               5                  10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Asn Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Val His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                   245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305 310 315 320
   Leu Leu Val Gln Asn Ala Asn Pro Asp Cys Lys Thr Ile Leu Lys Ala
   325 330 335
   Leu Gly Pro Ala Ala Thr Leu Glu Glu Met Met Thr Ala Cys Gln Gly
   340 345 350
   Val Gly Gly Pro Gly His Lys Ala Arg Val Leu Met Gly Pro Ile Ser
   355 360 365
   Pro Ile Glu Thr Val Pro Val Lys Leu Lys Pro Gly Met Asp Gly Pro
   370 375 380
   Lys Val Lys Gln Trp Pro Leu Thr Glu Glu Lys Ile Lys Ala Leu Val
   385 390 395 400
   Glu Ile Cys Thr Glu Met Glu Lys Glu Gly Lys Ile Ser Lys Ile Gly
   405 410 415
   Pro Glu Asn Pro Tyr Asn Thr Pro Val Phe Ala Ile Lys Lys Lys Asp
   420 425 430
   Ser Thr Lys Trp Arg Lys Leu Val Asp Phe Arg Glu Leu Asn Lys Arg
   435 440 445
   Thr Gln Asp Phe Trp Glu Val Gln Leu Gly Ile Pro His Pro Ala Gly
   450 455 460
   Leu Lys Lys Lys Lys Ser Val Thr Val Leu Asp Val Gly Asp Ala Tyr
   465 470 475 480
   Phe Ser Val Pro Leu Asp Glu Asp Phe Arg Lys Tyr Thr Ala Phe Thr
   485 490 495
   Ile Pro Ser Ile Asn Asn Glu Thr Pro Gly Ile Arg Tyr Gln Tyr Asn
   500 505 510
   Val Leu Pro Gln Gly Trp Lys Gly Ser Pro Ala Ile Phe Gln Ser Ser
   515 520 525
   Met Thr Lys Ile Leu Glu Pro Phe Arg Lys Gln Asn Pro Asp Ile Val
   530 535 540
   Ile Tyr Gln Tyr Met Asp Asp Leu Tyr Val Gly Ser Asp Leu Glu Ile
   545 550 555 560
   Gly Gln His Arg Thr Lys Ile Glu Glu Leu Arg Gln His Leu Leu Arg
   565 570 575
   Trp Gly Leu Thr Thr Pro Asp Lys Lys His Gln Lys Glu Pro Pro Phe
   580 585 590
   Leu Lys Met Gly Tyr Glu Leu His Pro Asp Lys Trp Thr Val Gln Pro
   595 600 605
   Ile Val Leu Pro Glu Lys Asp Ser Trp Thr Val Asn Asp Ile Gln Lys
   610 615 620
   Leu Val Gly Lys Leu Asn Trp Ala Ser Gln Ile Tyr Pro Gly Ile Lys
   625 630 635 640
   Val Arg Gln Leu Cys Lys Leu Leu Arg Gly Thr Lys Ala Leu Thr Glu
   645 650 655
   Val Ile Pro Leu Thr Glu Glu Ala Glu Leu Glu Leu Ala Glu Asn Arg
   660 665 670
   Glu Ile Leu Lys Glu Pro Val His Gly Val Tyr Tyr Asp Pro Ser Lys
   675 680 685
   Asp Leu Ile Ala Glu Ile Gln Lys Gln Gly Gln Gly Gln Trp Thr Tyr
   690 695 700
   Gln Ile Tyr Gln Glu Pro Phe Lys Asn Leu Lys Thr Gly Lys Tyr Ala
   705 710 715 720
   Arg Met Arg Gly Ala His Thr Asn Asp Val Lys Gln Leu Thr Glu Ala
   725 730 735
   Val Gln Lys Ile Thr Thr Glu Ser Ile Val Ile Trp Gly Lys Thr Pro
   740 745 750
   Lys Phe Lys Leu Pro Ile Gln Lys Glu Thr Trp Glu Thr Trp Trp Thr
   755 760 765
   Glu Tyr Trp Gln Ala Thr Trp Ile Pro Glu Trp Glu Phe Val Asn Thr
   770 775 780
   Pro Pro Leu Val Lys Leu Trp Tyr Gln Leu Glu Lys Glu Pro Ile Val
   785 790 795 800
   Gly Ala Glu Thr Phe Tyr Val Asp Gly Ala Ala Asn Arg Glu Thr Lys
                   805                 810                 815
   Leu Gly Lys Ala Gly Tyr Val Thr Asn Arg Gly Arg Gln Lys Val Val
               820                 825                 830
   Thr Leu Thr Asp Thr Thr Asn Gln Lys Thr Glu Leu Gln Ala Ile Tyr
           835                 840                 845
   Leu Ala Leu Gln Asp Ser Gly Leu Glu Val Asn Ile Val Thr Asp Ser
       850                 855                 860
   Gln Tyr Ala Leu Gly Ile Ile Gln Ala Gln Pro Asp Gln Ser Glu Ser
   865                 870                 875                 880
   Glu Leu Val Asn Gln Ile Ile Glu Gln Leu Ile Lys Lys Glu Lys Val
                   885                 890                 895
   Tyr Leu Ala Trp Val Pro Ala His Lys Gly Ile Gly Gly Asn Glu Gln
               900                 905                 910
   Val Asp Lys Leu Val Ser Ala Gly Ile Arg Lys Val Leu Met Val Gly
           915                 920                 925
   Phe Pro Val Thr Pro Gln Val Pro Leu Arg Pro Met Thr Tyr Lys Ala
       930                 935                 940
   Ala Val Asp Leu Ser His Phe Leu Lys Glu Lys Gly Gly Leu Glu Gly
   945                 950                 955                 960
   Leu Ile His Ser Gln Arg Arg Gln Asp Ile Leu Asp Leu Trp Ile Tyr
                   965                 970                 975
   His Thr Gln Gly Tyr Phe Pro Asp Trp Gln Asn Tyr Thr Pro Gly Pro
               980                 985                 990
   Gly Val Arg Tyr Pro Leu Thr Phe Gly Trp Cys Tyr Lys Leu Val Pro
           995                 1000                1005
   Val Glu Pro Asp Lys Val Glu Glu Ala Asn Lys Gly Glu Asn Thr Ser
       1010                1015                1020
   Leu Leu His Pro Val Ser Leu His Gly Met Asp Asp Pro Glu Arg Glu
   1025                1030                1035                1040
   Val Leu Glu Trp Arg Phe Asp Ser Arg Leu Ala Phe His His Val Ala
                   1045                1050                1055
   Arg Glu Leu His Pro Glu Tyr Phe Lys Asn Cys
               1060                1065
<210> 3
   <211> 4665
   <212> DNA
   <213> Artificial Sequence
<220>
   <223> HIV
<400> 3
   atggccgcca gagccagcat cctgagcggg ggcaagctgg acgcctggga gaagatcaga 60
   ctgaggcctg gcggcaagaa gaagtaccgg ctgaagcacc tggtgtgggc cagcagagag 120
   ctggatcgct tcgccctgaa tcctagcctg ctggagacca ccgagggctg ccagcagatc 180
   atgaaccagc tgcagcccgc cgtgaaaacc ggcaccgagg agatcaagag cctgttcaac 240
   accgtggcca ccctgtactg cgtgcaccag cggatcgacg tgaaggatac caaggaggcc 300
   ctggacaaga tcgaggagat ccagaacaag agcaagcaga aaacccagca ggccgctgcc 360
   gacaccggcg acagcagcaa agtgagccag aactacccca tcatccagaa tgcccagggc 420
   cagatgatcc accagaacct gagccccaga accctgaatg cctgggtgaa agtgatcgag 480
   gaaaaggcct tcagccccga agtgatccct atgttcagcg ccctgagcga gggcgccacc 540
   ccccaggacc tgaacgtgat gctgaacatt gtgggcggac accaggccgc catgcagatg 600
   ctgaaggaca ccatcaatga ggaggccgcc gagtgggaca gactgcaccc cgtgcaggcc 660
   ggacccatcc cccctggcca gatcagagag cccagaggca gcgacatcgc cggcaccacc 720
   tccacccctc aagaacagct gcagtggatg accggcaacc ctcccatccc tgtgggcaac 780
   atctacaagc ggtggatcat cctgggcctg aacaagattg tgcggatgta cagccccgtg 840
   tccatcctgg atatcaagca gggccccaag gagcccttca gagactacgt ggaccggttc 900
   ttcaaggccc tgagagccga gcaggccacc caggacgtga agggctggat gaccgagacc 960
   ctgctggtgc agaacgccaa ccccgactgc aagagcatcc tgaaggccct gggcagcggc 1020
   gccacactgg aggagatgat gaccgcctgc cagggagtgg gcggacccgg ccacaaggcc 1080
   agagtgctgg ccgaggccat gagccaggcc cagcagacca acatcatgat gcagcggggc 1140
   aacttcagag gccagaagcg gatcaagtgc ttcaactgcg gcaaggaggg ccacctggcc 1200
   agaaactgca gagcccccag gaagaagggc tgctggaagt gtggcaagga agggcaccag 1260
   atgaaggact gcaccgagag gcaggccaat ttcctgggca agatttggcc tagcagcaag 1320
   ggcagacccg gcaatttccc ccagagcaga cccgagccca ccgcccctcc cgccgagctg 1380
   ttcggcatgg gcgagggcat cgccagcctg cccaagcagg agcagaagga cagagagcag 1440
   gtgccccccc tggtgtccct gaagtccctg ttcggcaacg atcctctgag ccagggatcc 1500
   cccatcagcc ccatcgagac cgtgcccgtg accctgaagc ccggcatgga tggccccaaa 1560
   gtgaaacagt ggcccctgac cgaggagaag attaaggccc tgaccgaaat ctgtaccgag 1620
   atggagaagg agggcaagat cagcaagatc ggccccgaga acccctacaa cacccccatc 1680
   ttcgccatca agaagaagga cagcaccaag tggcggaaac tggtggactt ccgggagctg 1740
   aacaagagga cccaggactt ctgggaagtg cagctgggca tcccccaccc tgccggcctg 1800
   aagaagaaga agtccgtgac agtgctggat gtgggcgacg cctacttcag cgtgcccctg 1860
   gacgagaact tcaggaagta caccgccttc accatcccca gcaccaacaa cgagaccccc 1920
   ggagtgagat accagtacaa cgtgctgcct cagggctgga agggcagccc cgccatcttc 1980
   cagagcagca tgaccaagat cctggagccc ttccggagca agaaccccga gatcatcatc 2040
   taccagtaca tggccgccct gtatgtgggc agcgatctgg agatcggcca gcacaggacc 2100
   aagatcgaag agctgagggc ccacctgctg agctggggct tcaccacccc cgataagaag 2160
   caccagaagg agcccccttt cctgtggatg ggctacgagc tgcaccccga taagtggacc 2220
   gtgcagccca tcatgctgcc cgataaggag agctggaccg tgaacgacat ccagaaactg 2280
   gtgggcaagc tgaattgggc cagccaaatc tacgccggca ttaaagtgaa gcagctgtgc 2340
   aggctgctga gaggcgccaa agccctgaca gacatcgtga cactgacaga ggaggccgag 2400
   ctggagctgg ccgagaacag ggagatcctg aaggaccccg tgcacggcgt gtactacgac 2460
   cccagcaagg acctggtggc cgagattcag aagcagggcc aggaccagtg gacctaccaa 2520
   atctaccagg agcctttcaa gaacctgaaa accgggaagt acgccaggaa gagaagcgcc 2580
   cacaccaacg atgtgaggca gctggccgaa gtggtgcaga aagtggctat ggagagcatc 2640
   gtgatctggg gcaagacccc caagttcaag ctgcccatcc agaaggagac ctgggaaacc 2700
   tggtggatgg actactggca ggccacctgg attcctgagt gggagttcgt gaacaccccc 2760
   cctctggtga agctgtggta tcagctggag aaggacccca tcctgggcgc cgagaccttc 2820
   tacgtggacg gagccgccaa tagagagacc aagctgggca aggccggcta cgtgaccgac 2880
   agaggcagac agaaagtggt gtctctgacc gagacaacca accagaaaac cgagctgcac 2940
   gccatcctgc tggccctgca ggacagcggc agcgaagtga acatcgtgac cgactcccag 3000
   tacgccctgg gcatcattca ggcccagccc gatagaagcg agagcgagct ggtgaaccag 3060
   atcatcgaga agctgatcgg caaggacaaa atctacctga gctgggtgcc cgcccacaag 3120
   ggcatcggcg gcaacgagca ggtggacaag ctggtgtcca gcggcatccg gaaagtgctg 3180
   tttctggacg gcatcgacaa ggcccaggag gaccacgaga gataccacag caactggcgg 3240
   acaatggcca gcgacttcaa cctgcctccc atcgtggcca aggagatcgt ggccagctgc 3300
   gataagtgtc agctgaaggg cgaggccatg cacggccagg tggactgcag ccctggcatc 3360
   tggcagctgg cctgcaccca cctggagggc aaagtgattc tggtggccgt gcacgtggcc 3420
   agcggctaca tcgaggccga agtgattccc gccgagaccg gccaggagac cgcctacttc 3480
   ctgctgaagc tggccggcag atggcccgtg aaagtggtgc acaccgccaa cggcagcaac 3540
   ttcacctctg ccgccgtgaa ggccgcctgt tggtgggcca atatccagca ggagttcggc 3600
   atcccctaca accctcagag ccagggcgtg gtggccagca tgaacaagga gctgaagaag 3660
   atcatcggcc aggtgaggga ccaggccgag cacctgaaaa cagccgtgca gatggccgtg 3720
   ttcatccaca acttcaagcg gaagggcggc attggcggct acagcgccgg agagcggatc 3780
   atcgacatca tcgccaccga tatccagacc aaggaactgc agaagcagat caccaagatt 3840
   cagaacttca gagtgtacta ccgggacagc agggacccca tctggaaggg ccctgccaag 3900
   ctgctgtgga agggcgaagg cgccgtggtg atccaggaca acagcgacat caaagtggtg 3960
   ccccggagga aggccaagat tctgcgggac tacggcaaac agatggccgg cgatgactgc 4020
   gtggccggca ggcaggatga ggacagatct atgggcggca agtggtccaa gggcagcatt 4080
   gtgggctggc ccgagatccg ggagagaatg agaagagccc ctgccgccgc tcctggagtg 4140
   ggcgccgtgt ctcaggatct ggataagcac ggcgccatca ccagcagcaa catcaacaac 4200
   cccagctgtg tgtggctgga ggcccaggaa gaggaggaag tgggcttccc tgtgagaccc 4260
   caggtgcccc tgagacccat gacctacaag ggcgccttcg acctgagcca cttcctgaag 4320
   gagaagggcg gcctggacgg cctgatctac agccggaagc ggcaggagat cctggatctg 4380
   tgggtgtacc acacccaggg ctacttcccc gactggcaga attacacccc tggccctgga 4440
   gtgcggtatc ccctgacctt cggctggtgc ttcaagctgg tgcctatgga gcccgacgaa 4500
   gtggagaagg ccacagaggg cgagaacaac agcctgctgc accctatctg ccagcacggc 4560
   atggacgatg aggagcggga agtgctgatc tggaagttcg acagcaggct ggccctgaag 4620
   cacagagccc aggaactgca cccagagttc tacaaggact gctga 4665
<210> 4
   <211> 1554
   <212> PRT
   <213> Artificial Sequence
<220>
   <223> HIV
<400> 4
   Met Ala Ala Arg Ala Ser Ile Leu Ser Gly Gly Lys Leu Asp Ala Trp
    1               5                  10                  15
   Glu Lys Ile Arg Leu Arg Pro Gly Gly Lys Lys Lys Tyr Arg Leu Lys
               20                  25                  30
   His Leu Val Trp Ala Ser Arg Glu Leu Asp Arg Phe Ala Leu Asn Pro
           35                  40                  45
   Ser Leu Leu Glu Thr Thr Glu Gly Cys Gln Gln Ile Met Asn Gln Leu
       50                  55                  60
   Gln Pro Ala Val Lys Thr Gly Thr Glu Glu Ile Lys Ser Leu Phe Asn
   65                  70                  75                  80
   Thr Val Ala Thr Leu Tyr Cys Val His Gln Arg Ile Asp Val Lys Asp
                   85                  90                  95
   Thr Lys Glu Ala Leu Asp Lys Ile Glu Glu Ile Gln Asn Lys Ser Lys
               100                 105                 110
   Gln Lys Thr Gln Gln Ala Ala Ala Asp Thr Gly Asp Ser Ser Lys Val
           115                 120                 125
   Ser Gln Asn Tyr Pro Ile Ile Gln Asn Ala Gln Gly Gln Met Ile His
       130                 135                 140
   Gln Asn Leu Ser Pro Arg Thr Leu Asn Ala Trp Val Lys Val Ile Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Val Met Leu Asn Ile Val Gly
               180                 185                 190
   Gly His Gln Ala Ala Met Gln Met Leu Lys Asp Thr Ile Asn Glu Glu
           195                 200                 205
   Ala Ala Glu Trp Asp Arg Leu His Pro Val Gln Ala Gly Pro Ile Pro
       210                 215                 220
   Pro Gly Gln Ile Arg Glu Pro Arg Gly Ser Asp Ile Ala Gly Thr Thr
   225                 230                 235                 240
   Ser Thr Pro Gln Glu Gln Leu Gln Trp Met Thr Gly Asn Pro Pro Ile
                   245                 250                 255
   Pro Val Gly Asn Ile Tyr Lys Arg Trp Ile Ile Leu Gly Leu Asn Lys
               260                 265                 270
   Ile Val Arg Met Tyr Ser Pro Val Ser Ile Leu Asp Ile Lys Gln Gly
           275                 280                 285
   Pro Lys Glu Pro Phe Arg Asp Tyr Val Asp Arg Phe Phe Lys Ala Leu
       290                 295                 300
   Arg Ala Glu Gln Ala Thr Gln Asp Val Lys Gly Trp Met Thr Glu Thr
   305                 310                 315                 320
   Leu Leu Val Gln Asn Ala Asn Pro Asp Cys Lys Ser Ile Leu Lys Ala
                   325                 330                 335
   Leu Gly Ser Gly Ala Thr Leu Glu Glu Met Met Thr Ala Cys Gln Gly
               340                 345                 350
   Val Gly Gly Pro Gly His Lys Ala Arg Val Leu Ala Glu Ala Met Ser
           355                 360                 365
   Gln Ala Gln Gln Thr Asn Ile Met Met Gln Arg Gly Asn Phe Arg Gly
       370                 375                 380
   Gln Lys Arg Ile Lys Cys Phe Asn Cys Gly Lys Glu Gly His Leu Ala
   385                 390                 395                 400
   Arg Asn Cys Arg Ala Pro Arg Lys Lys Gly Cys Trp Lys Cys Gly Lys
                   405                 410                 415
   Glu Gly His Gln Met Lys Asp Cys Thr Glu Arg Gln Ala Asn Phe Leu
               420                 425                 430
   Gly Lys Ile Trp Pro Ser Ser Lys Gly Arg Pro Gly Asn Phe Pro Gln
           435                 440                 445
   Ser Arg Pro Glu Pro Thr Ala Pro Pro Ala Glu Leu Phe Gly Met Gly
       450                 455                 460
   Glu Gly Ile Ala Ser Leu Pro Lys Gln Glu Gln Lys Asp Arg Glu Gln
   465                 470                 475                 480
   Val Pro Pro Leu Val Ser Leu Lys Ser Leu Phe Gly Asn Asp Pro Leu
                   485                 490                 495
   Ser Gln Gly Ser Pro Ile Ser Pro Ile Glu Thr Val Pro Val Thr Leu
               500                 505                 510
   Lys Pro Gly Met Asp Gly Pro Lys Val Lys Gln Trp Pro Leu Thr Glu
           515                 520                 525
   Glu Lys Ile Lys Ala Leu Thr Glu Ile Cys Thr Glu Met Glu Lys Glu
       530                 535                 540
   Gly Lys Ile Ser Lys Ile Gly Pro Glu Asn Pro Tyr Asn Thr Pro Ile
   545                 550                 555                 560
   Phe Ala Ile Lys Lys Lys Asp Ser Thr Lys Trp Arg Lys Leu Val Asp
                   565                 570                 575
   Phe Arg Glu Leu Asn Lys Arg Thr Gln Asp Phe Trp Glu Val Gln Leu
               580                 585                 590
   Gly Ile Pro His Pro Ala Gly Leu Lys Lys Lys Lys Ser Val Thr Val
           595                 600                 605
   Leu Asp Val Gly Asp Ala Tyr Phe Ser Val Pro Leu Asp Glu Asn Phe
       610                 615                 620
   Arg Lys Tyr Thr Ala Phe Thr Ile Pro Ser Thr Asn Asn Glu Thr Pro
   625                 630                 635                 640
   Gly Val Arg Tyr Gln Tyr Asn Val Leu Pro Gln Gly Trp Lys Gly Ser
                   645                 650                 655
   Pro Ala Ile Phe Gln Ser Ser Met Thr Lys Ile Leu Glu Pro Phe Arg
               660                 665                 670
   Ser Lys Asn Pro Glu Ile Ile Ile Tyr Gln Tyr Met Ala Ala Leu Tyr
           675                 680                 685
   Val Gly Ser Asp Leu Glu Ile Gly Gln His Arg Thr Lys Ile Glu Glu
       690                 695                 700
   Leu Arg Ala His Leu Leu Ser Trp Gly Phe Thr Thr Pro Asp Lys Lys
   705                 710                 715                 720
   His Gln Lys Glu Pro Pro Phe Leu Trp Met Gly Tyr Glu Leu His Pro
                   725                 730                 735
   Asp Lys Trp Thr Val Gln Pro Ile Met Leu Pro Asp Lys Glu Ser Trp
               740                 745                 750
   Thr Val Asn Asp Ile Gln Lys Leu Val Gly Lys Leu Asn Trp Ala Ser
           755                 760                 765
   Gln Ile Tyr Ala Gly Ile Lys Val Lys Gln Leu Cys Arg Leu Leu Arg
       770                 775                 780
   Gly Ala Lys Ala Leu Thr Asp Ile Val Thr Leu Thr Glu Glu Ala Glu
   785                 790                 795                 800
   Leu Glu Leu Ala Glu Asn Arg Glu Ile Leu Lys Asp Pro Val His Gly
                   805                 810                 815
   Val Tyr Tyr Asp Pro Ser Lys Asp Leu Val Ala Glu Ile Gln Lys Gln
               820                 825                 830
   Gly Gln Asp Gln Trp Thr Tyr Gln Ile Tyr Gln Glu Pro Phe Lys Asn
           835                 840                 845
   Leu Lys Thr Gly Lys Tyr Ala Arg Lys Arg Ser Ala His Thr Asn Asp
       850                 855                 860
   Val Arg Gln Leu Ala Glu Val Val Gln Lys Val Ala Met Glu Ser Ile
   865                 870                 875                 880
   Val Ile Trp Gly Lys Thr Pro Lys Phe Lys Leu Pro Ile Gln Lys Glu
                   885                 890                 895
   Thr Trp Glu Thr Trp Trp Met Asp Tyr Trp Gln Ala Thr Trp Ile Pro
               900                 905                 910
   Glu Trp Glu Phe Val Asn Thr Pro Pro Leu Val Lys Leu Trp Tyr Gln
           915                 920                 925
   Leu Glu Lys Asp Pro Ile Leu Gly Ala Glu Thr Phe Tyr Val Asp Gly
       930                 935                 940
   Ala Ala Asn Arg Glu Thr Lys Leu Gly Lys Ala Gly Tyr Val Thr Asp
   945                 950                 955                 960
   Arg Gly Arg Gln Lys Val Val Ser Leu Thr Glu Thr Thr Asn Gln Lys
   965 970 975
   Thr Glu Leu His Ala Ile Leu Leu Ala Leu Gln Asp Ser Gly Ser Glu
   980 985 990
   Val Asn Ile Val Thr Asp Ser Gln Tyr Ala Leu Gly Ile Ile Gln Ala
   995 1000 1005
   Gln Pro Asp Arg Ser Glu Ser Glu Leu Val Asn Gln Ile Ile Glu Lys
   1010 1015 1020
   Leu Ile Gly Lys Asp Lys Ile Tyr Leu Ser Trp Val Pro Ala His Lys
   1025 1030 1035 1040
   Gly Ile Gly Gly Asn Glu Gln Val Asp Lys Leu Val Ser Ser Gly Ile
   1045 1050 1055
   Arg Lys Val Leu Phe Leu Asp Gly Ile Asp Lys Ala Gln Glu Asp His
   1060 1065 1070
   Glu Arg Tyr His Ser Asn Trp Arg Thr Met Ala Ser Asp Phe Asn Leu
   1075 1080 1085
   Pro Pro Ile Val Ala Lys Glu Ile Val Ala Ser Cys Asp Lys Cys Gln
   1090 1095 1100
   Leu Lys Gly Glu Ala Met His Gly Gln Val Asp Cys Ser Pro Gly Ile
   1105 1110 1115 1120
   Trp Gln Leu Ala Cys Thr His Leu Glu Gly Lys Val Ile Leu Val Ala
   1125 1130 1135
   Val His Val Ala Ser Gly Tyr Ile Glu Ala Glu Val Ile Pro Ala Glu
   1140 1145 1150
   Thr Gly Gln Glu Thr Ala Tyr Phe Leu Leu Lys Leu Ala Gly Arg Trp
   1155 1160 1165
   Pro Val Lys Val Val His Thr Ala Asn Gly Ser Asn Phe Thr Ser Ala
   1170 1175 1180
   Ala Val Lys Ala Ala Cys Trp Trp Ala Asn Ile Gln Gln Glu Phe Gly
   1185 1190 1195 1200
   Ile Pro Tyr Asn Pro Gln Ser Gln Gly Val Val Ala Ser Met Asn Lys
   1205 1210 1215
   Glu Leu Lys Lys Ile Ile Gly Gln Val Arg Asp Gln Ala Glu His Leu
   1220 1225 1230
   Lys Thr Ala Val Gln Met Ala Val Phe Ile His Asn Phe Lys Arg Lys
   1235 1240 1245
   Gly Gly Ile Gly Gly Tyr Ser Ala Gly Glu Arg Ile Ile Asp Ile Ile
   1250 1255 1260
   Ala Thr Asp Ile Gln Thr Lys Glu Leu Gln Lys Gln Ile Thr Lys Ile
   1265 1270 1275 1280
   Gln Asn Phe Arg Val Tyr Tyr Arg Asp Ser Arg Asp Pro Ile Trp Lys
   1285 1290 1295
   Gly Pro Ala Lys Leu Leu Trp Lys Gly Glu Gly Ala Val Val Ile Gln
   1300 1305 1310
   Asp Asn Ser Asp Ile Lys Val Val Pro Arg Arg Lys Ala Lys Ile Leu
   1315 1320 1325
   Arg Asp Tyr Gly Lys Gln Met Ala Gly Asp Asp Cys Val Ala Gly Arg
   1330 1335 1340
   Gln Asp Glu Asp Arg Ser Met Gly Gly Lys Trp Ser Lys Gly Ser Ile
   1345 1350 1355 1360
   Val Gly Trp Pro Glu Ile Arg Glu Arg Met Arg Arg Ala Pro Ala Ala
   1365 1370 1375
   Ala Pro Gly Val Gly Ala Val Ser Gln Asp Leu Asp Lys His Gly Ala
   1380 1385 1390
   Ile Thr Ser Ser Asn Ile Asn Asn Pro Ser Cys Val Trp Leu Glu Ala
   1395 1400 1405
   Gln Glu Glu Glu Glu Val Gly Phe Pro Val Arg Pro Gln Val Pro Leu
   1410 1415 1420
   Arg Pro Met Thr Tyr Lys Gly Ala Phe Asp Leu Ser His Phe Leu Lys
   1425 1430 1435 1440
   Glu Lys Gly Gly Leu Asp Gly Leu Ile Tyr Ser Arg Lys Arg Gln Glu
   1445 1450 1455
   Ile Leu Asp Leu Trp Val Tyr His Thr Gln Gly Tyr Phe Pro Asp Trp
                   1460                1465                1470
   Gln Asn Tyr Thr Pro Gly Pro Gly Val Arg Tyr Pro Leu Thr Phe Gly
               1475                1480                1485
   Trp Cys Phe Lys Leu Val Pro Met Glu Pro Asp Glu Val Glu Lys Ala
           1490                1495                1500
   Thr Glu Gly Glu Asn Asn Ser Leu Leu His Pro Ile Cys Gln His Gly
   1505                1510                1515                1520
   Met Asp Asp Glu Glu Arg Glu Val Leu Ile Trp Lys Phe Asp Ser Arg
                       1525                1530                1535
   Leu Ala Leu Lys His Arg Ala Gln Glu Leu His Pro Glu Phe Tyr Lys
                   1540                1545                1550
   Asp Cys
<210> 5
   <211> 2025
   <212> DNA
   <213> Artificial Sequence
<220>
   <223> HIV
<400> 5
   atgagggtga tggagatcca gcggaactgc cagcacctgc tgagatgggg catcatgatc 60
   ctgggcatga ttatcatctg cagcaccgcc gacaacctgt gggtgaccgt gtactacggc 120
   gtgcctgtgt ggagagatgc cgagaccacc ctgttctgcg ccagcgacgc caaggcctac 180
   agcaccgaga agcacaatgt gtgggccacc cacgcctgcg tgcctaccga tcccaaccct 240
   caggagatcc ccctggacaa cgtgaccgag gagttcaaca tgtggaagaa caacatggtg 300
   gaccagatgc acgaggacat catcagcctg tgggaccaga gcctgaagcc ctgcgtgcag 360
   ctgacccccc tgtgcgtgac cctgaactgc agcaacgcca gagtgaacgc caccttcaac 420
   tccaccgagg acagggaggg catgaagaac tgcagcttca acatgaccac cgagctgcgg 480
   gataagaagc agcaggtgta cagcctgttc taccggctgg acatcgagaa gatcaacagc 540
   agcaacaaca acagcgagta ccggctggtg aactgcaata ccagcgccat cacccaggcc 600
   tgccctaagg tgaccttcga gcccatcccc atccactact gcgcccctgc cggcttcgcc 660
   atcctgaagt gcaacgacac cgagttcaat ggcaccggcc cctgcaagaa tgtgagcacc 720
   gtgcagtgca cccacggcat caagcccgtg gtgtccaccc agctgctgct gaacggcagc 780
   ctggccgaga gagaagtgcg gatcaggagc gagaacatcg ccaacaacgc caagaacatc 840
   atcgtgcagt tcgccagccc cgtgaagatc aactgcatcc ggcccaacaa caatacccgg 900
   aagagctaca gaatcggccc tggccagacc ttctacgcca ccgacattgt gggcgacatc 960
   agacaggccc actgcaacgt gtccaggacc gactggaaca acaccctgag actggtggcc 1020
   aaccagctgc ggaagtactt cagcaacaag accatcatct tcaccaacag cagcggcgga 1080
   gacctggaga tcaccaccca cagcttcaat tgtggcggcg agttcttcta ctgcaacacc 1140
   tccggcctgt tcaatagcac ctggaccacc aacaacatgc aggagtccaa cgacaccagc 1200
   aacggcacca tcaccctgcc ctgccggatc aagcagatca tccggatgtg gcagcgcgtg 1260
   ggccaggcca tgtacgcccc tcccatcgag ggcgtgattc gctgcgagag caacatcacc 1320
   ggcctgatcc tgaccagaga tggcggcaac aacaattccg ccaacgagac cttcagacct 1380
   ggcggcggag atatccggga caactggcgg agcgagctgt acaagtacaa ggtggtgaag 1440
   atcgagcccc tgggcgtggc ccccaccaga gccaagagaa gagtggtgga gcgggagaag 1500
   agagccgtgg gcatcggcgc cgtgtttctg ggcttcctgg gagccgccgg atctacaatg 1560
   ggagccgcca gcatcaccct gaccgtgcag gccagacagc tgctgagcgg catcgtgcag 1620
   cagcagagca atctgctgag agccatcgag gcccagcagc agctgctgaa gctgacagtg 1680
   tggggcatca agcagctgca ggccagggtg ctggccgtgg agagatacct gagggaccag 1740
   cagctcctgg gcatctgggg ctgcagcggc aagctgatct gcaccaccaa cgtgccctgg 1800
   aatagcagct ggagcaacaa gagctacgac gacatctggc agaacatgac ctggctgcag 1860
   tgggacaagg agatcagcaa ctacaccgac atcatctaca gcctgatcga ggagagccag 1920
   aaccagcagg agaagaacga gcaggatctg ctggccctgg acaagtgggc caacctgtgg 1980
   aactggttcg acatcagcaa gtggctgtgg tacatcagat cttga                2025
<210> 6
   <211> 674
   <212> PRT
   <213> Artificial Sequence
<220>
   <223> HIV
<400> 6
   Met Arg Val Met Glu Ile Gln Arg Asn Cys Gln His Leu Leu Arg Trp
   1               5                   10                  15
   Gly Ile Met Ile Leu Gly Met Ile Ile Ile Cys Ser Thr Ala Asp Asn
               20                  25                  30
   Leu Trp Val Thr Val Tyr Tyr Gly Val Pro Val Trp Arg Asp Ala Glu
           35                  40                  45
   Thr Thr Leu Phe Cys Ala Ser Asp Ala Lys Ala Tyr Ser Thr Glu Lys
       50                  55                  60
   His Asn Val Trp Ala Thr His Ala Cys Val Pro Thr Asp Pro Asn Pro
   65                  70                  75                  80
   Gln Glu Ile Pro Leu Asp Asn Val Thr Glu Glu Phe Asn Met Trp Lys
                   85                  90                  95
   Asn Asn Met Val Asp Gln Met His Glu Asp Ile Ile Ser Leu Trp Asp
               100                 105                 110
   Gln Ser Leu Lys Pro Cys Val Gln Leu Thr Pro Leu Cys Val Thr Leu
           115                 120                 125
   Asn Cys Ser Asn Ala Arg Val Asn Ala Thr Phe Asn Ser Thr Glu Asp
       130                 135                 140
   Arg Glu Gly Met Lys Asn Cys Ser Phe Asn Met Thr Thr Glu Leu Arg
   145                 150                 155                 160
   Asp Lys Lys Gln Gln Val Tyr Ser Leu Phe Tyr Arg Leu Asp Ile Glu
                   165                 170                 175
   Lys Ile Asn Ser Ser Asn Asn Asn Ser Glu Tyr Arg Leu Val Asn Cys
               180                 185                 190
   Asn Thr Ser Ala Ile Thr Gln Ala Cys Pro Lys Val Thr Phe Glu Pro
           195                 200                 205
   Ile Pro Ile His Tyr Cys Ala Pro Ala Gly Phe Ala Ile Leu Lys Cys
       210                 215                 220
   Asn Asp Thr Glu Phe Asn Gly Thr Gly Pro Cys Lys Asn Val Ser Thr
   225                 230                 235                 240
   Val Gln Cys Thr His Gly Ile Lys Pro Val Val Ser Thr Gln Leu Leu
                   245                 250                 255
   Leu Asn Gly Ser Leu Ala Glu Arg Glu Val Arg Ile Arg Ser Glu Asn
               260                 265                 270
   Ile Ala Asn Asn Ala Lys Asn Ile Ile Val Gln Phe Ala Ser Pro Val
           275                 280                 285
   Lys Ile Asn Cys Ile Arg Pro Asn Asn Asn Thr Arg Lys Ser Tyr Arg
       290                 295                 300
   Ile Gly Pro Gly Gln Thr Phe Tyr Ala Thr Asp Ile Val Gly Asp Ile
   305                 310                 315                 320
   Arg Gln Ala His Cys Asn Val Ser Arg Thr Asp Trp Asn Asn Thr Leu
                   325                 330                 335
   Arg Leu Val Ala Asn Gln Leu Arg Lys Tyr Phe Ser Asn Lys Thr Ile
               340                 345                 350
   Ile Phe Thr Asn Ser Ser Gly Gly Asp Leu Glu Ile Thr Thr His Ser
           355                 360                 365
   Phe Asn Cys Gly Gly Glu Phe Phe Tyr Cys Asn Thr Ser Gly Leu Phe
       370                 375                 380
   Asn Ser Thr Trp Thr Thr Asn Asn Met Gln Glu Ser Asn Asp Thr Ser
   385                 390                 395                 400
   Asn Gly Thr Ile Thr Leu Pro Cys Arg Ile Lys Gln Ile Ile Arg Met
                   405                 410                 415
   Trp Gln Arg Val Gly Gln Ala Met Tyr Ala Pro Pro Ile Glu Gly Val
               420                 425                 430
   Ile Arg Cys Glu Ser Asn Ile Thr Gly Leu Ile Leu Thr Arg Asp Gly
           435                 440                 445
   Gly Asn Asn Asn Ser Ala Asn Glu Thr Phe Arg Pro Gly Gly Gly Asp
       450                 455                 460
   Ile Arg Asp Asn Trp Arg Ser Glu Leu Tyr Lys Tyr Lys Val Val Lys
   465                 470                 475                 480
   Ile Glu Pro Leu Gly Val Ala Pro Thr Arg Ala Lys Arg Arg Val Val
                   485                 490                 495
   Glu Arg Glu Lys Arg Ala Val Gly Ile Gly Ala Val Phe Leu Gly Phe
               500                 505                 510
   Leu Gly Ala Ala Gly Ser Thr Met Gly Ala Ala Ser Ile Thr Leu Thr
           515                 520                 525
   Val Gln Ala Arg Gln Leu Leu Ser Gly Ile Val Gln Gln Gln Ser Asn
       530                 535                 540
   Leu Leu Arg Ala Ile Glu Ala Gln Gln Gln Leu Leu Lys Leu Thr Val
   545                 550                 555                 560
   Trp Gly Ile Lys Gln Leu Gln Ala Arg Val Leu Ala Val Glu Arg Tyr
                   565                 570                 575
   Leu Arg Asp Gln Gln Leu Leu Gly Ile Trp Gly Cys Ser Gly Lys Leu
               580                 585                 590
   Ile Cys Thr Thr Asn Val Pro Trp Asn Ser Ser Trp Ser Asn Lys Ser
           595                 600                 605
   Tyr Asp Asp Ile Trp Gln Asn Met Thr Trp Leu Gln Trp Asp Lys Glu
       610                 615                 620
   Ile Ser Asn Tyr Thr Asp Ile Ile Tyr Ser Leu Ile Glu Glu Ser Gln
   625                 630                 635                 640
   Asn Gln Gln Glu Lys Asn Glu Gln Asp Leu Leu Ala Leu Asp Lys Trp
                   645                 650                 655
   Ala Asn Leu Trp Asn Trp Phe Asp Ile Ser Lys Trp Leu Trp Tyr Ile
               660                 665                 670
   Arg Ser
<210> 7
   <211> 1545
   <212> DNA
   <213> Artificial Sequence
<220>
   <223> HIV
<400> 7
   atgaaagtga aggagaccag gaagaattat cagcacttgt ggagatgggg caccatgctc 60
   cttgggatgt tgatgatctg tagtgctgca gaacaattgt gggtcacagt ctattatggg 120
   gtacctgtgt ggaaagaagc aactaccact ctattctgtg catcagatgc taaagcatat 180
   gatacagagg tacataatgt ttgggccaca catgcctgtg tacccacaga ccccaaccca 240
   caagaagtag tattgggaaa tgtgacagaa tattttaaca tgtggaaaaa taacatggta 300
   gaccagatgc atgaggatat aatcagttta tgggatcaaa gcttgaagcc atgtgtaaaa 360
   ttaaccccac tctgtgttac tttagattgc gatgatgtga ataccactaa tagtactact 420
   accactagta atggttggac aggagaaata aggaaaggag aaataaaaaa ctgctctttt 480
   aatatcacca caagcataag agataaggtt caaaaagaat atgcactttt ttataacctt 540
   gatgtagtac caatagatga tgataatgct actaccaaaa ataaaactac tagaaacttt 600
   aggttgatac attgtaactc ctcagtcatg acacaggcct gtccaaaggt atcatttgaa 660
   ccaattccca tacattattg tgccccggct ggttttgcga ttctgaagtg taacaataag 720
   acgtttgatg gaaaaggact atgtacaaat gtcagcacag tacaatgtac acatggaatt 780
   aggccagtag tgtcaactca actgctgtta aatggcagtc tagcagaaga agaggtagta 840
   attagatctg acaatttcat ggacaatact aaaaccataa tagtacagct gaatgaatct 900
   gtagcaatta attgtacaag acccaacaac aatacaagaa aaggtataca tataggacca 960
   gggagagcct tttatgcagc aagaaaaata ataggagata taagacaagc acattgtaac 1020
   cttagtagag cacaatggaa taacacttta aaacagatag ttataaaatt aagagaacac 1080
   tttgggaata aaacaataaa atttaatcaa tcctcaggag gggacccaga aattgtaagg 1140
   catagtttta attgtggagg ggaatttttc tactgtgata caacacaact gtttaatagt 1200
   acttggaatg gtactgaagg aaataacact gaaggaaata gcacaatcac actcccatgt 1260
   agaataaaac aaattataaa catgtggcag gaagtaggaa aagcaatgta tgcccctccc 1320
   atcggaggac aaattagatg ttcatcaaat attacagggc tgctattaac aagagatggt 1380
   ggtaccgaag ggaatgggac agagaatgag acagagatct tcagacctgg aggaggagat 1440
   atgagggaca attggagaag tgaattatat aaatataaag tagtaaaagt tgaaccacta 1500
   ggagtagcac ccaccagggc aaagagaaga gtggtgcaga gataa                 1545
<210> 8
   <211> 514
   <212> PRT
   <213> Artificial Sequence
<220>
   <223> HIV
<400> 8
   Met Lys Val Lys Glu Thr Arg Lys Asn Tyr Gln His Leu Trp Arg Trp
    1               5                  10                  15
   Gly Thr Met Leu Leu Gly Met Leu Met Ile Cys Ser Ala Ala Glu Gln
               20                  25                  30
   Leu Trp Val Thr Val Tyr Tyr Gly Val Pro Val Trp Lys Glu Ala Thr
           35                  40                  45
   Thr Thr Leu Phe Cys Ala Ser Asp Ala Lys Ala Tyr Asp Thr Glu Val
       50                  55                  60
   His Asn Val Trp Ala Thr His Ala Cys Val Pro Thr Asp Pro Asn Pro
   65                  70                  75                  80
   Gln Glu Val Val Leu Gly Asn Val Thr Glu Tyr Phe Asn Met Trp Lys
                   85                  90                  95
   Asn Asn Met Val Asp Gln Met His Glu Asp Ile Ile Ser Leu Trp Asp
               100                 105                 110
   Gln Ser Leu Lys Pro Cys Val Lys Leu Thr Pro Leu Cys Val Thr Leu
           115                 120                 125
   Asp Cys Asp Asp Val Asn Thr Thr Asn Ser Thr Thr Thr Thr Ser Asn
       130                 135                 140
   Gly Trp Thr Gly Glu Ile Arg Lys Gly Glu Ile Lys Asn Cys Ser Phe
   145                 150                 155                 160
   Asn Ile Thr Thr Ser Ile Arg Asp Lys Val Gln Lys Glu Tyr Ala Leu
                   165                 170                 175
   Phe Tyr Asn Leu Asp Val Val Pro Ile Asp Asp Asp Asn Ala Thr Thr
               180                 185                 190
   Lys Asn Lys Thr Thr Arg Asn Phe Arg Leu Ile His Cys Asn Ser Ser
           195                 200                 205
   Val Met Thr Gln Ala Cys Pro Lys Val Ser Phe Glu Pro Ile Pro Ile
       210                 215                 220
   His Tyr Cys Ala Pro Ala Gly Phe Ala Ile Leu Lys Cys Asn Asn Lys
   225                 230                 235                 240
   Thr Phe Asp Gly Lys Gly Leu Cys Thr Asn Val Ser Thr Val Gln Cys
                   245                 250                 255
   Thr His Gly Ile Arg Pro Val Val Ser Thr Gln Leu Leu Leu Asn Gly
               260                 265                 270
   Ser Leu Ala Glu Glu Glu Val Val Ile Arg Ser Asp Asn Phe Met Asp
           275                 280                 285
   Asn Thr Lys Thr Ile Ile Val Gln Leu Asn Glu Ser Val Ala Ile Asn
       290                 295                 300
   Cys Thr Arg Pro Asn Asn Asn Thr Arg Lys Gly Ile His Ile Gly Pro
   305                 310                 315                 320
   Gly Arg Ala Phe Tyr Ala Ala Arg Lys Ile Ile Gly Asp Ile Arg Gln
                   325                 330                 335
   Ala His Cys Asn Leu Ser Arg Ala Gln Trp Asn Asn Thr Leu Lys Gln
               340                 345                 350
   Ile Val Ile Lys Leu Arg Glu His Phe Gly Asn Lys Thr Ile Lys Phe
           355                 360                 365
   Asn Gln Ser Ser Gly Gly Asp Pro Glu Ile Val Arg His Ser Phe Asn
       370                 375                 380
   Cys Gly Gly Glu Phe Phe Tyr Cys Asp Thr Thr Gln Leu Phe Asn Ser
   385                 390                 395                 400
   Thr Trp Asn Gly Thr Glu Gly Asn Asn Thr Glu Gly Asn Ser Thr Ile
                           405                 410                 415
   Thr Leu Pro Cys Arg Ile Lys Gln Ile Ile Asn Met Trp Gln Glu Val
                   420                 425                 430
   Gly Lys Ala Met Tyr Ala Pro Pro Ile Gly Gly Gln Ile Arg Cys Ser
               435                 440                 445
   Ser Asn Ile Thr Gly Leu Leu Leu Thr Arg Asp Gly Gly Thr Glu Gly
       450                 455                 460
   Asn Gly Thr Glu Asn Glu Thr Glu Ile Phe Arg Pro Gly Gly Gly Asp
   465                 470                 475                 480
   Met Arg Asp Asn Trp Arg Ser Glu Leu Tyr Lys Tyr Lys Val Val Lys
                   485                 490                 495
   Val Glu Pro Leu Gly Val Ala Pro Thr Arg Ala Lys Arg Arg Val Val
               500                 505                 510
   Gln Arg
<210> 9
   <211> 2178
   <212> DNA
   <213> Artificial Sequence
<220>
   <223> Mycobacterium tuberculosis
<400> 9
   atgcatcaca cggccgcgtc cgataacttc cagctgtccc agggtgggca gggattcgcc 60
   attccgatcg ggcaggcgat ggcgatcgcg ggccagatcc gatcgggtgg ggggtcaccc 120
   accgttcata tcgggcctac cgccttcctc ggcttgggtg ttgtcgacaa caacggcaac 180
   ggcgcacgag tccaacgcgt ggtcgggagc gctccggcgg caagtctcgg catctccacc 240
   ggcgacgtga tcaccgcggt cgacggcgct ccgatcaact cggccaccgc gatggcggac 300
   gcgcttaacg ggcatcatcc cggtgacgtc atctcggtga cctggcaaac caagtcgggc 360
   ggcacgcgta cagggaacgt gacattggcc gagggacccc cggccgaatt catggtggat 420
   ttcggggcgt taccaccgga gatcaactcc gcgaggatgt acgccggccc gggttcggcc 480
   tcgctggtgg ccgcggctca gatgtgggac agcgtggcga gtgacctgtt ttcggccgcg 540
   tcggcgtttc agtcggtggt ctggggtctg acggtggggt cgtggatagg ttcgtcggcg 600
   ggtctgatgg tggcggcggc ctcgccgtat gtggcgtgga tgagcgtcac cgcggggcag 660
   gccgagctga ccgccgccca ggtccgggtt gctgcggcgg cctacgagac ggcgtatggg 720
   ctgacggtgc ccccgccggt gatcgccgag aaccgtgctg aactgatgat tctgatagcg 780
   accaacctct tggggcaaaa caccccggcg atcgcggtca acgaggccga atacggcgag 840
   atgtgggccc aagacgccgc cgcgatgttt ggctacgccg cggcgacggc gacggcgacg 900
   gcgacgttgc tgccgttcga ggaggcgccg gagatgacca gcgcgggtgg gctcctcgag 960
   caggccgccg cggtcgagga ggcctccgac accgccgcgg cgaaccagtt gatgaacaat 1020
   gtgccccagg cgctgcaaca gctggcccag cccacgcagg gcaccacgcc ttcttccaag 1080
   ctgggtggcc tgtggaagac ggtctcgccg catcggtcgc cgatcagcaa catggtgtcg 1140
   atggccaaca accacatgtc gatgaccaac tcgggtgtgt cgatgaccaa caccttgagc 1200
   tcgatgttga agggctttgc tccggcggcg gccgcccagg ccgtgcaaac cgcggcgcaa 1260
   aacggggtcc gggcgatgag ctcgctgggc agctcgctgg gttcttcggg tctgggcggt 1320
   ggggtggccg ccaacttggg tcgggcggcc tcggtcggtt cgttgtcggt gccgcaggcc 1380
   tgggccgcgg ccaaccaggc agtcaccccg gcggcgcggg cgctgccgct gaccagcctg 1440
   accagcgccg cggaaagagg gcccgggcag atgctgggcg ggctgccggt ggggcagatg 1500
   ggcgccaggg ccggtggtgg gctcagtggt gtgctgcgtg ttccgccgcg accctatgtg 1560
   atgccgcatt ctccggcagc cggcgatatc gccccgccgg ccttgtcgca ggaccggttc 1620
   gccgacttcc ccgcgctgcc cctcgacccg tccgcgatgg tcgcccaagt ggggccacag 1680
   gtggtcaaca tcaacaccaa actgggctac aacaacgccg tgggcgccgg gaccggcatc 1740
   gtcatcgatc ccaacggtgt cgtgctgacc aacaaccacg tgatcgcggg cgccaccgac 1800
   atcaatgcgt tcagcgtcgg ctccggccaa acctacggcg tcgatgtggt cgggtatgac 1860
   cgcacccagg atgtcgcggt gctgcagctg cgcggtgccg gtggcctgcc gtcggcggcg 1920
   atcggtggcg gcgtcgcggt tggtgagccc gtcgtcgcga tgggcaacag cggtgggcag 1980
   ggcggaacgc cccgtgcggt gcctggcagg gtggtcgcgc tcggccaaac cgtgcaggcg 2040
   tcggattcgc tgaccggtgc cgaagagaca ttgaacgggt tgatccagtt cgatgccgcg 2100
   atccagcccg gtgatgcggg cgggcccgtc gtcaacggcc taggacaggt ggtcggtatg 2160
   aacacggccg cgtcctag                                               2178
<210> 10
   <211> 725
   <212> PRT
   <213> Artificial Sequence
<220>
   <223> Mycobacterium tuberculosis
<400> 10
   Met His His Thr Ala Ala Ser Asp Asn Phe Gln Leu Ser Gln Gly Gly
    1               5                  10                  15
   Gln Gly Phe Ala Ile Pro Ile Gly Gln Ala Met Ala Ile Ala Gly Gln
               20                  25                  30
   Ile Arg Ser Gly Gly Gly Ser Pro Thr Val His Ile Gly Pro Thr Ala
           35                  40                  45
   Phe Leu Gly Leu Gly Val Val Asp Asn Asn Gly Asn Gly Ala Arg Val
       50                  55                  60
   Gln Arg Val Val Gly Ser Ala Pro Ala Ala Ser Leu Gly Ile Ser Thr
   65                  70                  75                  80
   Gly Asp Val Ile Thr Ala Val Asp Gly Ala Pro Ile Asn Ser Ala Thr
                   85                  90                  95
   Ala Met Ala Asp Ala Leu Asn Gly His His Pro Gly Asp Val Ile Ser
               100                 105                 110
   Val Thr Trp Gln Thr Lys Ser Gly Gly Thr Arg Thr Gly Asn Val Thr
           115                 120                 125
   Leu Ala Glu Gly Pro Pro Ala Glu Phe Met Val Asp Phe Gly Ala Leu
       130                 135                 140
   Pro Pro Glu Ile Asn Ser Ala Arg Met Tyr Ala Gly Pro Gly Ser Ala
   145                 150                 155                 160
   Ser Leu Val Ala Ala Ala Gln Met Trp Asp Ser Val Ala Ser Asp Leu
                   165                 170                 175
   Phe Ser Ala Ala Ser Ala Phe Gln Ser Val Val Trp Gly Leu Thr Val
               180                 185                 190
   Gly Ser Trp Ile Gly Ser Ser Ala Gly Leu Met Val Ala Ala Ala Ser
           195                 200                 205
   Pro Tyr Val Ala Trp Met Ser Val Thr Ala Gly Gln Ala Glu Leu Thr
       210                 215                 220
   Ala Ala Gln Val Arg Val Ala Ala Ala Ala Tyr Glu Thr Ala Tyr Gly
   225                 230                 235                 240
   Leu Thr Val Pro Pro Pro Val Ile Ala Glu Asn Arg Ala Glu Leu Met
                   245                 250                 255
   Ile Leu Ile Ala Thr Asn Leu Leu Gly Gln Asn Thr Pro Ala Ile Ala
               260                 265                 270
   Val Asn Glu Ala Glu Tyr Gly Glu Met Trp Ala Gln Asp Ala Ala Ala
           275                 280                 285
   Met Phe Gly Tyr Ala Ala Ala Thr Ala Thr Ala Thr Ala Thr Leu Leu
       290                 295                 300
   Pro Phe Glu Glu Ala Pro Glu Met Thr Ser Ala Gly Gly Leu Leu Glu
   305                 310                 315                 320
   Gln Ala Ala Ala Val Glu Glu Ala Ser Asp Thr Ala Ala Ala Asn Gln
                   325                 330                 335
   Leu Met Asn Asn Val Pro Gln Ala Leu Gln Gln Leu Ala Gln Pro Thr
               340                 345                 350
   Gln Gly Thr Thr Pro Ser Ser Lys Leu Gly Gly Leu Trp Lys Thr Val
           355                 360                 365
   Ser Pro His Arg Ser Pro Ile Ser Asn Met Val Ser Met Ala Asn Asn
       370                 375                 380
   His Met Ser Met Thr Asn Ser Gly Val Ser Met Thr Asn Thr Leu Ser
   385                 390                 395                 400
   Ser Met Leu Lys Gly Phe Ala Pro Ala Ala Ala Ala Gln Ala Val Gln
                   405                 410                 415
   Thr Ala Ala Gln Asn Gly Val Arg Ala Met Ser Ser Leu Gly Ser Ser
                   420                 425                 430
   Leu Gly Ser Ser Gly Leu Gly Gly Gly Val Ala Ala Asn Leu Gly Arg
               435                 440                 445
   Ala Ala Ser Val Gly Ser Leu Ser Val Pro Gln Ala Trp Ala Ala Ala
       450                 455                 460
   Asn Gln Ala Val Thr Pro Ala Ala Arg Ala Leu Pro Leu Thr Ser Leu
   465                 470                 475                 480
   Thr Ser Ala Ala Glu Arg Gly Pro Gly Gln Met Leu Gly Gly Leu Pro
                   485                 490                 495
   Val Gly Gln Met Gly Ala Arg Ala Gly Gly Gly Leu Ser Gly Val Leu
               500                 505                 510
   Arg Val Pro Pro Arg Pro Tyr Val Met Pro His Ser Pro Ala Ala Gly
           515                 520                 525
   Asp Ile Ala Pro Pro Ala Leu Ser Gln Asp Arg Phe Ala Asp Phe Pro
       530                 535                 540
   Ala Leu Pro Leu Asp Pro Ser Ala Met Val Ala Gln Val Gly Pro Gln
   545                 550                 555                 560
   Val Val Asn Ile Asn Thr Lys Leu Gly Tyr Asn Asn Ala Val Gly Ala
                   565                 570                 575
   Gly Thr Gly Ile Val Ile Asp Pro Asn Gly Val Val Leu Thr Asn Asn
               580                 585                 590
   His Val Ile Ala Gly Ala Thr Asp Ile Asn Ala Phe Ser Val Gly Ser
           595                 600                 605
   Gly Gln Thr Tyr Gly Val Asp Val Val Gly Tyr Asp Arg Thr Gln Asp
       610                 615                 620
   Val Ala Val Leu Gln Leu Arg Gly Ala Gly Gly Leu Pro Ser Ala Ala
   625                 630                 635                 640
   Ile Gly Gly Gly Val Ala Val Gly Glu Pro Val Val Ala Met Gly Asn
                   645                 650                 655
   Ser Gly Gly Gln Gly Gly Thr Pro Arg Ala Val Pro Gly Arg Val Val
               660                 665                 670
   Ala Leu Gly Gln Thr Val Gln Ala Ser Asp Ser Leu Thr Gly Ala Glu
           675                 680                 685
   Glu Thr Leu Asn Gly Leu Ile Gln Phe Asp Ala Ala Ile Gln Pro Gly
       690                 695                 700
   Asp Ala Gly Gly Pro Val Val Asn Gly Leu Gly Gln Val Val Gly Met
   705                 710                 715                 720
   Asn Thr Ala Ala Ser
                   725
<210> 11
   <211> 1149
   <212> DNA
   <213> Artificial Sequence
<220>
   <223> Plasmodium falciparum
<400> 11
   atgatgagaa aacttgccat cctcagcgtc agctctttcc tgttcgtgga ggccctcttc 60
   caggagtatc agtgctacgg aagcagcagc aatacaaggg tcctgaacga gctcaactat 120
   gacaacgctg gaacgaacct gtataacgag ctggagatga actactatgg caagcaggag 180
   aactggtata gcctgaagaa gaacagccgg tccctgggcg agaacgacga cggcaacaac 240
   aacaacggcg acaacggcag ggagggcaaa gatgaggaca agagggacgg gaacaacgag 300
   gataacgaga agctgcggaa gcccaagcac aagaaactca agcagcccgc cgacgggaac 360
   ccggacccca atgcaaatcc caacgtcgac ccaaacgcaa accctaacgt ggaccccaac 420
   gccaatccca acgtcgatcc taatgccaat ccaaatgcca accctaacgc aaatcctaat 480
   gcaaacccca acgccaatcc taacgccaac ccaaatgcca acccaaacgc taaccccaac 540
   gctaacccaa atgcaaatcc caatgctaac ccaaacgtgg accctaacgc taaccccaac 600
   gcaaacccta acgccaatcc taacgcaaac cccaatgcaa acccaaacgc aaatcccaac 660
   gctaacccta acgcaaaccc caacgccaac cctaatgcca accccaatgc taaccccaac 720
   gccaatccaa acgcaaatcc aaacgccaac ccaaatgcaa accccaacgc taatcccaac 780
   gccaacccaa acgccaatcc taacaagaac aatcagggca acgggcaggg ccataacatg 840
   ccgaacgacc ctaatcggaa tgtggacgag aacgccaacg ccaacagcgc cgtgaagaac 900
   aacaacaacg aggagccctc cgacaagcac atcaaggaat acctgaacaa gatccagaac 960
   agtctgagca ccgagtggtc cccctgctcc gtgacctgcg gcaacggcat ccaggtgagg 1020
   atcaagcccg gctccgccaa caagcccaag gacgagctgg actacgccaa cgacatcgag 1080
   aagaagatct gcaagatgga gaaatgcagc tctgtgttca acgtcgtgaa ctccgccatc 1140
   ggcctgtga                                                         1149
<210> 12
   <211> 382
   <212> PRT
   <213> Artificial Sequence
<220>
   <223> Plasmodium falciparum
<400> 12
   Met Met Arg Lys Leu Ala Ile Leu Ser Val Ser Ser Phe Leu Phe Val
    1               5                  10                  15
   Glu Ala Leu Phe Gln Glu Tyr Gln Cys Tyr Gly Ser Ser Ser Asn Thr
               20                  25                  30
   Arg Val Leu Asn Glu Leu Asn Tyr Asp Asn Ala Gly Thr Asn Leu Tyr
           35                  40                  45
   Asn Glu Leu Glu Met Asn Tyr Tyr Gly Lys Gln Glu Asn Trp Tyr Ser
       50                  55                  60
   Leu Lys Lys Asn Ser Arg Ser Leu Gly Glu Asn Asp Asp Gly Asn Asn
   65                  70                  75                  80
   Asn Asn Gly Asp Asn Gly Arg Glu Gly Lys Asp Glu Asp Lys Arg Asp
                   85                  90                  95
   Gly Asn Asn Glu Asp Asn Glu Lys Leu Arg Lys Pro Lys His Lys Lys
               100                 105                 110
   Leu Lys Gln Pro Ala Asp Gly Asn Pro Asp Pro Asn Ala Asn Pro Asn
           115                 120                 125
   Val Asp Pro Asn Ala Asn Pro Asn Val Asp Pro Asn Ala Asn Pro Asn
       130                 135                 140
   Val Asp Pro Asn Ala Asn Pro Asn Ala Asn Pro Asn Ala Asn Pro Asn
   145                 150                 155                 160
   Ala Asn Pro Asn Ala Asn Pro Asn Ala Asn Pro Asn Ala Asn Pro Asn
                   165                 170                 175
   Ala Asn Pro Asn Ala Asn Pro Asn Ala Asn Pro Asn Ala Asn Pro Asn
               180                 185                 190
   Val Asp Pro Asn Ala Asn Pro Asn Ala Asn Pro Asn Ala Asn Pro Asn
           195                 200                 205
   Ala Asn Pro Asn Ala Asn Pro Asn Ala Asn Pro Asn Ala Asn Pro Asn
       210                 215                 220
   Ala Asn Pro Asn Ala Asn Pro Asn Ala Asn Pro Asn Ala Asn Pro Asn
   225                 230                 235                 240
   Ala Asn Pro Asn Ala Asn Pro Asn Ala Asn Pro Asn Ala Asn Pro Asn
                   245                 250                 255
   Ala Asn Pro Asn Ala Asn Pro Asn Ala Asn Pro Asn Lys Asn Asn Gln
               260                 265                 270
   Gly Asn Gly Gln Gly His Asn Met Pro Asn Asp Pro Asn Arg Asn Val
           275                 280                 285
   Asp Glu Asn Ala Asn Ala Asn Ser Ala Val Lys Asn Asn Asn Asn Glu
       290                 295                 300
   Glu Pro Ser Asp Lys His Ile Lys Glu Tyr Leu Asn Lys Ile Gln Asn
   305                 310                 315                 320
   Ser Leu Ser Thr Glu Trp Ser Pro Cys Ser Val Thr Cys Gly Asn Gly
                   325                 330                 335
   Ile Gln Val Arg Ile Lys Pro Gly Ser Ala Asn Lys Pro Lys Asp Glu
               340                 345                 350
   Leu Asp Tyr Ala Asn Asp Ile Glu Lys Lys Ile Cys Lys Met Glu Lys
           355                 360                 365
   Cys Ser Ser Val Phe Asn Val Val Asn Ser Ala Ile Gly Leu
       370                 375                 380
<210> 13
   <211> 1275
   <212> DNA
   <213> Artificial Sequence
<220>
   <223> Plasmodium falciparum
<400> 13
   atgatggctc ccgatcctaa tgcaaatcca aatgcaaacc caaacgcaaa ccccaatgca 60
   aatcctaatg caaaccccaa tgcaaatcct aatgcaaatc ctaatgccaa tccaaatgca 120
   aatccaaatg caaacccaaa cgcaaacccc aatgcaaatc ctaatgccaa tccaaatgca 180
   aatccaaatg caaacccaaa tgcaaaccca aatgcaaacc ccaatgcaaa tcctaataaa 240
   aacaatcaag gtaatggaca aggtcacaat atgccaaatg acccaaaccg aaatgtagat 300
   gaaaatgcta atgccaacag tgctgtaaaa aataataata acgaagaacc aagtgataag 360
   cacataaaag aatatttaaa caaaatacaa aattctcttt caactgaatg gtccccatgt 420
   agtgtaactt gtggaaatgg tattcaagtt agaataaagc ctggctctgc taataaacct 480
   aaagacgaat tagattatgc aaatgatatt gaaaaaaaaa tttgtaaaat ggaaaaatgt 540
   tccagtgtgt ttaatgtcgt aaatagttca ataggattag ggcctgtgac gaacatggag 600
   aacatcacat caggattcct aggacccctg ctcgtgttac aggcggggtt tttcttgttg 660
   acaagaatcc tcacaatacc gcagagtcta gactcgtggt ggacttctct caattttcta 720
   gggggatcac ccgtgtgtct tggccaaaat tcgcagtccc caacctccaa tcactcacca 780
   acctcctgtc ctccaatttg tcctggttat cgctggatgt gtctgcggcg ttttatcata 840
   ttcctcttca tcctgctgct atgcctcatc ttcttattgg ttcttctgga ttatcaaggt 900
   atgttgcccg tttgtcctct aattccagga tcaacaacaa ccaatacggg accatgcaaa 960
   acctgcacga ctcctgctca aggcaactct atgtttccct catgttgctg tacaaaacct 1020
   acggatggaa attgcacctg tattcccatc ccatcgtcct gggctttcgc aaaataccta 1080
   tgggagtggg cctcagtccg tttctcttgg ctcagtttac tagtgccatt tgttcagtgg 1140
   ttcgtagggc tttcccccac tgtttggctt tcagctatat ggatgatgtg gtattggggg 1200
   ccaagtctgt acagcatcgt gagtcccttt ataccgctgt taccaatttt cttttgtctc 1260
   tgggtataca tttaa                                                  1275
<210> 14
   <211> 424
   <212> PRT
   <213> Artificial Sequence
<220>
   <223> Plasmodium falciparum
<400> 14
   Met Met Ala Pro Asp Pro Asn Ala Asn Pro Asn Ala Asn Pro Asn Ala
    1               5                  10                  15
   Asn Pro Asn Ala Asn Pro Asn Ala Asn Pro Asn Ala Asn Pro Asn Ala
               20                  25                  30
   Asn Pro Asn Ala Asn Pro Asn Ala Asn Pro Asn Ala Asn Pro Asn Ala
           35                  40                  45
   Asn Pro Asn Ala Asn Pro Asn Ala Asn Pro Asn Ala Asn Pro Asn Ala
       50                  55                  60
   Asn Pro Asn Ala Asn Pro Asn Ala Asn Pro Asn Ala Asn Pro Asn Lys
   65                  70                  75                  80
   Asn Asn Gln Gly Asn Gly Gln Gly His Asn Met Pro Asn Asp Pro Asn
                   85                  90                  95
   Arg Asn Val Asp Glu Asn Ala Asn Ala Asn Ser Ala Val Lys Asn Asn
               100                 105                 110
   Asn Asn Glu Glu Pro Ser Asp Lys His Ile Lys Glu Tyr Leu Asn Lys
           115                 120                 125
   Ile Gln Asn Ser Leu Ser Thr Glu Trp Ser Pro Cys Ser Val Thr Cys
       130                 135                 140
   Gly Asn Gly Ile Gln Val Arg Ile Lys Pro Gly Ser Ala Asn Lys Pro
   145                 150                 155                 160
   Lys Asp Glu Leu Asp Tyr Ala Asn Asp Ile Glu Lys Lys Ile Cys Lys
                   165                 170                 175
   Met Glu Lys Cys Ser Ser Val Phe Asn Val Val Asn Ser Ser Ile Gly
               180                 185                 190
   Leu Gly Pro Val Thr Asn Met Glu Asn Ile Thr Ser Gly Phe Leu Gly
           195                 200                 205
   Pro Leu Leu Val Leu Gln Ala Gly Phe Phe Leu Leu Thr Arg Ile Leu
       210                 215                 220
   Thr Ile Pro Gln Ser Leu Asp Ser Trp Trp Thr Ser Leu Asn Phe Leu
   225                 230                 235                 240
   Gly Gly Ser Pro Val Cys Leu Gly Gln Asn Ser Gln Ser Pro Thr Ser
                   245                 250                 255
   Asn His Ser Pro Thr Ser Cys Pro Pro Ile Cys Pro Gly Tyr Arg Trp
               260                 265                 270
   Met Cys Leu Arg Arg Phe Ile Ile Phe Leu Phe Ile Leu Leu Leu Cys
           275                 280                 285
   Leu Ile Phe Leu Leu Val Leu Leu Asp Tyr Gln Gly Met Leu Pro Val
       290                 295                 300
   Cys Pro Leu Ile Pro Gly Ser Thr Thr Thr Asn Thr Gly Pro Cys Lys
   305                 310                 315                 320
   Thr Cys Thr Thr Pro Ala Gln Gly Asn Ser Met Phe Pro Ser Cys Cys
                   325                 330                 335
   Cys Thr Lys Pro Thr Asp Gly Asn Cys Thr Cys Ile Pro Ile Pro Ser
               340                 345                 350
   Ser Trp Ala Phe Ala Lys Tyr Leu Trp Glu Trp Ala Ser Val Arg Phe
           355                 360                 365
   Ser Trp Leu Ser Leu Leu Val Pro Phe Val Gln Trp Phe Val Gly Leu
       370                 375                 380
   Ser Pro Thr Val Trp Leu Ser Ala Ile Trp Met Met Trp Tyr Trp Gly
   385                 390                 395                 400
   Pro Ser Leu Tyr Ser Ile Val Ser Pro Phe Ile Pro Leu Leu Pro Ile
                   405                 410                 415
   Phe Phe Cys Leu Trp Val Tyr Ile
               420
<210> 15
   <211> 3411
   <212> DNA
   <213> Artificial Sequence
<220>
   <223> HIV
<400> 15
   atggtcattg ttcagaacat acagggccaa atggtccacc aggcaattag tccgcgaact 60
   cttaatgcat gggtgaaggt cgtggaggaa aaggcattct ccccggaggt cattccgatg 120
   ttttctgcgc tatctgaggg cgcaacgccg caagacctta ataccatgct taacacggta 180
   ggcgggcacc aagccgctat gcaaatgcta aaagagacta taaacgaaga ggccgccgaa 240
   tgggatcgag tgcacccggt gcacgccggc ccaattgcac caggccagat gcgcgagccg 300
   cgcgggtctg atattgcagg aactacgtct acccttcagg agcagattgg gtggatgact 360
   aacaatccac caatcccggt cggagagatc tataagaggt ggatcatact gggactaaac 420
   aagatagtcc gcatgtattc tccgacttct atactggata tacgccaagg cccaaaggag 480
   ccgttcaggg actatgtcga ccgattctat aagacccttc gcgcagagca ggcatcccag 540
   gaggtcaaaa attggatgac agaaactctt ttggtgcaga atgcgaatcc ggattgtaaa 600
   acaattttaa aggctctagg accggccgca acgctagaag agatgatgac ggcttgtcag 660
   ggagtcggtg gaccggggca taaagcccgc gtcttacaca tgggcccgat atctccgata 720
   gaaacagttt cggtcaagct taaaccaggg atggatggtc caaaggtcaa gcagtggccg 780
   ctaacggaag agaagattaa ggcgctcgta gagatttgta ctgaaatgga gaaggaaggc 840
   aagataagca agatcgggcc agagaacccg tacaatacac cggtatttgc aataaagaaa 900
   aaggattcaa caaaatggcg aaagcttgta gattttaggg aactaaacaa gcgaacccaa 960
   gacttttggg aagtccaact agggatccca catccagccg gtctaaagaa gaagaaatcg 1020
   gtcacagtcc tggatgtagg agacgcatat tttagtgtac cgcttgatga ggacttccga 1080
   aagtatactg cgtttactat accgagcata aacaatgaaa cgccaggcat tcgctatcag 1140
   tacaacgtgc tcccgcaggg ctggaagggg tctccggcga tatttcagag ctgtatgaca 1200
   aaaatacttg aaccattccg aaagcagaat ccggatattg taatttacca atacatggac 1260
   gatctctatg tgggctcgga tctagaaatt gggcagcatc gcactaagat tgaggaactg 1320
   aggcaacatc tgcttcgatg gggcctcact actcccgaca agaagcacca gaaggagccg 1380
   ccgttcctaa agatgggcta cgagcttcat ccggacaagt ggacagtaca gccgatagtg 1440
   ctgcccgaaa aggattcttg gaccgtaaat gatattcaga aactagtcgg caagcttaac 1500
   tgggcctctc agatttaccc aggcattaag gtccgacagc tttgcaagct actgagggga 1560
   actaaggctc taacagaggt catcccatta acggaggaag cagagcttga gctggcagag 1620
   aatcgcgaaa ttcttaagga gccggtgcac ggggtatact acgacccctc caaggacctt 1680
   atagccgaga tccagaagca ggggcagggc caatggacgt accagatata tcaagaaccg 1740
   tttaagaatc tgaagactgg gaagtacgcg cgcatgcgag gggctcatac taatgatgta 1800
   aagcaactta cggaagcagt acaaaagatt actactgagt ctattgtgat atggggcaag 1860
   accccaaagt tcaagctgcc catacagaag gaaacatggg aaacatggtg gactgaatat 1920
   tggcaagcta cctggattcc agaatgggaa tttgtcaaca cgccgccact tgttaagctt 1980
   tggtaccagc ttgaaaagga gccgatagta ggggcagaga ccttctatgt cgatggcgcc 2040
   gcgaatcgcg aaacgaagct aggcaaggcg ggatacgtga ctaatagggg ccgccaaaag 2100
   gtcgtaaccc ttacggatac caccaatcag aagactgaac tacaagcgat ttaccttgca 2160
   cttcaggata gtggcctaga ggtcaacata gtcacggact ctcaatatgc gcttggcatt 2220
   attcaagcgc agccagatca aagcgaaagc gagcttgtaa accaaataat agaacagctt 2280
   ataaagaaag agaaggtata tctggcctgg gtccccgctc acaagggaat tggcggcaat 2340
   gagcaagtgg acaagctagt cagcgctggg attcgcaagg ttcttgcgat ggggggtaag 2400
   tggtctaagt ctagcgtagt cggctggccg acagtccgcg agcgcatgcg acgcgccgaa 2460
   ccagccgcag atggcgtggg ggcagcgtct agggatctgg agaagcacgg ggctataact 2520
   tccagtaaca cggcggcgac gaacgccgca tgcgcatggt tagaagccca agaagaggaa 2580
   gaagtagggt ttccggtaac tccccaggtg ccgttaaggc cgatgaccta taaggcagcg 2640
   gtggatcttt ctcacttcct taaggagaaa ggggggctgg agggcttaat tcacagccag 2700
   aggcgacagg atattcttga tctgtggatt taccataccc aggggtactt tccggactgg 2760
   cagaattaca ccccgggccc aggcgtgcgc tatcccctga ctttcgggtg gtgctacaaa 2820
   ctagtcccag tggaacccga caaggtcgaa gaggctaata agggcgagaa cacttctctt 2880
   cttcacccgg taagcctgca cgggatggat gacccagaac gagaggttct agaatggagg 2940
   ttcgactctc gacttgcgtt ccatcacgta gcacgcgagc tgcatccaga atatttcaag 3000
   aactgccgcc caatgggcgc cagggccagt gtacttagtg gcggagaact agatcgatgg 3060
   gaaaagatac gcctacgccc ggggggcaag aagaagtaca agcttaagca cattgtgtgg 3120
   gcctctcgcg aacttgagcg attcgcagtg aatccaggcc tgcttgagac gagtgaaggc 3180
   tgtaggcaaa ttctggggca gctacagccg agcctacaga ctggcagcga ggagcttcgt 3240
   agtctttata ataccgtcgc gactctctac tgcgttcatc aacgaattga aataaaggat 3300
   actaaagagg cccttgataa aattgaggag gaacagaata agtcgaaaaa gaaggcccag 3360
   caggccgccg ccgacaccgg gcacagcaac caggtgtccc aaaactacta a 3411
<210> 16
   <211> 1136
   <212> PRT
   <213> Artificial Sequence
<220>
   <223> HIV
<400> 16
   Met Val Ile Val Gln Asn Ile Gln Gly Gln Met Val His Gln Ala Ile
    1               5                   10                  15
   Ser Pro Arg Thr Leu Asn Ala Trp Val Lys Val Val Glu Glu Lys Ala
               20                  25                  30
   Phe Ser Pro Glu Val Ile Pro Met Phe Ser Ala Leu Ser Glu Gly Ala
           35                  40                  45
   Thr Pro Gln Asp Leu Asn Thr Met Leu Asn Thr Val Gly Gly His Gln
       50                  55                  60
   Ala Ala Met Gln Met Leu Lys Glu Thr Ile Asn Glu Glu Ala Ala Glu
   65                  70                  75                  80
   Trp Asp Arg Val His Pro Val His Ala Gly Pro Ile Ala Pro Gly Gln
                   85                  90                  95
   Met Arg Glu Pro Arg Gly Ser Asp Ile Ala Gly Thr Thr Ser Thr Leu
               100                 105                 110
   Gln Glu Gln Ile Gly Trp Met Thr Asn Asn Pro Pro Ile Pro Val Gly
           115                 120                 125
   Glu Ile Tyr Lys Arg Trp Ile Ile Leu Gly Leu Asn Lys Ile Val Arg
       130                 135                 140
   Met Tyr Ser Pro Thr Ser Ile Leu Asp Ile Arg Gln Gly Pro Lys Glu
   145                 150                 155                 160
   Pro Phe Arg Asp Tyr Val Asp Arg Phe Tyr Lys Thr Leu Arg Ala Glu
                   165                 170                 175
   Gln Ala Ser Gln Glu Val Lys Asn Trp Met Thr Glu Thr Leu Leu Val
               180                 185                 190
   Gln Asn Ala Asn Pro Asp Cys Lys Thr Ile Leu Lys Ala Leu Gly Pro
           195                 200                 205
   Ala Ala Thr Leu Glu Glu Met Met Thr Ala Cys Gln Gly Val Gly Gly
       210                 215                 220
   Pro Gly His Lys Ala Arg Val Leu His Met Gly Pro Ile Ser Pro Ile
   225                 230                 235                 240
   Glu Thr Val Ser Val Lys Leu Lys Pro Gly Met Asp Gly Pro Lys Val
                   245                 250                 255
   Lys Gln Trp Pro Leu Thr Glu Glu Lys Ile Lys Ala Leu Val Glu Ile
               260                 265                 270
   Cys Thr Glu Met Glu Lys Glu Gly Lys Ile Ser Lys Ile Gly Pro Glu
           275                 280                 285
   Asn Pro Tyr Asn Thr Pro Val Phe Ala Ile Lys Lys Lys Asp Ser Thr
       290                 295                 300
   Lys Trp Arg Lys Leu Val Asp Phe Arg Glu Leu Asn Lys Arg Thr Gln
   305                 310                 315                 320
   Asp Phe Trp Glu Val Gln Leu Gly Ile Pro His Pro Ala Gly Leu Lys
                   325                 330                 335
   Lys Lys Lys Ser Val Thr Val Leu Asp Val Gly Asp Ala Tyr Phe Ser
               340                 345                 350
   Val Pro Leu Asp Glu Asp Phe Arg Lys Tyr Thr Ala Phe Thr Ile Pro
           355                 360                 365
   Ser Ile Asn Asn Glu Thr Pro Gly Ile Arg Tyr Gln Tyr Asn Val Leu
       370                 375                 380
   Pro Gln Gly Trp Lys Gly Ser Pro Ala Ile Phe Gln Ser Cys Met Thr
   385                 390                 395                 400
   Lys Ile Leu Glu Pro Phe Arg Lys Gln Asn Pro Asp Ile Val Ile Tyr
                   405                 410                 415
   Gln Tyr Met Asp Asp Leu Tyr Val Gly Ser Asp Leu Glu Ile Gly Gln
               420                 425                 430
   His Arg Thr Lys Ile Glu Glu Leu Arg Gln His Leu Leu Arg Trp Gly
           435                 440                 445
   Leu Thr Thr Pro Asp Lys Lys His Gln Lys Glu Pro Pro Phe Leu Lys
       450                 455                 460
   Met Gly Tyr Glu Leu His Pro Asp Lys Trp Thr Val Gln Pro Ile Val
   465                 470                 475                 480
   Leu Pro Glu Lys Asp Ser Trp Thr Val Asn Asp Ile Gln Lys Leu Val
                   485                 490                 495
   Gly Lys Leu Asn Trp Ala Ser Gln Ile Tyr Pro Gly Ile Lys Val Arg
               500                 505                 510
   Gln Leu Cys Lys Leu Leu Arg Gly Thr Lys Ala Leu Thr Glu Val Ile
           515                 520                 525
   Pro Leu Thr Glu Glu Ala Glu Leu Glu Leu Ala Glu Asn Arg Glu Ile
       530                 535                 540
   Leu Lys Glu Pro Val His Gly Val Tyr Tyr Asp Pro Ser Lys Asp Leu
   545                 550                 555                 560
   Ile Ala Glu Ile Gln Lys Gln Gly Gln Gly Gln Trp Thr Tyr Gln Ile
                   565                 570                 575
   Tyr Gln Glu Pro Phe Lys Asn Leu Lys Thr Gly Lys Tyr Ala Arg Met
               580                 585                 590
   Arg Gly Ala His Thr Asn Asp Val Lys Gln Leu Thr Glu Ala Val Gln
           595                 600                 605
   Lys Ile Thr Thr Glu Ser Ile Val Ile Trp Gly Lys Thr Pro Lys Phe
       610                 615                 620
   Lys Leu Pro Ile Gln Lys Glu Thr Trp Glu Thr Trp Trp Thr Glu Tyr
   625                 630                 635                 640
   Trp Gln Ala Thr Trp Ile Pro Glu Trp Glu Phe Val Asn Thr Pro Pro
                   645                 650                 655
   Leu Val Lys Leu Trp Tyr Gln Leu Glu Lys Glu Pro Ile Val Gly Ala
               660                 665                 670
   Glu Thr Phe Tyr Val Asp Gly Ala Ala Asn Arg Glu Thr Lys Leu Gly
           675                 680                 685
   Lys Ala Gly Tyr Val Thr Asn Arg Gly Arg Gln Lys Val Val Thr Leu
       690                 695                 700
   Thr Asp Thr Thr Asn Gln Lys Thr Glu Leu Gln Ala Ile Tyr Leu Ala
   705                 710                 715                 720
   Leu Gln Asp Ser Gly Leu Glu Val Asn Ile Val Thr Asp Ser Gln Tyr
                   725                 730                 735
   Ala Leu Gly Ile Ile Gln Ala Gln Pro Asp Gln Ser Glu Ser Glu Leu
               740                 745                 750
   Val Asn Gln Ile Ile Glu Gln Leu Ile Lys Lys Glu Lys Val Tyr Leu
           755                 760                 765
   Ala Trp Val Pro Ala His Lys Gly Ile Gly Gly Asn Glu Gln Val Asp
       770                 775                 780
   Lys Leu Val Ser Ala Gly Ile Arg Lys Val Leu Ala Met Gly Gly Lys
   785                 790                 795                 800
   Trp Ser Lys Ser Ser Val Val Gly Trp Pro Thr Val Arg Glu Arg Met
                   805                 810                 815
   Arg Arg Ala Glu Pro Ala Ala Asp Gly Val Gly Ala Ala Ser Arg Asp
               820                 825                 830
   Leu Glu Lys His Gly Ala Ile Thr Ser Ser Asn Thr Ala Ala Thr Asn
           835                 840                 845
   Ala Ala Cys Ala Trp Leu Glu Ala Gln Glu Glu Glu Glu Val Gly Phe
       850                 855                 860
   Pro Val Thr Pro Gln Val Pro Leu Arg Pro Met Thr Tyr Lys Ala Ala
   865                 870                 875                 880
   Val Asp Leu Ser His Phe Leu Lys Glu Lys Gly Gly Leu Glu Gly Leu
                   885                 890                 895
   Ile His Ser Gln Arg Arg Gln Asp Ile Leu Asp Leu Trp Ile Tyr His
               900                 905                 910
   Thr Gln Gly Tyr Phe Pro Asp Trp Gln Asn Tyr Thr Pro Gly Pro Gly
           915                 920                 925
   Val Arg Tyr Pro Leu Thr Phe Gly Trp Cys Tyr Lys Leu Val Pro Val
       930                 935                 940
   Glu Pro Asp Lys Val Glu Glu Ala Asn Lys Gly Glu Asn Thr Ser Leu
   945                 950                 955                 960
   Leu His Pro Val Ser Leu His Gly Met Asp Asp Pro Glu Arg Glu Val
                   965                 970                 975
   Leu Glu Trp Arg Phe Asp Ser Arg Leu Ala Phe His His Val Ala Arg
               980                 985                 990
   Glu Leu His Pro Glu Tyr Phe Lys Asn Cys Arg Pro Met Gly Ala Arg
           995                 1000                1005
   Ala Ser Val Leu Ser Gly Gly Glu Leu Asp Arg Trp Glu Lys Ile Arg
       1010                1015                1020
   Leu Arg Pro Gly Gly Lys Lys Lys Tyr Lys Leu Lys His Ile Val Trp
   1025                1030                1035                1040
   Ala Ser Arg Glu Leu Glu Arg Phe Ala Val Asn Pro Gly Leu Leu Glu
                   1045                1050                1055
   Thr Ser Glu Gly Cys Arg Gln Ile Leu Gly Gln Leu Gln Pro Ser Leu
               1060                1065                1070
   Gln Thr Gly Ser Glu Glu Leu Arg Ser Leu Tyr Asn Thr Val Ala Thr
           1075                1080                1085
   Leu Tyr Cys Val His Gln Arg Ile Glu Ile Lys Asp Thr Lys Glu Ala
       1090                1095                1100
   Leu Asp Lys Ile Glu Glu Glu Gln Asn Lys Ser Lys Lys Lys Ala Gln
   1105                1110                1115                1120
   Gln Ala Ala Ala Asp Thr Gly His Ser Asn Gln Val Ser Gln Asn Tyr
                   1125                1130                1135

## Claims

1. One or more first immunogenic polypeptides for use in a method of raising an immune response against *Mycobacterium spp.* wherein the one or more first immunogenic polypeptides are administered concomitantly with (i) one or more adenoviral vectors comprising one or more heterologous polynucleotides encoding one or more second immunogenic polypeptides and (ii) an adjuvant which comprises QS21 and 3D-MPL, wherein the one or more first and second polypeptides comprises residues 4-725 of SEQ ID NO: 10, and wherein the term "concomitantly" means wherein the one or more immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more immunogenic polypeptides, the one or more adenoviral vectors and the adjuvant are administered sequentially or simultaneously .

2. One or more adenoviral vectors comprising one or more heterologous polynucleotides encoding one or more second immunogenic polypeptides derived from *Mycobacterium spp.* for use in a method of raising an immune response against said *Mycobacterium spp.* wherein the one or more adenoviral vectors are administered concomitantly with (i) one or more first immunogenic polypeptides derived from said *Mycobacterium spp.* and (ii) an adjuvant which comprises QS21 and 3D-MPL, wherein the one or more first and second polypeptides comprises residues 4-725 of SEQ ID NO: 10, and wherein the term "concomitantly" means wherein the one or more immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more immunogenic polypeptides, the one or more adenoviral vectors and the adjuvant are administered sequentially or simultaneously.

3. The one or more first immunogenic polypeptides for use or the one or more adenoviral vectors for use according to either claim 1 or 2, wherein the one or more first immunogenic polypeptides are co-formulated with the adjuvant.

4. The one or more first immunogenic polypeptides for use or the one or more adenoviral vectors for use according to any one of claims 1 to 3 wherein the method of raising an immune response against *Mycobacterium spp.* consists of (a) concomitantly administering (i) said one or more first immunogenic polypeptides; (ii) said one or more adenoviral vectors; and (iii) an adjuvant; and (b) optionally repeating the steps of (a) on a second occasion, and wherein the term "concomitantly" means wherein the one or more immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more immunogenic polypeptides, the one or more adenoviral vectors and the adjuvant are administered sequentially or simultaneously.

5. The one or more first immunogenic polypeptides for use or the one or more adenoviral vectors for use according to any one of claims 1 to 4 wherein the method of raising an immune response against *Mycobacterium spp.* does not involve administering any priming dose of immunogenic polypeptide or polynucleotide encoding immunogenic polypeptide.

6. The one or more first immunogenic polypeptides for use or the one or more adenoviral vectors for use according to any one of claims 1 to 5, wherein the one or more first immunogenic polypeptides, one or more adenoviral vectors and adjuvant are co-formulated.

7. The one or more first immunogenic polypeptides for use or the one or more adenoviral vectors for use according to any one of claims 1 to 5 wherein the one or more first immunogenic polypeptides, one or more adenoviral vectors and adjuvant are not co-formulated and are administered by different routes of administration.

8. A vaccine composition comprising (i) one or more first immunogenic polypeptides derived from *Mycobacterium spp.;* (ii) one or more adenoviral vectors comprising one or more heterologous polynucleotides encoding one or more second immunogenic polypeptides derived from Mycobacterium spp.; and (iii) an adjuvant which comprises QS21 and 3D-MPL, wherein the one or more first and second polypeptides comprises residues 4-725 of SEQ ID NO: 10.

9. A kit comprising (i) one or more first immunogenic polypeptides derived from *Mycobacterium spp*.; (ii) one or more adenoviral vectors comprising one or more heterologous polynucleotides encoding one or more second immunogenic polypeptides derived from said *Mycobacterium spp*.; and (iii) an adjuvant which comprises QS21 and 3D-MPL, wherein the one more first and second polypeptides comprises residues 4-725 of SEQ ID NO: 10, and wherein the one or more immunogenic polypeptides, the one or more adenoviral vectors and the adjuvant are for administration concomitantly, wherein the term "concomitantly" means wherein the one or more immunogenic polypeptides, the one or more adenoviral vectors and the adjuvant are administered within a period of no more than 12 hours eg within a period of no more than 1 hour, typically on one occasion e.g. in the course of the same visit to the health professional, for example the one or more immunogenic polypeptides, the one or more adenoviral vectors and the adjuvant are administered sequentially or simultaneously.

10. The one or more first immunogenic polypeptides for use, the one or more adenoviral vectors for use, the vaccine composition or the kit according to any one of claims 1 to 9 wherein the *Mycobacterium spp.* is *Mycobacterium tuberculosis.*

11. The one or more first immunogenic polypeptides for use, the one or more adenoviral vectors for use, the vaccine composition or the kit according to any one of claims 1 to 10 wherein one or more of the adenoviral vectors is derived from a non-human primate adenovirus.

12. The one or more first immunogenic polypeptides for use, the one or more adenoviral vectors for use, the vaccine composition or the kit according to any one of claims 1 to 11 wherein the adjuvant contains liposomes.

## Patentansprüche

1. Ein oder mehrere erste(s) immunogene(s) Polypeptid(e) zur Verwendung in einem Verfahren zum Hervorrufen einer Immunantwort gegen *Mycobacterium spp.,* wobei das/die eine oder mehreren erste(n) immunogene(n) Polypeptid(e) gleichzeitig zusammen mit (i) einem oder mehreren adenoviralen Vektor(en), welche(r) ein oder mehrere zweite(s) immunogene(s) Polypeptid(e) codiert/codieren, und (ii) einem Adjuvans, welches QS21 und 3D-MPL umfasst, verabreicht wird/werden, wobei die einen oder mehreren ersten und zweiten Polypeptide Reste 4-725 von SEQ ID NO: 10 umfassen, und wobei der Terminus "gleichzeitig zusammen" bedeutet wobei das/die eine oder mehreren immunogene Polypeptid(e), der/die eine oder mehrere adenovirale Vektor(en) und das Adjuvans innerhalb eines Zeitraums von nicht mehr als 12 Stunden verabreicht werden, z.B. innerhalb eines Zeitraums von nicht mehr als 1 Stunde, typischerweise zu einer Gelegenheit, z.B. während des gleichen Besuchs bei einer medizinischen Fachkraft, zum Beispiel werden die ein oder mehreren immunogenen Polypeptide, der eine oder mehrere adenovirale Vektor und das Adjuvans sequenziell oder gleichzeitig verabreicht.

2. Ein oder mehrere adenoviral(er) Vektor(en), umfassend ein oder mehrere heterologe Polynukleotide, welche(r) ein oder mehrere zweite(s) immunogene(s) Polypeptid(e), abgeleitet von *Mycobacterium spp.,* codiert/codieren, zur Verwendung in einem Verfahren zum Hervorrufen einer Immunantwort gegen das *Mycobacterium spp.,* wobei der/die eine oder mehreren adenovirale(n) Vektor(en) gleichzeitig zusammen mit (i) einem oder mehreren ersten immunogenen Polypeptid(en), welche(s) von *Mycobacterium spp.* abgeleitet ist/sind, und (ii) einem Adjuvans, welches QS21 und 3D-MPL umfasst, verabreicht wird/werden, wobei die einen oder mehreren ersten und zweiten Polypeptide Reste 4-725 von SEQ ID NO: 10 umfassen, und wobei der Terminus "gleichzeitig zusammen" bedeutet wobei das/die eine oder mehreren immunogene Polypeptid(e), der/die eine oder mehrere adenovirale Vektor(en) und das Adjuvans innerhalb eines Zeitraums von nicht mehr als 12 Stunden verabreicht werden, z.B. innerhalb eines Zeitraums von nicht mehr als 1 Stunde, typischerweise zu einer Gelegenheit, z.B. während des gleichen Besuchs bei einer medizinischen Fachkraft, zum Beispiel werden die ein oder mehreren immunogenen Polypeptide, der eine oder mehrere adenovirale Vektor und das Adjuvans sequenziell oder gleichzeitig verabreicht.

3. Ein oder mehrere erste(s) immunogene(s) Polypeptid(e) zur Verwendung oder ein oder mehrere adenoviral(er) Vektor(en) zur Verwendung gemäß Anspruch 1 oder 2, wobei das/die eine oder mehreren ersten immunogene(n) Polypeptid(e) mit dem Adjuvans co-formuliert sind.

4. Ein oder mehrere erste(s) immunogene(s) Polypeptid(e) zur Verwendung oder ein oder mehrere adenoviral(er) Vektor(en) zur Verwendung gemäß irgendeinem der Ansprüche 1 bis 3, wobei das Verfahren zum Auslösen einer Immunantwort gegen *Mycobacterium spp.* aus (a) gleichzeitig zusammen Verabreichen von (i) des/der einen oder mehreren ersten Polypeptids/Polypeptide; (ii) des/der einen oder mehreren adenoviralen Vektors/Vektoren; und (iii) einem Adjuvans; und (b) optional dem Wiederholen der Schritte von (a) zu einer zweiten Gelegenheit besteht, und wobei der Terminus "gleichzeitig zusammen" bedeutet wobei das/die eine oder mehreren immunogene Polypeptid(e), der/die eine oder mehrere adenovirale Vektor(en) und das Adjuvans innerhalb eines Zeitraums von nicht mehr als 12 Stunden verabreicht werden, z.B. innerhalb eines Zeitraums von nicht mehr als 1 Stunde, typischerweise zu einer Gelegenheit, z.B. während des gleichen Besuchs bei einer medizinischen Fachkraft, zum Beispiel werden die ein oder mehreren immunogenen Polypeptide, der eine oder mehrere adenovirale Vektor und das Adjuvans sequenziell oder gleichzeitig verabreicht.

5. Ein oder mehrere erste(s) immunogene(s) Polypeptid(e) zur Verwendung oder ein oder mehrere adenoviral(er) Vektor(en) zur Verwendung gemäß irgendeinem der Ansprüche 1 bis 4, wobei das Verfahren zum Auslösen einer Immunantwort gegen *Mycobacterium spp.* kein Verabreichen irgendeiner Priming-Dosis eines immunogenen Polypeptids oder eines Polynukleotides, welches ein immunogenes Polypeptid codiert, umfasst.

6. Ein oder mehrere erste(s) immunogene(s) Polypeptid(e) zur Verwendung oder ein oder mehrere adenoviral(er) Vektor(en) zur Verwendung gemäß irgendeinem der Ansprüche 1 bis 5, wobei das/die eine oder mehreren ersten immunogene(n) Polypeptid(e), der/die ein oder mehrere adenoviral(en) Vektor(en), und das Adjuvans co-formuliert sind.

7. Ein oder mehrere erste(s) immunogene(s) Polypeptid(e) zur Verwendung oder ein oder mehrere adenoviral(er) Vektor(en) zur Verwendung gemäß irgendeinem der Ansprüche 1 bis 5, wobei das/die eine oder mehreren ersten immunogene(n) Polypeptid(e), der/die ein oder mehrere adenoviral(en) Vektor(en), und das Adjuvans nicht co-formuliert sind und mittels unterschiedlicher Verabreichungsformen verabreicht werden.

8. Impfstoff-Zusammensetzung, umfassend (i) ein oder mehrere erste(s) immunogene(s) Polypeptid(e), welche(s) von *Mycobacterium spp.* abgeleitet ist/sind; (ii) einen oder mehrere adenoviral(e) Vektor(en), welche(r) ein oder mehrere heterologe(s) Polynukleotid(e) umfasst/umfassen, welche(s) ein oder mehrere zweite(s) immunogene(s) Polypeptid(e) codiert, welche(s) von *Mycobacterium spp.* abgeleitet ist/sind; und (iii) ein Adjuvans, welches QS21 und 3D-MPL umfasst, wobei die einen oder mehreren ersten und zweiten Polypeptide Reste 4-725 von SEQ ID NO: 10 umfassen.

9. Kit, umfassend (i) ein oder mehrere erste(s) immunogene(s) Polypeptid(e), welche(s) von *Mycobacterium spp.* abgeleitet ist/sind; (ii) einen oder mehrere adenoviral(e) Vektor(en), welche(r) ein oder mehrere heterologe Polynukleotide umfasst/umfassen, welche(s) ein oder mehrere zweite(s) immunogene(s) Polypeptid(e) codiert, welche(s) von *Mycobacterium spp.* abgeleitet ist/sind; und (iii) ein Adjuvans, welches QS21 und 3D-MPL umfasst, wobei die einen oder mehreren ersten und zweiten Polypeptide Reste 4-725 von SEQ ID NO: 10 umfassen, und wobei das/die ein oder mehreren immunogene Polypeptid(e), der/die eine oder mehreren adenoviralen Vektoren, und das Adjuvans für gleichzeitige Verabreichung sind, wobei der Terminus "gleichzeitig zusammen" bedeutet wobei das/die eine oder mehreren immunogene Polypeptid(e), der/die eine oder mehrere adenovirale Vektor(en) und das Adjuvans innerhalb eines Zeitraums von nicht mehr als 12 Stunden verabreicht werden, z.B. innerhalb eines Zeitraums von nicht mehr als 1 Stunde, typischerweise zu einer Gelegenheit, z.B. während des gleichen Besuchs bei einer medizinischen Fachkraft, zum Beispiel werden die ein oder mehreren immunogenen Polypeptide, der eine oder mehrere adenovirale Vektor und das Adjuvans sequenziell oder gleichzeitig verabreicht.

10. Ein oder mehrere erste(s) immunogene(s) Polypeptid(e) zur Verwendung oder ein oder mehrere adenoviral(er) Vektor(en) zur Verwendung, die Impfstoff-Zusammensetzung oder das Kit gemäß irgendeinem der Ansprüche 1 bis 9, wobei das *Mycobacterium spp. Mycobacterium tuberculosis* ist.

11. Ein oder mehrere erste(s) immunogene(s) Polypeptid(e) zur Verwendung oder ein oder mehrere adenoviral(er) Vektor(en) zur Verwendung, die Impfstoff-Zusammensetzung oder das Kit gemäß irgendeinem der Ansprüche 1 bis 10, wobei ein oder mehrere adenovirale(r) Vektor(en) von nicht-humanem-Primaten-Adenovirus abgeleitet ist.

12. Ein oder mehrere erste(s) immunogene(s) Polypeptid(e) zur Verwendung oder ein oder mehrere adenoviral(er) Vektor(en) zur Verwendung, die Impfstoff-Zusammensetzung oder das Kit gemäß irgendeinem der Ansprüche 1 bis 11, wobei das Adjuvans Liposomen umfasst.

## Revendications

1. Un ou plusieurs premiers polypeptides immunogènes pour une utilisation dans un procédé d'induction d'une réponse immunitaire dirigée contre *Mycobacterium spp.,* dans lesquels les un ou plusieurs premiers polypeptides immunogènes sont administrés de façon concomitante avec (i) un ou plusieurs vecteurs adénoviraux comprenant un ou plusieurs polynucléotides hétérologues codant pour un ou plusieurs seconds polypeptides immunogènes et (ii) un adjuvant qui comprend QS21 et 3D-MPL, dans lesquels les un ou plusieurs premiers et seconds polypeptides comprennent les résidus 4 à 725 de SEQ ID NO : 10, et dans lesquels le terme « de façon concomitante » signifie que les un ou plusieurs polypeptides immunogènes, les un ou plusieurs vecteurs adénoviraux et l'adjuvant sont administrés dans une période d'au plus 12 heures par exemple dans une période d'au plus 1 heure, généralement à une occasion par exemple au cours de la même visite chez le professionnel de santé, par exemple les un ou plusieurs polypeptides immunogènes, les un ou plusieurs vecteurs adénoviraux et l'adjuvant sont administrés séquentiellement ou simultanément.

2. Un ou plusieurs vecteurs adénoviraux comprenant un ou plusieurs polynucléotides hétérologues codant pour un ou plusieurs seconds polypeptides immunogènes dérivés de *Mycobacterium spp.* pour une utilisation dans un procédé d'induction d'une réponse immunitaire dirigée contre ledit *Mycobacterium spp.* dans lesquels les un ou plusieurs vecteurs adénoviraux sont administrés de façon concomitante avec (i) un ou plusieurs premiers polypeptides immunogènes dérivés dudit *Mycobacterium spp.* et (ii) un adjuvant qui comprend QS21 et 3D-MPL, dans lesquels les un ou plusieurs premiers et seconds polypeptides comprennent les résidus 4 à 725 de SEQ ID NO : 10, et dans lesquels le terme « de façon concomitante » signifie que les un ou plusieurs polypeptides immunogènes, les un ou plusieurs vecteurs adénoviraux et l'adjuvant sont administrés dans une période d'au plus 12 heures, par exemple dans une période d'au plus 1 heure, généralement à une occasion, par exemple au cours de la même visite chez le professionnel de santé, par exemple les un ou plusieurs polypeptides immunogènes, les un ou plusieurs vecteurs adénoviraux et l'adjuvant sont administrés séquentiellement ou simultanément.

3. Un ou plusieurs premiers polypeptides immunogènes pour une utilisation ou les un ou plusieurs vecteurs adénoviraux pour une utilisation selon la revendication 1 ou 2, dans lesquels les un ou plusieurs premiers polypeptides immunogènes sont co-formulés avec l'adjuvant.

4. Un ou plusieurs premiers polypeptides immunogènes pour une utilisation ou les un ou plusieurs vecteurs adénoviraux pour une utilisation selon l'une quelconque des revendications 1 à 3 dans lesquels le procédé d'induction d'une réponse immunitaire dirigée contre *Mycobacterium spp.* consiste en (a) l'administration de façon concomitante (i) desdits un ou plusieurs premiers polypeptides immunogènes ; (ii) desdits un ou plusieurs vecteurs adénoviraux ; et (iii) d'un adjuvant ; et (b) facultativement la répétition des étapes de (a) lors d'une seconde occasion, et dans lesquels le terme « de façon concomitante » signifie que les un ou plusieurs polypeptides immunogènes, les un ou plusieurs vecteurs adénoviraux et l'adjuvant sont administrés dans une période d'au plus 12 heures par exemple dans une période d'au plus 1 heure, généralement à une occasion, par exemple au cours de la même visite chez le professionnel de santé, par exemple les un ou plusieurs polypeptides immunogènes, les un ou plusieurs vecteurs adénoviraux et l'adjuvant sont administrés séquentiellement ou simultanément.

5. Un ou plusieurs premiers polypeptides immunogènes pour une utilisation ou les un ou plusieurs vecteurs adénoviraux pour une utilisation selon l'une quelconque des revendications 1 à 4 dans lesquels le procédé d'induction d'une réponse immunitaire dirigée contre *Mycobacterium spp.* n'implique pas l'administration d'une quelconque dose de sensibilisation d'un polypeptide immunogène ou d'un polynucléotide codant pour un polypeptide immunogène.

6. Un ou plusieurs premiers polypeptides immunogènes pour une utilisation ou les un ou plusieurs vecteurs adénoviraux pour une utilisation selon l'une quelconque des revendications 1 à 5, dans lesquels les un ou plusieurs premiers polypeptides immunogènes, les un ou plusieurs vecteurs adénoviraux et l'adjuvant sont co-formulés.

7. Un ou plusieurs premiers polypeptides immunogènes pour une utilisation ou les un ou plusieurs vecteurs adénoviraux pour une utilisation selon l'une quelconque des revendications 1 à 5 dans lesquels les un ou plusieurs premiers polypeptides immunogènes, les un ou plusieurs vecteurs adénoviraux et l'adjuvant ne sont pas co-formulés et sont administrés par des voies d'administration différentes.

8. Composition de vaccin comprenant (i) un ou plusieurs premiers polypeptides immunogènes dérivés de *Mycobacterium spp.* ; (ii) un ou plusieurs vecteurs adénoviraux comprenant un ou plusieurs polynucléotides hétérologues codant pour un ou plusieurs seconds polypeptides immunogènes dérivés de *Mycobacterium spp.* ; et (iii) un adjuvant qui comprend QS21 et 3D-MPL, dans laquelle les un ou plusieurs premiers et seconds polypeptides comprennent les résidus 4 à 725 de SEQ ID NO : 10.

9. Kit comprenant (i) un ou plusieurs premiers polypeptides immunogènes dérivés de *Mycobacterium spp.* ; (ii) un ou plusieurs vecteurs adénoviraux comprenant un ou plusieurs polynucléotides hétérologues codant pour un ou plusieurs seconds polypeptides immunogènes dérivés dudit *Mycobacterium spp.* ; et (iii) un adjuvant qui comprend QS21 et 3D-MPL, dans lequel les un ou plusieurs premiers et seconds polypeptides comprennent les résidus 4 à 725 de SEQ ID NO : 10, et dans lequel les un ou plusieurs polypeptides immunogènes, les un ou plusieurs vecteurs adénoviraux et l'adjuvant sont destinés à être administrés de façon concomitante, dans lequel le terme « de façon concomitante » signifie que les un ou plusieurs polypeptides immunogènes, les un ou plusieurs vecteurs adénoviraux et l'adjuvant sont administrés dans une période d'au plus 12 heures, par exemple dans une période d'au plus 1 heure, généralement à une occasion, par exemple au cours de la même visite chez le professionnel de santé, par exemple les un ou plusieurs polypeptides immunogènes, les un ou plusieurs vecteurs adénoviraux et l'adjuvant sont administrés séquentiellement ou simultanément.

10. Un ou plusieurs premiers polypeptides immunogènes pour une utilisation, les un ou plusieurs vecteurs adénoviraux pour une utilisation, la composition de vaccin ou le kit selon l'une quelconque des revendications 1 à 9 dans lesquels le *Mycobacterium spp.* est *Mycobacterium tuberculosis.*

11. Un ou plusieurs premiers polypeptides immunogènes pour une utilisation, les un ou plusieurs vecteurs adénoviraux pour une utilisation, la composition de vaccin ou le kit selon l'une quelconque des revendications 1 à 10 dans lesquels un ou plusieurs des vecteurs adénoviraux sont dérivés d'un adénovirus de primate non humain.

12. Un ou plusieurs premiers polypeptides immunogènes pour une utilisation, les un ou plusieurs vecteurs adénoviraux pour une utilisation, la composition de vaccin ou le kit selon l'une quelconque des revendications 1 à 11 dans lesquels l'adjuvant contient des liposomes.
